(19) Europäisches Patentamt
European Patent Office
Office européen des brevets

(11) Veröffentlichungsnummer: **0 519 307 A2**

(12) **EUROPÄISCHE PATENTANMELDUNG**

(21) Anmeldenummer: **92109745.7**

(22) Anmeldetag: **10.06.92**

(51) Int. Cl.[5]: **C07D 495/22**, A61K 31/55, C07D 487/14, C07D 495/14, C07D 487/04, //(C07D495/22, 333:00,249:00,243:00,235:00), (C07D495/22,333:00,243:00, 235:00,235:00),(C07D495/22, 333:00,243:00,235:00,231:00), (C07D487/14,249:00,243:00, 235:00)

(30) Priorität: **17.06.91 CH 1791/91**
**08.04.92 CH 1140/92**

(43) Veröffentlichungstag der Anmeldung:
**23.12.92 Patentblatt 92/52**

(84) Benannte Vertragsstaaten:
**AT BE CH DE DK ES FR GB GR IT LI LU MC NL PT SE**

(71) Anmelder: **F. HOFFMANN-LA ROCHE AG**
**Postfach 3255**
**CH-4002 Basel(CH)**

(72) Erfinder: **Borer, René**
**Therwilerstrasse 62**
**CH-4153 Reinach(CH)**
Erfinder: **Gassner, Walter**
**Zehntenfreistrasse 31**
**CH-4103 Bottmingen(CH)**
Erfinder: **Gerecke, Max**
**Bruderholzstrasse 47**
**CH-4153 Reinach(CH)**
Erfinder: **Kyburz, Emilio**
**Unterer Rebbergweg 127**
**CH-4153 Reinach(CH)**

(74) Vertreter: **Bertschinger, Christoph, Dr. et al**
**Grenzacherstrasse 124 P.O. Box 3255**
**CH-4002 Basel(CH)**

(54) **Tetracyclische Imidazodiazepine.**

(57) Die neuen Imidazodiazepin-Derivate der allgemeinen Formel

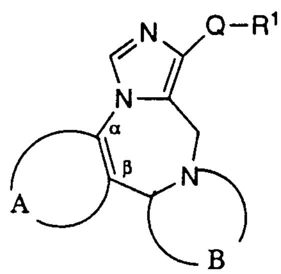

I

worin A zusammen mit den beiden mit $\alpha$ und $\beta$ bezeichneten Kohlenstoffatomen eine der Gruppen

EP 0 519 307 A2

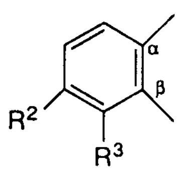 , 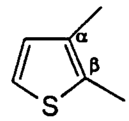 , 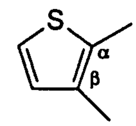 ,

(a) (b) (c)

B einen der Reste

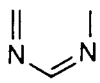 , 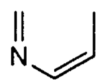 , 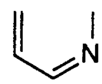 ,

(d) (e) (f)

Q eine der Gruppen

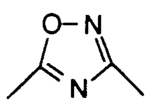 , 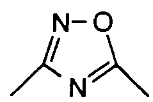 ,

(g) (h)

$R^1$ eine gegebenenfalls durch Hydroxy, niederes Alkoxy, Aryl, $C_{3-6}$Cycloalkyl, Aroyl, Aryloxy, Heteroaroyloxy, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, eine Aroylgruppe, einen über ein Kohlenstoffatom gebundenen fünfgliedrigen Heterocyclus oder gegebenenfalls durch Acyl oder niederes Alkyl substituiertes $C_{3-6}$-Cycloalkyl,

$R^2$ und $R^3$ je Wasserstoff, Halogen oder niederes Alkyl,

$R^4$ Wasserstoff oder niederes Alkyl,

$R^5$ Wasserstoff, Aryl, Acyl, $C_{3-6}$-Cycloalkyl, Aralkoxycarbonyl oder ggfs durch Aryl, Morpholino, niederes Alkoxy, Hydroxy, 2,2-Dimethyl-1,3-dioxolan-4-yl, Alkoxycarbonyl, Carbamoyl, Alkoxycarbonylamino, Aralkoxycarbonyl oder Amino substituiertes niederes Alkyl oder $R^4$ und $R^5$ zusammen mit dem Stickstoffatom entweder Phthalimino oder einen sechsgliedrigen gesättigten Heterocyclus bedeuten,

können zur Bekämpfung oder Verhütung von Krankheiten verwendet werden. Sie sind insbesondere geeignet zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

Die vorliegende Erfindung betrifft neue Verbindungen der allgemeinen Formel

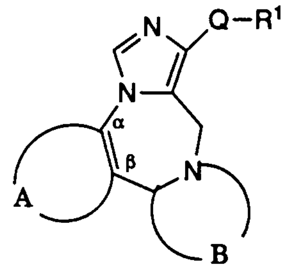

I

worin A zusammen mit den beiden mit α und β bezeichneten Kohlenstoffatomen eine der Gruppen

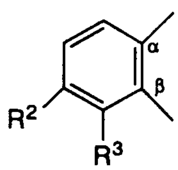

,

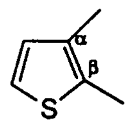

,

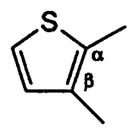

,

(a) (b) (c)

B einen der Reste

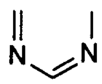

,

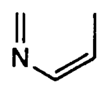

,

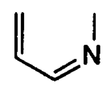

,

(d) (e) (f)

Q eine der Gruppen

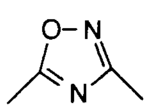

,

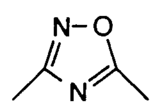

,

(g) (h)

$R^1$ eine gegebenenfalls durch Hydroxy, niederes Alkoxy, Aryl, $C_{3-6}$-Cycloalkyl, Aroyl, Aryloxy, Heteroaroyloxy, Acyloxy, Aryl-(niederes)-alkoxy, Halogen, die Gruppe -$NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, eine Aroylgruppe, einen über ein Kohlenstoffatom gebundenen fünfgliedrigen Heterocyclus oder gegebenenfalls durch Acyl oder niederes Alkyl substituiertes $C_{3-6}$-Cycloalkyl,

$R^2$ und $R^3$ je Wasserstoff, Halogen oder niederes Alkyl,

$R^4$ Wasserstoff oder niederes Alkyl,

$R^5$ Wasserstoff, Aryl, Acyl, $C_{3-6}$-Cycloalkyl, Aralkoxycarbonyl oder gegebenenfalls durch Aryl, Morpholino, niederes Alkoxy, Hydroxy, 2,2-Dimethyl-1,3-dioxolan-4-yl, Alkoxycarbonyl, Carbamoyl, Alkoxycarbonylamino, Aralkoxycarbonyl oder Amino substituiertes niederes Alkyl oder $R^4$ und $R^5$ zusammen mit dem Stickstoffatom entweder Phthalimino oder einen sechsgliedrigen gesättigten Heterocyclus bedeuten,

und pharmazeutisch annehmbare Säureadditionssalze von basischen Verbindungen der Formel I.

Diese Imidazodiazepin-Derivate besitzen wertvolle pharmakologische Eigenschaften und können zur Bekämpfung oder Verhütung von Krankheiten verwendet werden. Sie sind insbesondere geeignet zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

Gegenstand der vorliegenden Erfindung sind: Die obigen Verbindungen der Formel I und die erwähnten Salze davon als solche; Verfahren und Zwischenprodukte zu deren Herstellung; die obigen Verbindungen der Formel I und die erwähnten Salze davon zur Anwendung als therapeutische Wirkstoffe; Arzneimittel auf der Basis dieser neuen Wirkstoffe und deren Herstellung; die Verwendung dieser Wirkstoffe bei der Bekämpfung oder Verhütung von Krankheiten; sowie deren Verwendung zur Herstellung von Arzneimitteln.

Der Ausdruck "nieder" bezeichnet Reste und Verbindungen mit höchstens sieben, vorzugsweise höchstens vier Kohlenstoffatomen. Der Ausdruck "Alkyl" bezeichnet geradkettige oder verzweigte, gesättigte Kohlenwasserstoffreste, wie Methyl, Ethyl, Propyl, Isopropyl und t-Butyl. Der Ausdruck "Alkoxy" bezeichnet über ein Sauerstoffatom gebundene Alkylgruppen, wie Methoxy und Ethoxy.

Der Ausdruck "Cycloalkyl" bezeichnet Reste wie Cyclopropyl.

Der Ausdruck "Halogen" bedeutet Fluor, Chlor, Brom und Jod.

Der Ausdruck "Aryl" bezeichnet gegebenenfalls durch niederes Alkyl substituierte Phenylreste, wie Xylyl, oder gegebenenfalls durch Halogen, Hydroxy, niederes Alkoxy, Trifluormethyl, Trifluormethyloxy, oder durch Alkylendioxy wie beispielsweise Methylendioxy, oder durch seinerseits gegebenenfalls substituiertes Benzyloxy wie beispielsweise 4-Brombenzyloxy substituiertes Phenyl.

Die Ausdrücke "Aryloxy" und "Heteroaryloxy" bezeichnen über ein Sauerstoffatom gebundene Arylreste bzw. Heteroarylreste.

Der Ausdruck "Heteroaryl" bezeichnet dabei den Rest eines aromatischen Heterocyclus, insbesondere eines sechsgliedrigen Heterocyclus, welcher ein oder mehrere Stickstoffatome enthält, wie Pyridyl, beispielsweise 3-Pyridyl.

Der über ein Kohlenstoffatom gebundene fünfgliedrige Heterocyclus kann aromatisch oder gesättigt sein; er kann ein Stickstoff-, Sauerstoff- oder Schwefelatom und gegebenenfalls zusätzlich ein Stickstoffatom als Ringglieder enthalten und kann unsubstituiert oder durch niederes Alkyl substituiert sein oder in Nachbarschaft zu einem nichtaromatischen Stickstoffatom eine Oxogruppe enthalten. Beispiele hierfür sind 2-Thienyl-, 3-Thienyl-, 2-Furyl-, 5-Oxazolyl- oder 2-Tetrahydrofuryl-Gruppen.

Der über ein Stickstoffatom gebundene fünfgliedrige Heterocyclus ist aromatisch und kann als zusätzliches Ringglied gegebenenfalls ein zweites Stickstoffatom enthalten, wie beispielsweise eine 1-Imidazolylgruppe.

Der sechsgliedrige gesättigte Heterocyclus kann als Ringglied zusätzlich ein Sauerstoffatom oder die Gruppe >N-$R^6$ enthalten, wobei $R^6$ niederes Alkyl, Aryl, niederes Alkenyl oder niederes Alkinyl bedeutet. Beispiele hierfür sind die 4-Morpholino- oder die 1-Piperazinylgruppe, welche in 4-Stellung durch niederes Alkyl, Aryl, niederes Alkenyl oder niederes Alkinyl substituiert ist.

Das Symbol $R^1$ bedeutet vorzugsweise Cyclopropyl.

In einer bevorzugten Ausführungsform bedeutet das Symbol A die Gruppe a).

In einer weiteren bevorzugten Ausführungsform bedeutet $R^3$ Wasserstoff und $R^2$ Wasserstoff, Fluor oder Chlor.

Das Symbol B bedeutet bevorzugt den Rest d) oder e).

Die nachfolgend aufgeführten Verbindungen sind besonders bevorzugte Vertreter der durch die Formel I definierten Stoffklasse.

10-(3-Cyclopropyl-1,2,4-oxadiazol-5-yl)-3-fluor-9H-imidazo[1,5-a][1,2,4]-triazolo[1,5-d][1,4]benzodiazepin,
10-(5-Cyclopropyl-1,2,4-oxadiazol-3-yl)-3-fluor-9H-imidazo[1,5-a][1,2,4]-triazolo[1,5-d][1,4]benzodiazepin,
10-(5-Cyclopropyl-1,2,4-oxadiazol-3-yl)-9H-imidazo[1,5-a][1,2,4]triazolo[1,5-d][1,4]benzodiazepin,
10-(3-Cyclopropyl-1,2,4-oxadiazol-5-yl)-9H-imidazo[1,5-a][1,2,4]triazolo[1,5-d][1,4]benzodiazepin,
10-(3-Cyclopropyl-1,2,4-oxadiazol-5-yl)-3-fluor-9H-diimidazo[1,5-a:1',2'-d][1,4]benzodiazepin,
10-(5-Cyclopropyl-1,2,4-oxadiazol-3-yl)-3-fluor-9H-diimidazo[1,5-a:1',2'-d][1,4]benzodiazepin,
10-(5-Cyclopropyl-1,2,4-oxadiazol-3-yl)-9H-diimidazo [1,5`a:1',2'-d][1,4]-benzodiazepin,
3-Chlor-10-(5-cyclopropyl-1,2,4-oxadiazol-3-yl)-9H-diimidazo[1,5-a:1',2'][1,4]benzodiazepin,
4-Chlor-10-(3-cyclopropyl-1,2,4-oxadiazol-5-yl)-9H-diimidazo[1,5-a:1',2'-d][1,4]benzodiazepin,
4-Chlor-10-(5-cyclopropyl-1,2,4-oxadiazol-3-yl)-9H-diimidazo[1,5-a:1',2'-d][1,4]benzodiazepin.

Die Verbindungen der Formel I und die pharmazeutisch annehmbaren Säureadditionssalze von basischen Verbindungen der Formel I können erfindungsgemäss hergestellt werden, indem man

a) ein reaktionsfähiges funktionelles Derivat einer Carbonsäure der allgemeinen Formel

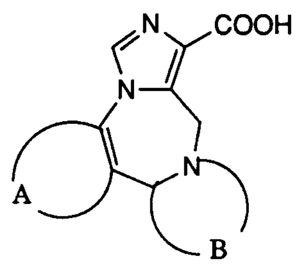

II

worin A und B die obige Bedeutung besitzen,

mit einem Oxim der allgemeinen Formel

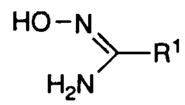

III

worin $R^1$ obige Bedeutung besitzt,

umsetzt, oder

b) eine Verbindung der allgemeinen Formel

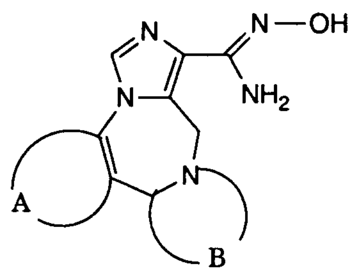

IV

worin A und B obige Bedeutung besitzen,

mit einem reaktionsfähigen funktionellen Derivat einer Carbonsäure der allgemeinen Formel

$R^1$-COOH V

worin $R^1$ obige Bedeutung besitzt,

umsetzt, oder

c) eine Verbindung der allgemeinen Formel

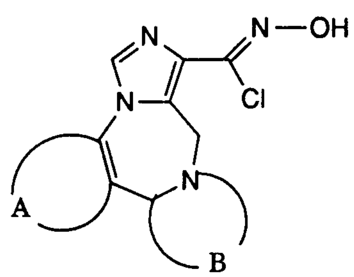

VI

worin A und B obige Bedeutung haben,

mit einem Nitril der Formel

$R^{11}$-C≡N VII

worin $R^{11}$ Arylamino-alkyl bedeutet,
umsetzt, oder
d) eine Verbindung der allgemeinen Formel

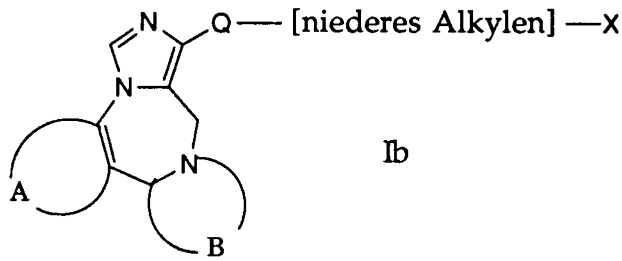

Ib

worin A, B und Q die obige Bedeutung haben und X eine Abgangsgruppe bedeutet,
mit einem Amin der Formel

$HNR^4R^5$ VIII

worin $R^4$ und $R^5$ obige Bedeutung haben,
umsetzt, oder
e) eine Verbindung der allgemeinen Formel

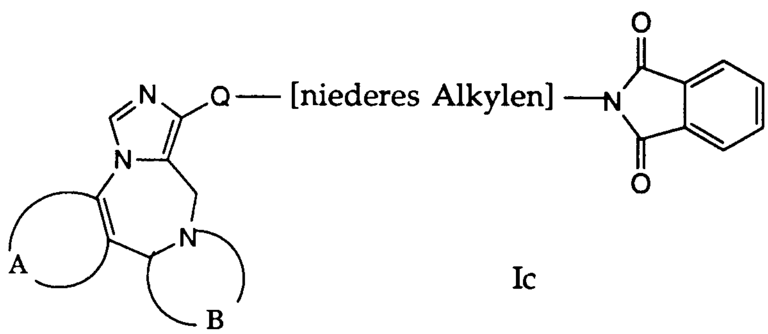

Ic

worin A, B und Q obige Bedeutung besitzen,
in das entsprechende Amin überführt, oder
f) eine Verbindung der allgemeinen Formel

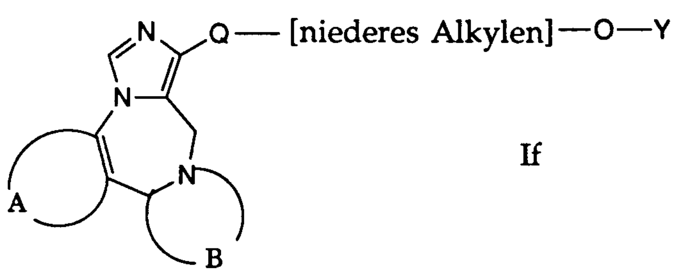

If

worin A, B und Q obige Bedeutung besitzen und Y eine leicht abspaltbare Arylalkylgruppe darstellt,
einer Etherspaltung unterzieht, oder
g) eine Verbindung der allgemeinen Formel

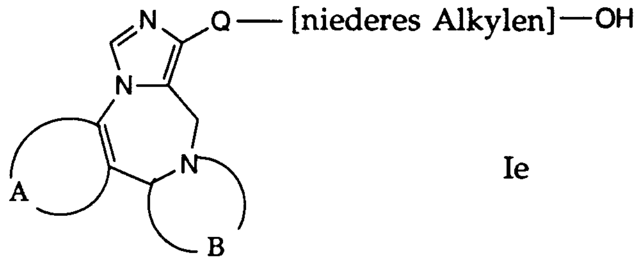

Ie

verestert, oder

h) eine Verbindung der allgemeinen Formel

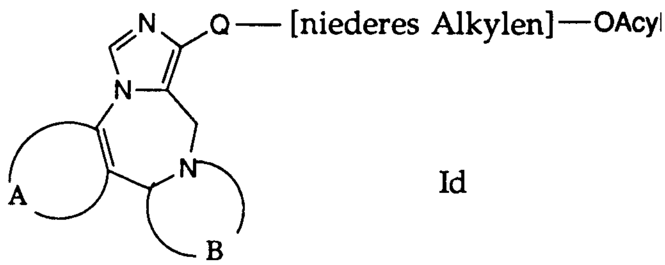

Id

worin A, B und Q obige Bedeutung besitzen,

verseift, oder

i) eine Verbindung der allgemeinen Formel

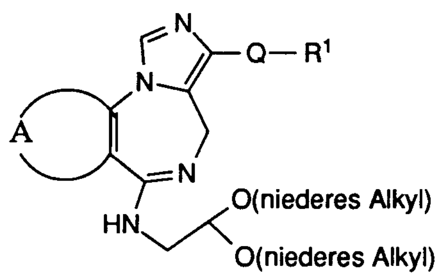

IX

worin A und Q obige Bedeutung besitzen,

mit einer Säure behandelt, oder

j) eine Verbindung der allgemeinen Formel

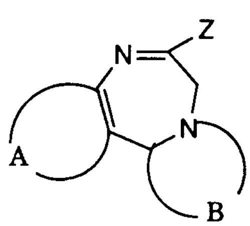

X

worin A und B obige Bedeutung besitzen und Z eine Abgangsgruppe bedeutet,

in Gegenwart einer Base mit einem Isonitril der Formel

$C = N\text{-}CH_2\text{-}Q\text{-}R^1$ XI

worin Q und $R^1$ obige Bedeutung besitzen,

umsetzt, und

k) erwünschtenfalls eine erhaltene basische Verbindung der Formel I in ein pharmazeutisch annehmbares Säureadditionssalz überführt.

Gemäss Verfahrensvariante a) können Verbindungen der Formel I hergestellt werden, worin A und B obige Bedeutung haben und Q die Gruppe g) bedeutet.

Die gewünschte Reaktion kann dadurch bewerkstelligt werden, dass man ein in an sich bekannter Weise hergestelltes reaktives Derivat der Carbonsäure der Formel II mit einer Verbindung der Formel III umsetzt, zweckmässigerweise in Gegenwart einer Base. Als reaktive Derivate verwendet man beispielsweise die entsprechenden Carbonsäurechloride, welche man vorzugsweise mittels Thionylchlorid in Gegenwart einer geringen Menge an N,N-Dimethylformamid in Toluol herstellt. Bevorzugt werden jedoch die entsprechenden Imidazolide als reaktive Carbonsäurederivate verwendet, welche man aus den entsprechenden Carbonsäuren durch Behandeln mit 1,1'-Carbonyldiimidazol in einem inerten Lösungsmittel, wie beispielsweise N,N-Dimethylformamid herstellt.

Als Basen eignen sich beispielsweise Amine wie Triethylamin, Pyridin und dergleichen. Die Reaktion wird vorzugsweise in einem Temperaturbereich von etwa Raumtemperatur bis zur Rückflusstemperatur des Reaktionsgemisches durchgeführt, wobei man zweckmässigerweise bei Raumtemperatur arbeitet.

Gemäss Verfahrensvariante b) können Verbindungen der Formel I hergestellt werden, worin A und B obige Bedeutung haben und Q die Gruppe (h) bedeutet.

Die gewünschte Reaktion kann analog Verfahrensvariante a) durchgeführt werden, d.h. dass man eine Carbonsäure der Formel V zunächst in an sich bekannter Weise in ein reaktives Derivat überführt und dieses dann gegebenenfalls in Gegenwart einer Base mit einer Verbindung der Formel IV umsetzt. Als reaktive Derivate können beispielsweise die entsprechenden Carbonsäurechloride eingesetzt werden, welche man bevorzugt durch Behandeln der entsprechenden Carbonsäure mit Thionylchlorid in Gegenwart einer geringen Menge an N,N-Dimethylformamid in Toluol herstellt. Des weiteren können auch die entsprechenden Imidazolide als reaktive Derivate verwendet werden, welche aus der entsprechenden Carbonsäure durch Behandeln mit 1,1-Carbonyldiimidazol in einem inerten Lösungsmittel wie Dimethylformamid zugänglich sind.

Als Basen können beispielsweise Amine, wie Triethylamin, Pyridin und dergleichen eingesetzt werden. Die Reaktion wird vorzugsweise bei Raumtemperatur bis zur Rückflusstemperatur des Reaktionsgemisches durchgeführt, wobei man zweckmässigerweise bei Raumtemperatur arbeitet.

Gemäss Verfahrensvariante c) können Verbindungen der allgemeinen Formel I hergestellt werden, worin A und B obige Bedeutung haben, Q die Gruppe (h) und $R^{11}$ Arylamino-alkyl wie beispielsweise 2,6-Dimethylxylidino bedeutet.

Die gewünschte Reaktion kann dadurch bewerkstelligt werden, dass man ein Carbonylchloridoxim der allgemeinen Formel VI in einem inerten Lösungsmittel wie beispielsweise 1,2-Dimethoxyethan suspendiert und mit einem Nitril der Formel VII in Gegenwart einer Base umsetzt. Diese Reaktion wird vorzugsweise bei der Rückflusstemperatur des Reaktionsgemisches durchgeführt. Als Basen können beispielsweise Amine wie Triethylamin, Pyridin und dergleichen verwendet werden.

Gemäss Verfahrensvariante d) können Verbindungen der allgemeinen Formel I hergestellt werden, worin A, B und Q obige Bedeutung haben und $R^1$ eine durch die Gruppe $-NR^4R^5$ substituierte Kohlenwasserstoffgruppe darstellt.

Die in Formel Ib mit X bezeichnete Gruppe ist eine leicht abspaltbare Gruppe wie beispielsweise Halogen, Tosylat oder ähnliches. Die bei dieser Reaktion verwendeten Verbindungen der Formel Ib sind nach der Verfahrensvariante a) bzw. b) zugänglich oder in Analogie dazu. Die Verbindungen der Formel Ib werden in einem geeigneten Lösungsmittel, wie beispielsweise Dimethylformamid gelöst und mit einem entsprechenden Amin der Formel VIII umgesetzt. Diese Reaktion wird in einem Temperaturbereich von etwa Raumtemperatur bis zur Rückflusstemperatur des Reaktionsgemisches durchgeführt, wobei die Temperaturen von 80° - 100°C bevorzugt sind.

Als Basen können bei dieser Reaktion die als Reaktionskomponenten verwendeten Amine der Formel VIII benutzt werden, wobei dieses Amin im Ueberschuss eingesetzt wird. Die Reaktion kann aber auch in Gegenwart von Basen wie Triethylamin, Pyridin und dergleichen durchgeführt werden.

Gemäss Verfahrensvariante e) können Verbindungen der allgemeinen Formel I hergestellt werden, worin A, B und Q obige Bedeutung haben und $R^1$ eine Aminoalkylgruppe bedeutet. Die bei dieser Reaktion verwendeten Verbindungen der Formel Ic sind nach der Verfahrensvariante a) bzw. b) zugänglich oder in Analogie dazu.

Die Phthalimide der Formel Ic werden in einem geeigneten Lösungsmittel suspendiert. Bevorzugt wird ein niederer Alkohol dazu verwendet. Für diese Reaktion verwendet man vorzugsweise Hydrazinhydrat und als Lösungsmittel vorzugsweise einen niederen Alkohol, wie Methanol oder Ethanol. Es wird vorzugsweise

in einem Temperaturbereich von Raumtemperatur bis zur Rückflusstemperatur des Reaktionsgemisches gearbeitet.

Gemäss Verfahrensvariante f) können Verbindungen der allgemeinen Formel I hergestellt werden, worin A, B und Q obige Bedeutung haben und $R^1$ eine Hydroxyalkylgruppe bedeutet. Die bei dieser Reaktion verwendeten Verbindungen der Formel If sind nach der Verfahrensvariante a) oder b) zugänglich oder in Analogie dazu. Die Verbindungen der Formel If werden in einem geeigneten Lösungsmittel gelöst, wie beispielsweise Essigsäure. Diese Reaktion wird vorzugsweise durch Behandeln mit Bromwasserstoff durchgeführt. Die Reaktionstemperatur liegt vorzugsweise bei Raumtemperatur. Die erhaltenen Ester werden bei Raumtemperatur in Ethanol mit einer Natriummethylat-Lösung und durch Zugabe von Wasser verseift.

Gemäss Verfahrensvariante g) können Verbindungen der allgemeinen Formel I hergestellt werden, worin A, B und Q obige Bedeutung haben und $R^1$ eine Acyloxyalkylgruppe bedeutet. Die bei dieser Reaktion verwendeten Verbindungen der Formel Ie, sind nach den Verfahrensvarianten a), b) oder f) zugänglich oder in Analogie dazu.

Die Verbindungen der Formel Ie werden mit reaktionsfähigen Derivaten von Carbonsäure umgesetzt. Als Carbonsäurederivate eignen sich beispielsweise Essigsäureanhydrid, Acetylchlorid und ähnliches. Zweckmässigerweise erfolgt die Reaktion in einem Lösungsmittel, wie Pyridin.

Gemäss Verfahrensvariante j) können Verbindungen der allgemeinen Formel I aus Verbindungen der allgemeinen Formel X und einem Isonitril der allgemeinen Formel XI hergestellt werden. Die in Formel X mit Z bezeichnete Abgangsgruppe ist beispielsweise eine leicht abspaltbare Phosphinylgruppe, z.B. eine Gruppe der Formel

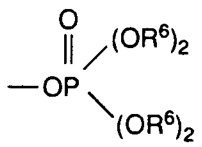

oder

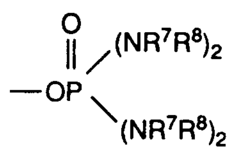

worin $R^6$ niederes Alkyl und $R^7$ und $R^8$ je niederes Alkyl, Allyl, Phenyl oder substituiertes Phenyl oder zusammen mit dem Stickstoffatom einen unsubstituierten oder substituierten heterocyclischen Ring mit 3 bis 8 Gliedern (wie Morpholin), bedeuten,

ein Halogenatom, eine Alkylthiogruppe, eine Aralkylthiogruppe, eine N-Nitrosoalkylaminogruppe, eine Alkyloxygruppe, eine Mercaptogruppe und dergleichen. Die Reaktion einer Verbindung der allgemeinen Formel X mit einem Isonitril der Formel XI erfolgt in einem inerten Lösungsmittel, wie Dimethylformamid, Hexamethylphosphorsäuretriamid, Dimethylsulfoxid, Tetrahydrofuran oder in irgend einem anderen geeigneten organischen Lösungsmittel und in Gegenwart einer Base, die genügend stark basisch ist, um das Anion des Isonitrils zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, Butyllithium, tertiäre Amine, wie Triäthylamin, und dergleichen. Die Reaktionstemperatur liegt zweckmässigerweise zwischen etwa -70 °C und etwa Raumtemperatur.

Gemäss Verfahrensvariante k) können basische Verbindung der Formel I in pharmazeutisch annehmbare Säureadditionssalze übergeführt werden. Es kommen dabei sowohl Salze mit anorganischen als auch Salze mit organischen Säuren in Betracht. Beispiele derartiger Salze sind Hydrochloride, Hydrobromide, Sulfate, Nitrate, Citrate, Acetate, Maleate, Succinate, Methansulfonate, p-Toluolsulfonate und dergleichen. Diese Salze können nach an sich bekannten und jedem Fachmann geläufigen Methoden hergestellt werden.

Die verschiedenen als Ausgangsstoffe verwendeten Verbindungen können beispielsweise den nachfolgenden Reaktionsschemata I-IV und der jeweils davon anschliessenden Erläuterung der verschiedenen Reaktionen hergestellt werden.

Reaktionsschema I

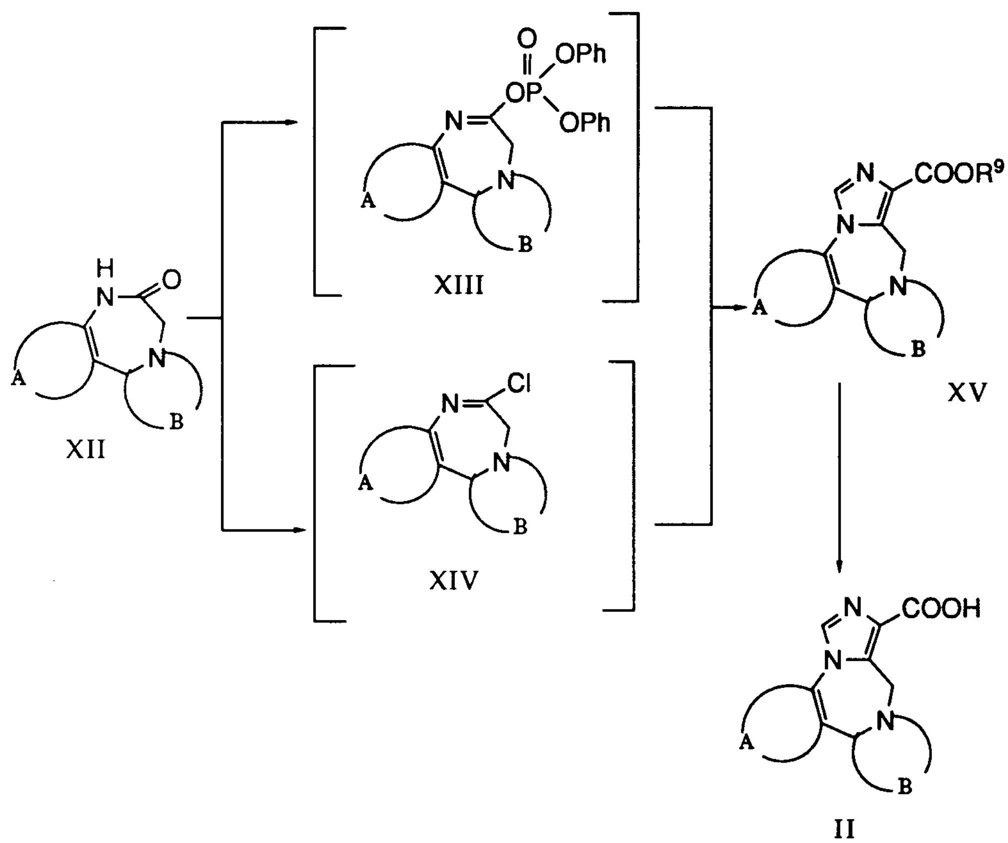

$R^9$ bedeutet niederes Alkyl, wie tert-Butyl oder Ethyl; A und B besitzen die oben angegebene Bedeutung.

Durch Behandeln einer Verbindung der Formel XII mit erstens einer Dispersion eines Alkalihydrids, beispielsweise Natriumhydrid, in einem inerten Suspensionsmittel wie einem Mineralöl und zweitens mit Diphenylphosphorylchlorid erhält man eine Verbindung der Formel XIII. Diese Reaktion wird vorzugsweise bei einer Temperatur von ca. -10 °C bis ca. -60 °C in einem inerten Lösungsmittel wie beispielsweise N,N-Dimethylformamid durchgeführt.

Andererseits erhält man durch Behandeln einer Verbindung der Formel XII mit N,N,4-Trimethylanilin und Phosphoroxychlorid in einem inerten Suspensionsmittel, vorzugsweise Chloroform, eine Verbindung der allgemeinen Formel XIV. Diese Reaktion wird vorzugsweise bei der Rückflusstemperatur des Reaktionsgemisches durchgeführt. Zweckmässigerweise werden die gebildeten Verbindungen der allgemeinen Formel XIII und XIV nicht isoliert, sondern direkt weiterverarbeitet.

Zur Herstellung von Estern der allgemeinen Formel XV setzt man die Verbindungen der Formel XIII oder XIV mit einem Isonitril der Formel XI.

Diese Umsetzung erfolgt in einem inerten Lösungsmittel, wie Dimethylformamid, Hexamethylphosphorsäuretriamid, Dimethylsulfoxid, Tetrahydrofuran oder in irgend einem anderen geeigneten organischen Lösungsmittel und in Gegenwart einer Base, die genügend stark basisch ist, um das Anion des Isonitril zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmässigerweise zwischen etwa -60 °C und etwa Raumtemperatur.

Die Carbonsäuren der allgemeinen Formel II können durch Hydrolyse der Estergruppe in Verbindungen der Formel XV nach geläufigen Methoden, beispielsweise durch Behandlung mit wässrigem Natrium- oder Kaliumhydroxid oder durch Behandlung mit Trifluoressigsäure oder einer anderen starken Säure, erhalten werden.

Die Lactame der allgemeinen Formel XII, worin B die Gruppe d) bedeutet, sind bekannt oder können nach an sich bekannten Methoden hergestellt werden, siehe Breuer, Tetrahedron Letters 1976, 1935-1938. Zudem enthalten einige der nachfolgenden Beispiele ausführliche Angaben betreffend die Herstellung solcher Verbindungen der Formel XII.

## Reaktionsschema II

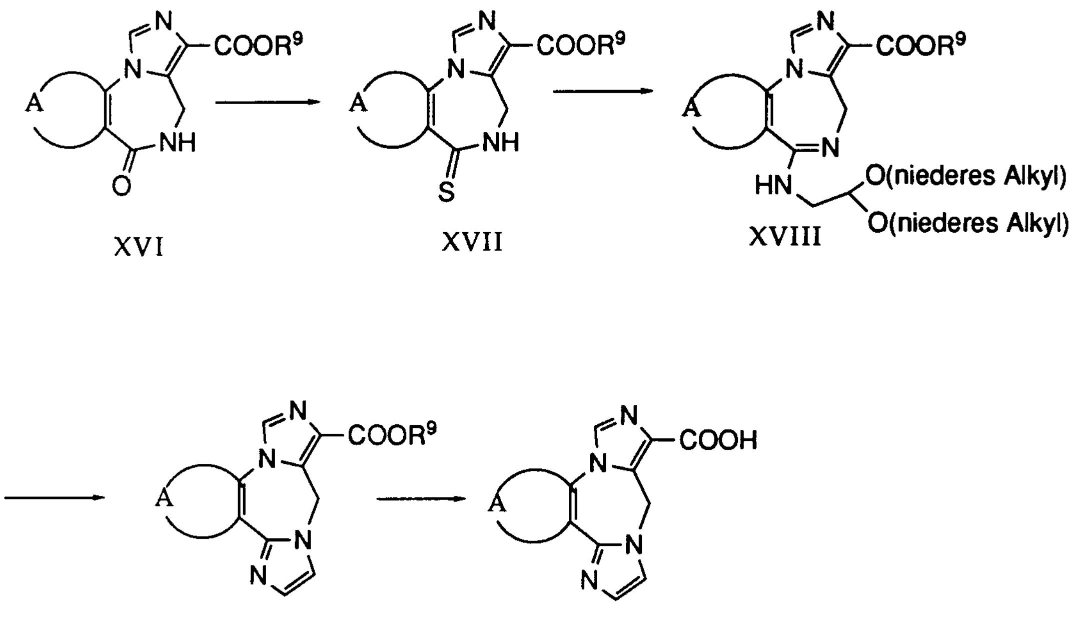

XVI XVII XVIII

XVa IIa

A besitzt die oben angegebene Bedeutung, $R^9$ bedeutet niederes Alkyl wie beispielsweise Ethyl.

Die Verbindungen der allgemeinen Formel XVI gehören einer an sich bekannten Stoffklasse an oder können nach an sich bekannten Methoden hergestellt werden, siehe beispielsweise die Europäische Patentpublikation Nr. 27214 vom 22. April 1981.

Durch Behandeln einer Verbindung der Formel XVI mit einem Dithiadiphosphetan ("Lawesson-Reagens") in einem inerten, hochsiedenden Lösungsmittel, wie beispielsweise Pyridin oder Toluol erhält man eine Verbindung der Formel XVII. Diese Reaktion wird vorzugsweise bei der Rückflusstemperatur des Reaktionsgemisches durchgeführt.

Die Ester der Formel XVII werden mit einem Aminoacetaldehyd-di(nieder)alkylacetal behandelt, wobei Verbindungen der Formel XVIII entstehen. Es wird zweckmässigerweise bei erhöhter Temperatur, vorzugsweise in einem Temperaturbereichvon ca. 80 ° C bis ca. 100 ° C gearbeitet.

Durch Erhitzen einer Verbindung der Formel XVIII in Gegenwart einer organischen Säure, wie zum Beispiel Essigsäure, erhält man die gewünschte Verbindung der Formel XVa, d.h. eine Verbindung der Formel XV, worin B die Gruppe =N-CH=CH- bedeutet. Diese Cyclisierung wird zweckmässigerweise zwischen ca. 70 ° C und ca. 110 ° C durchgeführt.

Die Carbonsäuren der allgemeinen Formel IIa werden nach geläufigen Methoden aus den Carbonsäureestern der Formel XVa hergestellt, beispielsweise durch Behandeln mit einem Alkalihydroxid in Wasser.

## Reaktionsschema III

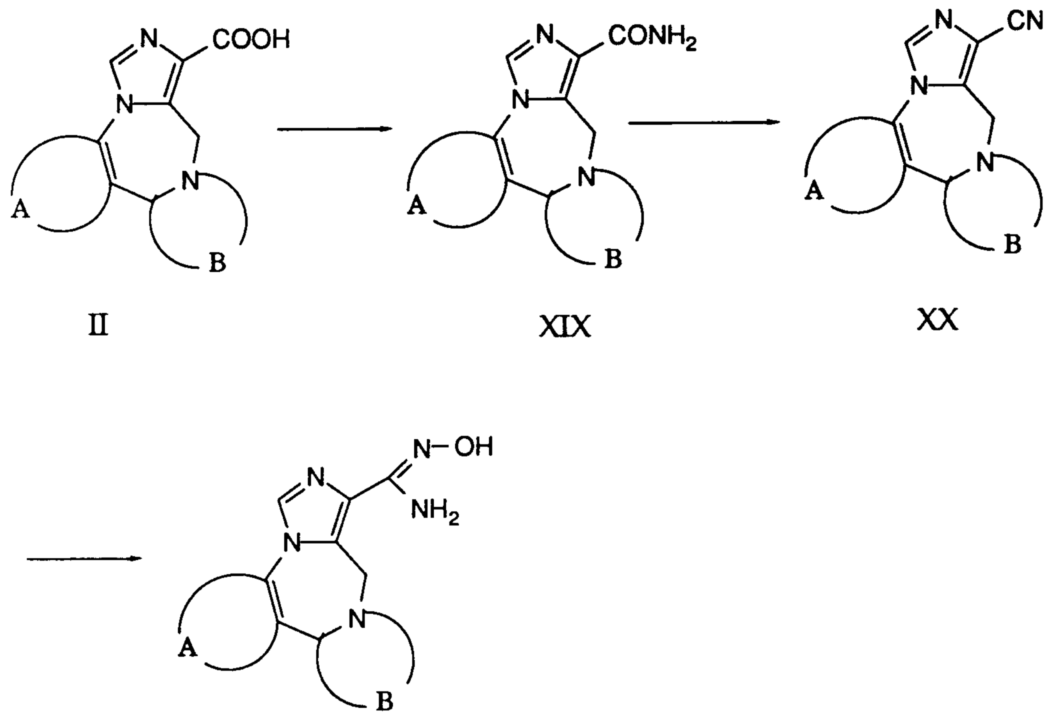

II XIX XX

IV

A und B besitzen die oben angegebene Bedeutung.

Aus den Carbonsäuren der Formel II erhält man nach bekannten Methoden Carboxamide der Formel XIX, beispielsweise durch Umsetzung mit Ammoniak-Wasser in einem geeigneten Lösungsmittel wie N,N-Dimethylformamid oder durch Ueberführung einer Verbindung der Formel II in ein reaktionsfähiges Derivat wie beispielsweise ein Carbonsäurechlorid oder ein Carbonsäure-imidazolid und anschliessender Umsetzung mit Ammoniak.

Durch Behandeln einer Verbindung der Formel XIX mit Trifluoressigsäureanhydrid in Gegenwart etwa der gleichen Menge Pyridin in Dioxan oder ähnlichen erhält man ein Nitril der Formel XX. Diese Reaktion wird vorzugsweise bei Raumtemperatur durchgeführt.

Durch Behandeln einer Verbindung der Formel XX mit Hydroxylaminhydrochlorid erhält man die gewünschte Verbindung der Formel IV. Man verwendet als Lösungsmittel vorzugsweise einen niederen Alkohol wie Ethanol oder Methanol und arbeitet zweckmässigerweise bei erhöhter Temperatur bis zur Rückflusstemperatur des Reaktionsgemisches.

Analog zu den im Reaktionsschema III angegebenen Schritten lassen sich Carbonsäuren der Formel V in Oxime der Formel III umwandeln.

## Reaktionsschema IV

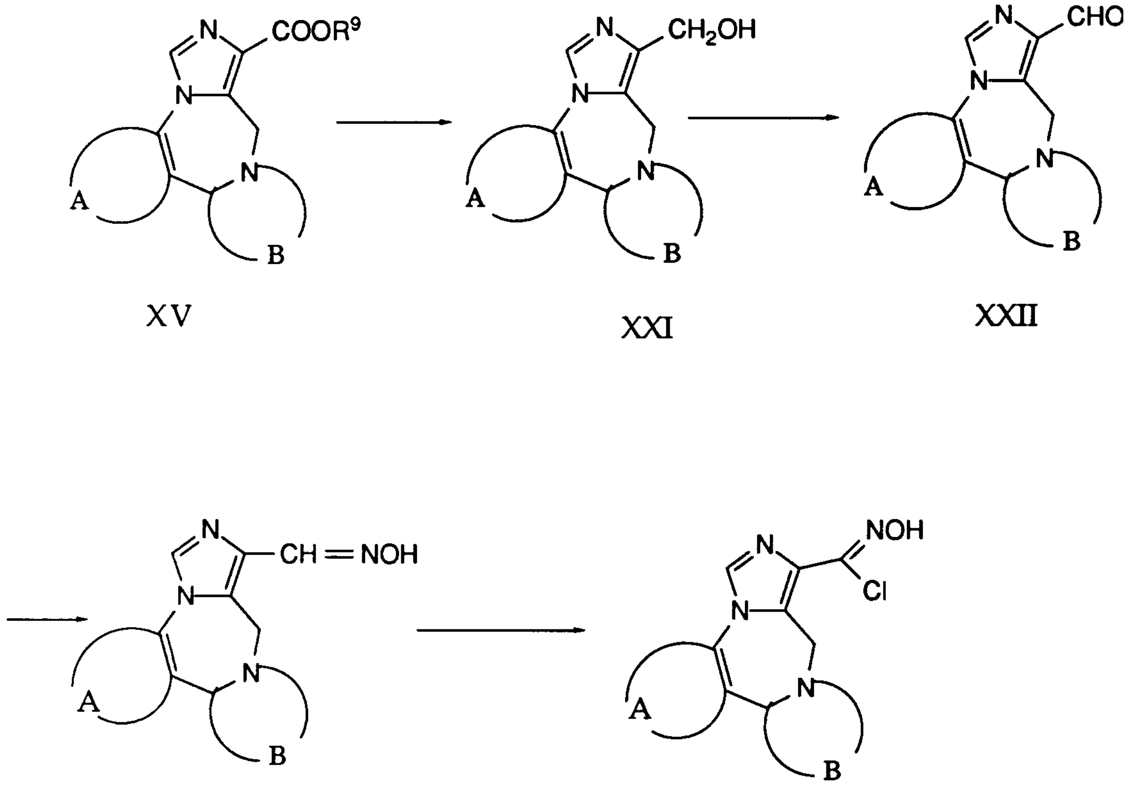

XV XXI XXII

XXIII VI

$R^9$ bedeutet niederes Alkyl wie Ethyl, A und B besitzen die oben angegebenen Bedeutungen.

Durch Behandeln des Carbonsäureesters XV mit einem Reduktionsmittel wie beispielsweise Lithiumborhydrid in einem inerten Lösungsmittel erhält man die Hydroxyverbindung der Formel XXI. Diese Reaktion wird in einem Temperaturbereich von Raumtemperatur bis zur Rückflusstemperatur durchgeführt, wobei die Rückflusstemperatur besonders bevorzugt ist. Als Lösungsmittel wird beispielsweise Tetrahydrofuran oder ein ähnlich inertes Lösungsmittel verwendet.

Die so erhaltene Verbindung der Formel XXI wird mit einem geeigneten Oxidationsmittel wie beispielsweise Mangandioxid in einen Aldehyd der allgemeinen Formel XXII umgewandelt. Die Reaktion erfolgt in Gegenwart eines inerten Lösungsmittels, beispielsweise Dichlormethan. Man arbeitet zweckmässigerweise bei Raumtemperatur oder erhöhter Temperatur.

Durch Umsetzen einer Verbindung der Formel XXII mit Hydroxylaminhydrochlorid in Gegenwart eines inerten Lösungsmittels wie beispielsweise Tetrahydrofuran und einer Base, bevorzugt eines Amins, wie Triethylamin, Pyridin und dergleichen erhält man ein Oxim der Formel XXIII. Bevorzugt wird diese Umsetzung bei der Rückflusstemperatur des Reaktionsgemisches durchgeführt.

Durch Behandeln des Oxims der Formel XXIII mit N-Chlorsuccinimid und Chlorwasserstoff erhält man die gewünschte Verbindung der Formel V. Diese Umsetzung wird bevorzugt zwischen etwa Raumtemperatur und ca. 40 °C durchgeführt. Als Lösungsmittel werden inerte Lösungsmittel, wie beispielsweise Dimethylformamid verwendet.

Die als Zwischenprodukte verwendeten Verbindungen der Formeln II, IV, VI, IX, X, XIII-XV und XVIII-XXIII sowie diejenigen der XII, worin B die Gruppe (e) oder (f) bedeutet sind neu und ebenfalls Gegenstand der vorliegenden Erfindung. Die übrigen als Ausgangsstoffe oder Zwischenprodukte verwendeten Verbindungen gehören an sich bekannten Stoffklassen an.

Wie eingangs erwähnt sind die Verbindungen der Formel I neu; sie besitzen äusserst wertvolle pharmakodynamische Eigenschaften und weisen nur eine geringe Toxizität auf. Sie besitzen als gemeinsames Merkmal eine ausgeprägte Affinität zu den zentralen Benzodiazepin-Rezeptoren und haben entweder

ausgeprägte anxiolytische, antikonvulsive, muskelrelaxierende und sedativ-hypnotische Eigenschaften, und/oder sie antagonisieren selektiv einige oder alle Wirkungen, welche tranquilisierend wirksame 1,4-Benzodiazepine oder andere Substanzen über die zentralen Benzodiazepin-Rezeptoren entfalten, partiell oder vollständig. Diese Eigenschaften können in den nachstehend beschriebenen Versuchen erfasst werden.

3H-Flumazenil-Bindungs-Test

Die Affinität von Verbindungen der allgemeinen Formel I zu den zentralen Benzodiazepin-Rezeptoren wurde in vitro nach derin Nature 294, 763-765 (1981) und J. Neurochemistry 37, 714-722 (1981) beschriebenen Methode festgestellt. Nach dieser Methode ermittelt man die Hemmung der Bindung von tritiiertem Flumazenil an die spezifischen Benzodiazepin-Rezeptoren im Ratten-Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung des tritiierten Flumazenils an die spezifischen Benzodiazepin-Rezeptoren im Ratten-Cortex bewirkt.

Konflikt-Test an der Ratte

Die Testapparatur ist eine Eintasten-Skinner-Box mit Futterpillengeber. Zur Prüfung potentieller Anxiolytika werden pro Substanz und Dosis in der Regel mindestens 8 hungrige Ratten eingesetzt. Man verwendet dabei Ratten, welche auf das bekannte Anxiolytikum Chlordiazepoxid ansprechen. Die Testsubstanzen, welche in einem Gemisch von 10 ml destilliertem Wasser und 2 Tropfen Tween 80 gelöst oder suspendiert sind, werden den Versuchstieren mit Hilfe einer Schlundsonde 30 Minuten vor dem 1-stündigen Konflikttest verabreicht. Während des Versuches, in welchem jeder Tastendruck für eine Futterpille mit einem Fuss-Schock kombiniert ist (Konflikt), wird die Anzahl Tastenbetätigungen registriert. Jedes Versuchstier dient dabei als seine eigene Kontrolle, indem es einmal mit Testsubstanz und einmal mit Kochsalzlösung vorbehandelt wird.

Die erste signifikant anxiolytisch wirksame Dosis (FSD) wird mit dem Wilcoxon-Test (Paarvergleich) bestimmt, indem die Anzahl der Tastenbetätigungen im Hauptversuch (Futterpille + Fuss-Schock, nach Vorbehandlung durch Testsubstanz) mit der Anzahl der Tastenbetätigungen im Kontrollversuch (Futterpille + Fuss-Schock, nach Vorbehandlung mit Kochsalzlösung) direkt verglichen wird.

Audiogene Anfälle

Die antikonvulsiven Eigenschaften der Verbindungen der Formel I können beispielsweise im nachfolgend beschriebenen Test nachgewiesen werden.

Die Testapparatur ist eine schallisolierte Eintasten-Box mit einer eingebauten Schallquelle. Zur Prüfung der Substanzen werden 7-11 g schwere, 21 Tage alte männliche DBA/2J-Mäuse eingesetzt. Solche Mäuse sind ein Tiermodell für Epilepsie, bei welcher Beschallung Anfälle hervorruft. Die Testsubstanzen werden als wässrige Suspensionen verschiedener Konzentration eingesetzt und den Versuchstieren 30 Minuten vor Testbeginn oral oder intraperitoneal in einer Menge von 10 ml/kg verabreicht. Die Versuchstiere werden 60 Sekunden einer Beschallung von 110 dB/14 KHz ausgesetzt; dies erzeugt bei unbehandelten Tieren Symptome wie Herumrennen, klonische Anfälle und tonische Konvulsionen. Man bestimmt den $ED_{50}$-Wert, d.h. diejenige Dosis einer Testsubstanz, welche bei 50% der Versuchstiere die durch die Beschallung verursachten tonischen Konvulsionen verhindert.

Aufhebung der Wirkung von Meclonazepam im Rotierstab-Test

Beschreibung des Tests: Es werden weibliche Mäuse (Charles-River, Paris) von 19-21 g Gewicht verwendet. Bis 1 h vor Beginn des Versuches haben sie freien Zugang zu Futter und Trinkwasser. Mindestens 30 min vor dem Versuch werden sie in das Versuchslabor gebracht.

Beim Rotierstab-Test werden die Tiere auf einen waagrecht angeordneten, glatten Metallstab von 3 cm Durchmesser, der sich mit 2 Umdrehungen pro min dreht, aufgesetzt. Zunächst lässt man den Tieren 30 sec Gelegenheit, sich mit der Test-Situation vertraut zu machen. Anschliessend werden diejenigen Tiere ausgewählt, denen es gelingt, mindestens 1 min lang auf dem Stab zu verbleiben.

Ermittlung der Aufhebung der Meclonazepam-Wirkung: Den Tieren wird 5 mg/kg Meclonazepam als Suspension in einer 0,3-%igen, wässerigen Tween 80-Lösung intraperitoneal verabreicht. Dies verursacht eine mehrere Stunden andauernde Unfähigkeit, sich auf dem Rotierstab zu halten. 20 min später wird eine

feine Suspension des Versuchspräparates in einer 0,3-%igen Tween 80-Lösung in Wasser intravenös verabreicht. 30 min später wird bestimmt, ob sich die Tiere mindestens 1 min lang auf dem Stab halten können. Es wird die Dosis ermittelt, bei welcher sich 50% der Tiere auf dem Stab zu halten vermögen (ID50).

In der nachfolgenden Tabelle werden die Resultate dargestellt, welche mit repräsentativen Vertretern der durch die allgemeine Formel I definierten Verbindungsklasse in den vorstehend geschilderten Versuchen erhalten worden sind.

Tabelle

| Verbindung | $^3$H-Flumazenil-Bindungs-Test in vitro, $IC_{50}$ nmol/l | Konflikt-Test Ratte, FSD mg/kg p.o. | Audiogene Anfälle Maus, $ED_{50}$ mg/kg p.o. | Anti-Meclonazepam, Rotierstab Maus, $ED_{50}$ mg/kg p.o. |
|---|---|---|---|---|
| A | 1,3 | 0,3 | - | 1,5 |
| B | 2,5 | 0,1 | 0,27 | 0,8 |
| C | 0,91 | 0,003 | 0,015 | 0,17 |
| D | 0,33 | 3,0 | 4,2 | 0,08 |
| E | 2,9 | 1,0 | 0,21 | - |
| F | 3,0 | 0,1 | 0,11 | 1,8 |
| G | 0,6 | 3,0 | - | 0,0047 |
| H | 0,29 | 0,001 | 0,0039 | 0,1 |
| I | 0,3 | 0,03 | 0,00011 | - |

A= 10-(3-Cyclopropyl-1,2,4-oxadiazol-5-yl)-9H-imidazo[1,5-a][1,2,4]triazolo-[1,5-d][1,4]benzodiazepin

B= 10-(3-Cyclopropyl-1,2,4-oxadiazol-5-yl)-3-fluor-9H-imidazo[1,5-a][1,2,4]-triazolo[1,5-d][1,4]benzodiazepin

C= 10-(5-Cyclopropyl-1,2,4-oxadiazol-3-yl)-3-fluor-9H-imidazo[1,5-a][1,2,4]-triazolo[1,5-d][1,4]benzodiazepin

D = 10-(5-Cyclopropyl-1,2,4-oxadiazol-3-yl)--9H-imidazo[1,5-a][1,2,4]triazolo-[1,5-d][1,4]benzodiazepin

E = 3-Chlor-10-(5-cyclopropyl-1,2,4-oxadiazol-3-yl)-9H-diimidazo-[1,5-a:1',2'-d][1,4]benzodiazepin

F = 10-[5-(Ethoxymethyl)-1,2,4-oxadiazol-3-yl]-3-fluor-9H-imidazo[1,5-a]-[1,2,4]triazolo[1,5-d][1,4]benzodiazepin

G = 4-Chlor-10-(5-cyclopropyl-1,2,4-oxadiazol-3-yl)-9H-imidazo[1,5-a][1,2,4]-triazolo[1,5-d][1,4]benzodiazepin

H = 4-Chlor-10-(3-cyclopropyl-1,2,4-oxadiazol-5-yl)-9H-diimidazo-[1,5-a:1',2'-d][1,4]benzodiazepin

I = 4-Chlor-10-(5-cyclopropyl-1,2,4-oxadiazol-3-yl)-9H-diimidazo-[1,5-a:1',2'-d][1,4]benzodiazepin

Die Verbindungen der Formel I und ihre pharmazeutisch annehmbaren Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen. Für Injektionslösungen eignen sich als Träger beispielsweise Wasser, Alkohole, Polyole, Glyzerin, pflanzliche Oele und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Wie eingangs erwähnt sind Arzneimittel, enthaltend eine Verbindung der Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der Formel I oder pharmazeutisch annehmbare Säureadditionssalze davon und gegebenenfalls einen oder mehrere andere therapeutisch wertvolle Stoffe in eine galenische Darreichungsform bringt.

Wie eingangs erwähnt, können die Verbindungen der Formel I und ihre pharmazeutisch annehmbaren Säureadditionssalze bei der Bekämpfung bzw. Verhütung von Krankheiten verwendet werden und zwar insbesondere bei der Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 0,1 mg bis 100 mg angemessen sein.

Die nachfolgenden Beispiele sollen die vorliegende Erfindung näher erläutern, ihren Umfangjedoch in keiner Weise beschränken. Sämtliche Temperaturen sind in Celsiusgraden angegeben.

Beispiel 1

1) 1.1. 177 g 2-Aminobenzonitril und 135,9 g Chloracetonitril werden in 1,5 l absolutem Dioxan gelöst

und die Lösung auf 5° C abgekühlt. Anschliessend wird während 7 h ein schwacher Strom von trockenem Salzsäure-Gas bei 5° C eingeleitet. Man rührt 15 h bei Raumtemperatur weiter und kühlt sodann wieder auf 5° C ab. Man gibt nochmals 68,0 g Chloracetonitril hinzu, leitet abermals während 7 h Salzsäure-Gas ein, und rührt 15 h bei Raumtemperatur. Die Suspension wird anschliessend im Vakuum bei 30° C eingedampft. Der kristalline Rückstand wird mit 2,5 l Wasser verrührt, auf 0° bis 5° C abgekühlt und filtriert. Die Kristalle werden mit Wasser gewaschen und im Vakuum getrocknet. Man erhält 245 g bis 288 g 4-Chlor-2-(chlormethyl)chinazolin vom Smp. 99 - 101° C. Ausbeute: 76,5 bis 90%.

Auf analoge Weise erhält man:

1) 1.2. 4,5-Dichlor-2-(chlormethyl)chinazolin, Smp. 140-143° C, aus 6-Chlor-2-aminobenzonitril;

1) 1.3. 4-Chlor-2-(chlormethyl)-6-fluorchinazolin, Smp. 123-124° C, aus 5-Fluor-2-aminobenzonitril;

1) 1.4. 4,6-Dichlor-2-(chlormethyl)chinazolin, Smp. 97-98° C, aus 5-Chlor-2-aminobenzonitril.

Literatur: C.J.Shishoo et al., Tetrahedron Lett., 1983, 4611-4612.

1) 2.1. 46,0 g 4-Chlor-2-(chlormethyl)chinazolin werden in 600 ml absolutem Tetrahydrofuran suspendiert. Die Lösung wird auf 0° C abgekühlt. Es werden 23,0 ml Hydrazinhydrat innerhalb von 5 min zugetropft, wobei eine Lösung entsteht und die Temperatur auf 15° bis 20° C ansteigt. Man rührt 4 h bei Raumtemperatur und dampft dann das Reaktionsgemisch im Vakuum ein. Der Rückstand wird mit 0,5 l Dichlormethan und 1 l einer gesättigten wässerigen Lösung von Natriumhydrogencarbonat verrührt und dann filtriert. Die Kristalle werden mit Wasser neutral gewaschen und im Vakuum getrocknet. Man erhält 38 g 2-(Chlormethyl)-4-hydrazinochinazolin. Die Dichlormethan-Phase des Filtrats wird abgetrennt und die wässerige Phase mit Dichlormethan extrahiert. Durch Abdampfen der organischen Phasen erhält man weitere 5,8 g 2-(Chlormethyl)-4-hydrazinochinazolin. Insgesamt erhält man 43,8 g 2-(Chlormethyl)-4-hydrazinochinazolin. Smp. 192° C (Zers.).

Auf analoge Weise erhält man:

1) 2.2. 5-Chlor-2-(chlormethyl)-4-hydrazinochinazolin, Smp. 128-129° C, aus 4,5-Dichlor-2-(chlormethyl)-chinazolin;

1) 2.3. 2-(Chlormethyl)-6-fluor-4-hydrazinochinazolin, Smp. über 162° C (Zers.), aus 6-Fluor-4-chlor-2-(chlormethyl)chinazolin;

1) 2.4. 6-Chlor-2-(chlormethyl)-4-hydrazinochinazolin, Smp. über 160° C (Zers.), aus 4,6-Dichlor-2-(chlormethyl)chinazolin.

1) 3.1. 88 g 2-(Chlormethyl)-4-hydrazinochinazolin werden in 1,2 l Orthoameisensäuretriethylester suspendiert. Die Suspension wird unter Rühren aufgeheizt (Badtemp. 125° C) und das entstehende Ethanol abdestilliert. Nach 1 h kühlt man auf 15° C ab, filtriert den Niederschlag ab, und wäscht ihn mit 100 ml Diethylether. Das Produkt wird im Vakuum getrocknet. Man erhält 80 g rohes 5-(Chlormethyl)-1,2,4-triazolo[4,3-c]chinazolin. Das Filtrat wird im Vakuum eingeengt und der Rückstand in 20 ml Ethanol aufgekocht. Man kühlt auf 0° C ab, filtriert, wäscht den Filterkuchen mit Diethylether und trocknet die Kristalle im Vakuum. Man erhält eine zweite Portion von 5,3 g rohem 5-(Chlormethyl)-1,2,4-triazolo[4,3-c]chinazolin. Die beiden Rohkristallisate (zusammen 85,3 g) werden in 8,5 l Dioxan 1 h bei 85° C gerührt. Der unlösliche Teil wird abfiltriert und das Filtrat im Vakuum bis auf ca. 300 ml eingeengt. Die Suspension wird abgekühlt, filtriert und der Filterkuchen mit 100 ml Diethylether gewaschen. Man erhält 66 g 5-(Chlormethyl)-1,2,4-triazolo[4,3-c]chinazolin vom Smp. 189° C (Zers.). Nach Einengen des Filtrates erhält man eine zweite Portion von 2,5 g 5-(Chlormethyl)-1,2,4-triazolo[4,3-c]chinazolin.

Auf analoge Weise erhält man:

1) 3.2. 10-Chlor-5-(chlormethyl)-1,2,4-triazolo[4,3-c]chinazolin, Smp. 184-186° C, aus 5-Chlor-2-(chlormethyl)-4-hydrazinochinazolin;

1) 3.3. 5-(Chlormethyl)-9-fluor-1,2,4-triazolo[4,3-c]chinazolin, Smp. 183° C (Zers.), aus 2-(Chlormethyl)-6-fluor-4-hydrazinochinazolin;

1) 3.4. 9-Chlor-5-(chlormethyl)-1,2,4-triazolo[4,3-c]chinazolin, Smp. 187-188° C, aus 6-Chlor-2-(chlormethyl)-4-hydrazinochinazolin.

1) 4.1. 77 g 5-(Chlormethyl)-1,2,4-triazolo[4,3-c]chinazolin werden in 2,0 l Aceton (oder Dioxan) suspendiert und auf 5° C abgekühlt. Man gibt 410 ml 1 N Natriumhydroxid-Lösung so zu, dass die Temperatur auf ca. 13° C steigt. Man rührt 17 h bei Raumtemperatur. Das Reaktionsgemisch wird dann mit 3 N Salzsäure schwach sauer (pH 6) gestellt und im Vakuum eingeengt. Der kristalline Niederschlag wird abfiltriert, gewaschen und getrocknet. Man erhält 65 g rohes 5H-[1,2,4]Triazolo[1,5-d][1,4]benzodiazepin-6(7H)-on. Das Filtrat wird mit Natriumchlorid gesättigt und 3 mal mit je 0,5 l Dichlormethan extrahiert. Die organischen Phasen werden im Vakuum eingedampft. Man erhält 12 g rohes 5H-[1,2,4]Triazolo[1,5-d]-[1,4]benzodiazepin-6(7H)-on. Beide Rohkristallisate (77 g) werden zusammen in 5 l Dichlormethan und 250 ml Ethanol bei Rückflusstemperatur gelöst. Ein kleiner unlöslicher Anteil wird durch Filtration abgetrennt, das Filtrat auf Raumtemperatur abgekühlt und durch eine Säule aus 1,5 kg Kieselgel filtriert.

Die Säule wird noch mit einem Gemisch aus 5,7 l Dichlormethan und 0,3 l Ethanol ausgewaschen. Nach Einengen aller Eluate erhält man 62 g gelbe Kristalle. Diese werden in 600 ml Essigsäureethylester bei Rückflusstemperatur als Suspension gerührt und dann auf 0 ° C abgekühlt. Die Kristalle werden abfiltriert, mit Diethylether gewaschen und getrocknet. Man erhält 51 g 5H-[1,2,4]Triazolo[1,5-d][1,4]benzodiazepin-6(7H)-on vom Smp. 223-225 ° C.

Auf analoge Weise erhält man:

1) 4.2. 11-Chlor-5H-[1,2,4]triazolo[1,5-d][1,4]benzodiazepin-6(7H)-on, Smp. 280-282 ° C, aus 10-Chlor-5-(chlormethyl)-1,2,4-triazolo[4,3-c]chinazolin;

1) 4.3. 10-Fluor-5H-[1,2,4]triazolo[1,5-d][1,4]benzodiazepin-6(7H)-on, Smp. 243-245 ° C, aus 9-Fluor-5-(chlormethyl)-1,2,4-triazolo[4,3-c]chinazolin;

1) 4.4. 10-Chlor-5H-[1,2,4]triazolo[1,5-d][1,4]benzodiazepin-6(7H)-on, Smp. 227-228 ° C, aus 9-Chlor-5-(chlormethyl)-1,2,4-triazolo[4,3-c]chinazolin.

Literatur: Breuer, Tetrahedron Lett., 1976, 1935-1938

1) 5.1. 16 g 5H-[1,2,4]Triazolo[1,5-d][1,4]benzodiazepin-6(7H)-on werden in 1 l Chloroform und 60 ml N,N,4-Trimethylanilin suspendiert. Man gibt 23 ml Phosphoroxychlorid dazu, und rührt das Gemisch 16,5 h bei Rückflusstemperatur. Man gibt nochmals 12 ml N,N,4-Trimethylanilin und 4 ml Phosphoroxychlorid hinzu und erhitzt weitere 1,5 h auf Rückflusstemperatur. Das abgekühlte Reaktionsgemisch wird in 4 l gesättigte wässerige Natriumhydrogencarbonat-Lösung gegossen und 30 min intensiv gerührt. Die wässerige Phase wird abgetrennt und noch 2 mal mit je 0,5 l Chloroform extrahiert. Die vereinigten Chloroform-Extrakte werden im Vakuum eingedampft. Der Rückstand besteht aus einem Gemisch von 6-Chlor-5H-[1,2,4]-triazolo[1,5-d][1,4]benzodiazepin und N,N,4-Trimethylanilin, das in 100 ml Tetrahydrofuran gelöst wird.

Eine Lösung von 15,3 g Isocyanessigsäure-tert-butylester in 480 ml Tetrahydrofuran wird auf-25 ° C gekühlt. Es werden 13,4 g Kalium-tert-butylat zugegeben. Man rührt diese Lösung 1 h bei -10 ° C, kühlt sie auf-60 ° C und versetzt sie mit der Lösung von 6-Chlor-5H-[1,2,4]triazolo[1,5-d][1,4]benzodiazepin und N,N,4-Trimethylanilin, wobei die Temperatur auf-15 ° C ansteigt. Das Reaktionsgemisch wird noch 2,5 h bei Raumtemperatur weiter gerührt und sodann in 5 l gesättigte wässerige Natriumchlorid-Lösung gegossen. Man extrahiert viermal mit Chloroform. Die organischen Extrakte werden im Vakuum eingeengt. Der Rückstand wird in Chloroform gelöst und durch Kieselgel chromatographiert. Mit Chloroform/Ethanol (99,8:0,2 bis 99:1) werden 18,6 g roher 9H-Imidazo[1,5-a][1,2,4]triazolo]1,5-d][1,4]-benzodiazepin-10-carbonsäure-tert-butylester eluiert. Dieser wird aus Essigsäureethylester/Diisopropylether umkristallisiert. Man erhält 15,1 g 9H-Imidazo[1,5-a][1,2,4]triazolo-[1,5-d][1,4]benzodiazepin-10-carbonsäure-tert-butylester vom Smp. 247-249 ° C (Zers.).

Auf analoge Weise erhält man:

1) 5.2. 4-Chlor-9H-imidazo[1,5-a][1,2,4]triazolo[1,5-d][1,4]benzodiazepin-10-carbonsäure-tert-butylester, Smp. 271 ° C, aus 11-Chlor-5H-[1,2,4]triazolo[1,5-d][1,4]benzodiazepin-6(7H)-on;

1) 5.3. 3-Fluor-9H-imidazo[1,5-a][1,2,4]triazolo[1,5-d][1,4]benzodiazepin-10-carbonsäure-tert-butylester, Smp. 192-193 ° C, aus 10-Fluor-5H-[1,2,4]triazolo[1,5-d][1,4]benzodiazepin-6(7H)-on;

1) 5.4. 3-Chlor-9H-imidazo[1,5-a][1,2,4]triazolo[1,5-d][1,4]benzodiazepin-10-carbonsäure-tert-butylester, Smp. 217-218 ° C, aus 10-Chlor-5H-[1,2,4]triazolo[1,5-d][1,4]benzodiazepin-6(7H)-on.

1) 6.1. 9,5 g 9H-Imidazo[1,5-a][1,2,4]triazolo[1,5-d][1,4]benzodiazepin-10-carbonsäure-tert-butylester werden in 200 ml Trifluoressigsäure gelöst und 5 h bei Raumtemperatur stehen gelassen. Man dampft die Trifluoressigsäure im Vakuum ab. Der Rückstand wird aus Essigsäureethylester umkristallisiert. Man erhält 7,5 g 9H-Imidazo-[1,5-a][1,2,4]triazolo[1,5-d][1,4]benzodiazepin-10-carbonsäure vom Smp. 283-285 ° C.

Auf analoge Weise erhält man:

1) 6.2. 4-Chlor-9H-imidazo[1,5-a][1,2,4]triazolo[1,5-d][1,4]benzodiazepin-10-carbonsäure` Smp. 276-277 ° C(Zers.), aus 4-Chlor-9H-imidazo[1,5-a][1,2,4]triazolo[1,5-d][1,4]benzodiazepin-10-carbonsäure-tert-butylester;

1) 6.3. 3-Fluor-9H-imidazo[1,5-a][1,2,4]triazolo[1,5-d][1,4]benzodiazepin-10-carbonsäure, Smp. 276 ° C, aus 3-Fluor-9H-imidazo[1,5-a][1,2,4]triazolo[1,5-d][1,4]benzodiazepin-10-carbonsäure-tert-butylester;

1) 6.4. 3-Chlor-9H-imidazo[1,5-a][1,2,4]triazolo[1,5-d][1,4]benzodiazepin-10-carbonsäure, Smp. 270-271 ° C, aus 3-Chlor-9H-imidazo[1,5-a][1,2,4]triazolo[1,5-d][1,4]benzodiazepin-10-carbonsäure-tert-butylester.

1) 7.1. Zu einer Lösung von 10,9 g 9H-Imidazo[1,5-a][1,2,4]triazolo[1,5-d][1,4]benzodiazepin-10-carbonsäure in 700 ml Dimethylformamid werden 13,6 g 1,1'-Carbonyldiimidazol zugegeben. Man rührt dieses Gemisch 4 h bei Raumtemperatur und gibt dann 485 ml 25%-ige wässerige Ammoniak-Lösung dazu. Nach 5 min Rühren wird die Klare Lösung mit 1,2 l Wasser versetzt. Der entstandene Niederschlag wird

abfiltriert, mit Wasser gewaschen und im Vakuum getrocknet. Man erhält 10,0 g 9H-Imidazo[1,5-a][1,2,4]-triazolo[1,5-d][1,4]benzodiazepin-10-carboxamid vom Smp. 324-329 ° C.

Auf analoge Weise erhält man:

1) 7.2. 4-Chlor-9H-imidazo[1,5-a][1,2,4]triazolo[1,5-d][1,4]benzodiazepin-10-carboxamid, Smp. 311-312 ° C, aus 4-Chlor-9H-imidazo[1,5-a][1,2,4]triazolo[1,5-d]-[1,4]benzodiazepin-10-carbonsäure;

1) 7.3. 3-Fluor-9H-imidazo[1,5-a][1,2,4]triazolo[1,5-d][1,4]benzodiazepin-10-carboxamid, Smp. 340-343 ° C, aus 3-Fluor-9H-imidazo[1,5-a][1,2,4]triazolo[1,5-d][1,4]benzodiazepin-10-carbonsäure;

1) 7.4. 3-Chlor-9H-imidazo[1,5-a][1,2,4]triazolo[1,5-d][1,4]benzodiazepin-10-carboxamid, Smp. über 295 ° C, aus 3-Chlor-9H-imidazo[1,5-a][1,2,4]triazolo[1,5-d][1,4]benzodiazepin-10-carbonsäure;

1) 7.5. 3-Methyl-9H-imidazo[1,5-a][1,2,4]triazolo[1,5-d][1,4]benzodiazepin-10-carboxamid, Smp. über 300 ° C, aus 3-Methyl-9H-imidazo[1,5-a][1,2,4]triazolo[1,5-d][1,4]benzodiazepin-10-carbonsäure (hergestellt gemäss Beispiel 19) 7.).

1) 8.1. Zu einer Lösung von 10,0 g 9H-Imidazo[1,5-a][1,2,4]triazolo[1,5-d][1,4]benzodiazepin-10-carboxamid in 280 ml Tetrahydrofuran und 7,3 ml Pyridin werden 6,25 ml Trifluoressigsäureanhydrid innerhalb von 15 min bei 5 ° bis 10 ° C zugegeben, und 2 h bei Raumtemperatur gerührt. Man gibt nochmals 1,45 ml Pyridin und 1,4 ml Trifluoressigsäureanhydrid dazu. Nach 1 h Rühren bei Raumtemperatur wird das Gemisch in 2,0 l eiskalte gesättigte wässerige Natriumhydrogencarbonat-Lösung gegossen. Man extrahiert 5 mal mit Chloroform. Die Chloroform-Extrakte werden mit gesättigter wässeriger Natriumchlorid-Lösung gewaschen, getrocknet, und im Vakuum eingedampft. Der Rückstand wird aus Essigsäureethylester/Diisopropylether umkristallisiert. Man erhält 8,35 g 9H-Imidazo[1,5-a][1,2,4]triazolo-[1,5-d][1,4]benzodiazepin-10-carbonitril vom Smp. 228-232 ° C.

Auf analoge Weise erhält man:

1) 8.2. 4-Chlor-9H-imidazo[1,5-a][1,2,4]triazolo[1,5-d][1,4]benzodiazepin-10-carbonitril, Smp. 283-285 ° C, aus 4-Chlor-9H-imidazo[1,5-a][1,2,4]triazolo[1,5-d][1,4]-benzodiazepin-10-carboxamid;

1) 8.3. 3-Fluor-9H-imidazo[1,5-a][1,2,4]triazolo[1,5-d][1,4]benzodiazepin-10-carbonitril, Smp. 257-258 ° C, aus 3-Fluor-9H-imidazo[1,5-a][1,2,4]triazolo[1,5-d][1,4]benzodiazepin-10-carboxamid;

1) 8.4. 3-Chlor-9H-imidazo[1,5-a][1,2,4]triazolo[1,5-d][1,4]benzodiazepin-10-carbonitril, Smp. 255-256 ° C, aus 3-Chlor-9H-imidazo[1,5-a][1,2,4]triazolo[1,5-d][1,4]benzodiazepin-10-carboxamid;

1) 8.5. 3-Methyl-9H-imidazo[1,5-a][1,2,4]triazolo[1,5-d][1,4]benzodiazepin-10-carbonitril, Smp. 275-276 ° C, aus 3-Methyl-9H-imidazo[1,5-a][1,2,4]triazolo[1,5-d][1,4]benzodiazepin-10-carboxamid.

1) 9.1. Zu einer Suspension von 8,35 g 9H-Imidazo[1,5-a][1,2,4]triazolo[1,5-d][1,4]benzodiazepin-10-carbonitril in 160 ml Ethanol werden zunächst 2,7 g Hydroxylaminhydrochlorid, dann eine Lösung von 3,34 g Natriumhydrogencarbonat in 40 ml Wasser zugegeben. Man rührt 1,5 h bei Rückflusstemperatur. Der entstandene Niederschlag wird abfiltriert und mit Wasser gewaschen. Man erhält 7,6 g 9H-Imidazo-[1,5-a][1,2,4]triazolo[1,5-d][1,4]benzodiazepin-10-carboxamidoxim. Durch Einengen des Filtrates lassen sich weitere 0,5 g dieser Verbindung gewinnen.

Auf analoge Weise erhält man:

1) 9.2. 4-Chlor-9H-imidazo[1,5-a][1,2,4]triazolo[1,5-d][1,4]benzodiazepin-10-carboxamidoxim, Smp. 298-300 ° C, aus 4-Chlor-9H-imidazo[1,5-a][1,2,4]triazolo[1,5-d][1,4]benzodiazepin-10-carbonitril;

1) 9.3. 3-Fluor-9H-imidazo[1,5-a][1,2,4]triazolo[1,5-d][1,4]benzodiazepin-10-carboxamidoxim, Smp. 287-288 ° C, aus 3-Fluor-9H-imidazo[1,5-a][1,2,4]triazolo[1,5-d][1,4]benzodiazepin-10-carbonitril;

1) 9.4. 3-Chlor-9H-imidazo[1,5-a][1,2,4]triazolo[1,5-d][1,4]benzodiazepin-10-carboxamidoxim, Smp. 279-280 ° C, aus 3-Chlor-9H-imidazo[1,5-a][1,2,4]triazolo[1,5-d][1,4]benzodiazepin-10-carbonitril;

1) 9.5. 3-Methyl-9H-imidazo[1,5-a][1,2,4]triazolo[1,5-d][1,4]benzodiazepin-10-carboxamidoxim, Smp. 282-283 ° C, aus 3-Methyl-9H-imidazo[1,5-a][1,2,4]triazolo[1,5-d][1,4]benzodiazepin-10-carbonitril.

1) 10.1. 3,46 ml Cyclopropancarbonsäure werden in 240 ml Dimethylformamid gelöst und bei 35 ° C mit 7,0 g 1,1'-Carbonyldiimidazol versetzt. Man rührt 1 h bei 35 ° C und 2 h bei Raumtemperatur, dann gibt man 8,1 g 9H-Imidazo[1,5-a][1,2,4]triazolo[1,5-d][1,4]benzodiazepin-10-carboxamidoxim dazu und rührt 15 h bei 80 ° C. Das Reaktionsgemisch wird im Hochvakuum eingedampft. Der Rückstand wird in 50 ml Cyclopropancarbonsäure gelöst und 3,5 h auf 130 ° C erhitzt. Die Lösung wird im Vakuum eingedampft. Der Rückstand wird durch Kieselgel chromatographiert. Mit Dichlormethan/Ethanol 99:1 eluiert man 7,8 g rohes 10-(5-cyclopropyl-1,2,4-oxadiazol-3-yl)-9H-imidazo[1,5-a][1,2,4]triazolo[1,5-d][1,4]benzodiazepin. Dieses wird aus Ethanol umkristallisiert. Man erhält 7,4 g reines 10-(5-cyclopropyl-1,2,4-oxadiazol-3-yl)-9H-imidazo[1,5-a][1,2,4]triazolo[1,5-d][1,4]benzodiazepin vom Smp. 216-217 ° C.

Auf analoge Weise erhält man:

1) 10.2. 4-Chlor-10-(5-cyclopropyl-1,2,4-oxadiazol-3yl)-9H-imidazo[1,5-a][1,2,4]triazolo[1,5-d][1,4]-benzodiazepin, Smp. 266-267 ° C, aus 4-Chlor-9H-imidazo[1,5-a][1,2,4]triazolo[1,5-d][1,4]benzodiazepin-10-carboxamidoxim;

1) 10.3. 10-(5-Cyclopropyl-1,2,4-oxadiazol-3-yl)-3-fluor-9H-imidazo-[1,5-a][1,2,4]triazolo[1,5-d][1,4]-benzodiazepin, Smp. 223-224 ° C, aus 3-Fluor-9H-imidazo-[1,5-a][1,2,4]triazolo[1,5-d][1,4]benzodiazepin-10-carboxamidoxim;

1) 10.4. 3-Chlor-10-(5-cyclopropyl-1,2,4-oxadiazol-3-yl)-9H-imidazo[1,5-a][1,2,4]triazolo[1,5-d][1,4]-benzodiazepin, Smp. 261-262 ° C, aus 3-Chlor-9H-imidazo-[1,5-a][1,2,4]triazolo[1,5-d][1,4]benzodiazepin-10-carboxamidoxim;

1) 10.5. 10-(5-Cyclopropyl-1,2,4-oxadiazol-3-yl)-3-methyl-9H-imidazo[1,5-a][1,2,4]triazolo[1,5-d][1,4]-benzodiazepin, Smp. 238-239 ° C, aus 3-Methyl-9H-imidazo[1,5-a][1,2,4]triazolo[1,5-d][1,4]benzodiazepin-10-carboxamidoxim.

1) 10.6. Eine Lösung von 1,1 ml Propionsäure in 90 ml Dimethylformamid wird bei 35 ° C mit 2,3 g 1,1'-Carbonyldiimidazol versetzt. Man rührt 2 h bei Raumtemperatur, dann gibt man 3,0 g 3-Chlor-9H-imidazo[1,5-a][1,2,4]triazolo[1,5-d][1,4]benzodiazepin-10-carboxamidoxim dazu und rührt 16 h bei 80 ° C. Das Reaktionsgemisch wird im Hochvakuum eingedampft. Der Rückstand wird in 25 ml Propionsäure gelöst und 4 h auf 130 ° C erhitzt. Die Lösung wird im Vakuum eingedampft. Der Rückstand wird in Dichlormethan gelöst und zweimal mit gesättigter wässeriger Natriumhydrogencarbonat-Lösung gewaschen. Die organische Phase wird im Vakuum eingedampft. Der Rückstand (3,1 g) wird durch Kieselgel chromatographiert. Mit Dichlormethan/Essigsäureethylester (8:2 bis 6:4) eluiert man 3,0 g rohes 3-Chlor-10-(5-ethyl-1,2,4-oxadiazol-3-yl)-9H-imidazo[1,5-a][1,2,4]triazolo[1,5-d][1,4]benzodiazepin. Dieses wird aus Dichlormethan/Essigsäureethylester umkristallisiert. Man erhält 2,9 g reines 3-Chlor-10-(5-ethyl-1,2,4-oxadiazol-3-yl)-9H-imidazo[1,5-a][1,2,4]triazolo[1,5-d][1,4]benzodiazepin vom Smp. 267-269 ° C.

Auf analoge Weise erhält man:

1) 10.7. 3-Fluor-10-(5-methyl-1,2,4-oxadiazol-3-yl)-9H-imidazo[1,5-a][1,2,4]triazolo[1,5-d][1,4]-benzodiazepin, Smp. 293-294 ° C, aus 3-Fluor-9H-imidazo[1,5-a][1,2,4]triazolo[1,5-d][1,4]benzodiazepin-10-carboxamidoxim und Essigsäure;

1) 10.8. 3-Fluor-10-(5-isopropyl-1,2,4-oxadiazol-3-yl)-9H-imidazo[1,5-a][1,2,4]triazolo[1,5-d][1,4]-benzodiazepin, Smp. 207-209 ° C, aus 3-Fluor-9H-imidazo[1,5a][1,2,4]triazolo[1,5-d][1,4]benzodiazepin-10-carboxamidoxim und Isobuttersäure;

1) 10.9. 3-Chlor-10-(5-isopropyl-1,2,4-oxadiazol-3-yl)-9H-imidazo[1,5-a][1,2,4]triazolo[1,5-d][1,4]-benzodiazepin, Smp. 222-223 ° C, aus 3-Chlor-9H-imidazo[1,5-a][1,2,4]triazolo[1,5-d][1,4]benzodiazepin-10-carboxamidoxim und Isobuttersäure;

1) 10.10. 10-(5-Allyl-1,2,4-oxadiazol-3-yl)-3-fluor-9H-imidazo[1,5-a][1,2,4]triazolo[1,5-d][1,4]benzodiazepin, Smp. 290-292 ° C, aus 3-Fluor-9H-imidazo[1,5-a][1,2,4]triazolo[1,5-d][1,4]benzodiazepin-10-carboxamidoxim und Vinylessigsäure.

1) 10.11. 1,9 ml 3-Methoxypropionsäure werden in 100 ml Dimethylformamid gelöst und mit 3,2 g 1,1'-Carbonyldiimidazol versetzt. Man rührt 3 h bei Raumtemperatur, dann gibt man 4,0 g 3-Fluor-9H-imidazo[1,5-a][1,2,4]triazolo[1,5-d][1,4]benzodiazepin-10-carboxamidoxim dazu und rührt 18 h bei 80 ° C. Das Reaktionsgemisch wird im Hochvakuum eingedampft. Der Rückstand wird in 50 ml Essigsäure gelöst und 5 h auf Rückflusstemperatur erhitzt. Die Lösung wird im Vakuum eingedampft. Der Rückstand wird in Dichlormethan gelöst und mit gesättigter wässeriger Natriumhydrogencarbonat-Lösung gewaschen. Die organische Phase wird im Vakuum eingedampft. Der Rückstand wird durch Kieselgel chromatographiert. Mit Dichlormetham/Methanol (99:1 bis 97:3) eluiert man 3,6 g Produkt, welches aus Essigsäureethylester umkristallisiert wird. Man erhält 3,1 g reines 10-[5-(2-methoxyethyl)-1,2,4-oxadiazol-3-yl]-3-fluor-9H-imidazo[1,5-a][1,2,4]triazolo[1,5-d][1,4]benzodiazepin vom Smp. 196-197 ° C.

Auf analoge Weise erhält man:

1) 10.12 3-Fluor-10-(5-vinyl-1,2,4-oxadiazol-3-yl)-9H-imidazo[1,5-a][1,2,4]triazolo[1,5-d][1,4]benzodiazepin, Smp. 270-272 ° C, aus 3-Fluor-9H-imidazo[1,5-a][1,2,4]triazolo[1,5-d][1,4]benzodiazepin-10-carboxamidoxim und Acrylsäure;

1) 10.13. 10-[5-(Ethoxymethyl)-1,2,4-oxadiazol-3-yl]-3-fluor-9H-imidazo[1,5-a][1,2,4]triazolo[1,5-d][1,4]-benzodiazepin, Smp. 187-188 ° C, aus 3-Fluor-9H-imidazo[1,5-a][1,2,4]triazolo[1,5-d][1,4]benzodiazepin-10-carboxamidoxim und Ethoxyessigsäure;

1) 10.14. 10-[5-[2-(Benzyloxy)ethyl]-1,2,4-oxadiazol-3-yl]-3-fluor-9H-imidazo[1,5-a][1,2,4]triazolo[1,5-d]-[1,4]benzodiazepin, Smp. 123-124 ° C, aus 3-Fluor-9H-imidazo[1,5-a][1,2,4]triazolo[1,5-d][1,4]-benzodiazepin-10-carboxamidoxim und 3-(Benzyloxy)propionsäure;

1) 10.15. 3-(3-Fluor-9H-imidazo[1,5-a][1,2,4]triazolo[1,5-d][1,4]benzodiazepin-10-yl)-α,α-dimethyl-1,2,4-oxadiazol-5-methanol, Smp. 248-249 ° C, aus 3-Fluor-9H-imidazo[1,5-a][1,2,4]triazolo[1,5-d][1,4]-benzodiazepin-10-carboxamidoxim und 2-Methyl-2-hydroxypropionsäure;

1) 10.16. 3-[3-(3-Fluor-9H-imidazo[1,5-a][1,2,4]triazolo[1,5-d][1,4]benzodiazepin-10-yl)-1,2,4-oxadiazol-5-yl]propiophenon, Smp. 178-179 ° C, aus 3-Fluor-9H-imidazo[1,5-a][1,2,4]triazolo[1,5-d][1,4]benzodiazepin-10-carboxamidoxim und 3-Benzoylpropionsäure;

1) 10.17. 10-[5-(2-Chlor-1,1-dimethylethyl)-1,2,4-oxadiazol-3-yl]-3-fluor-9H-imidazo[1,5-a][1,2,4]triazolo-[1,5-d][1,4]benzodiazepin, Smp. 192-193°C, aus 3-Fluor-9H-imidazo[1,5-a][1,2,4]triazolo[1,5-d][1,4]-benzodiazepin-10-carboxamidoxim und 3-Chlor-2,2-dimethylpropionsäure;

1) 10.18. 10-[5-[(Dimethylamino)methyl]-1,2,4-oxadiazol-3-yl]-3-fluor-9H-imidazo-[1,5-a][1,2,4]triazolo[1,5-d][1,4]benzodiazepin, Smp. 196-197°C, aus 3-Fluor-9H-imidazo[1,5-a][1,2,4]triazolo[1,5-d][1,4]-benzodiazepin-10-carboxamidoxim und N,N-Dimethylglycin;

1) 10.19. 3-Fluor-10-[5-(2,6-xylidinomethyl)-1,2,4-oxadiazol-3-yl]-9H-imidazo-[1,5-a][1,2,4]triazolo[1,5-d]-[1,4]benzodiazepin, Smp. 201-205°C, aus 3-Fluor-9H-imidazo[1,5-a][1,2,4]triazolo[1,5-d][1,4]-benzodiazepin-10-carboxamidoxim und N-(2,6-Dimethylphenyl)glycin;

1) 10.20. N-[[3-(3-Fluor-9H-imidazo[1,5-a][1,2,4]triazolo[1,5-d][1,4]benzodiazepin-10-yl)-1,2,4-oxadiazol-5-yl]methyl]acetamid, Smp. 228-229°C, aus 3-Fluor-9H-imidazo[1,5-a][1,2,4]triazolo[1,5-d][1,4]-benzodiazepin-10-carboxamidoxim und N-Acetylglycin;

1) 10.21. N-[[3-(3-Fluor-9H-imidazo[1,5-a][1,2,4]triazolo[1,5-d][1,4]benzodiazepin-10-yl)-1,2,4-oxadiazol-5-yl]methyl]phthalimid, Smp. 265-266°C, aus 3-Fluor-9H- imidazo[1,5-a][1,2,4]triazolo[1,5-d][1,4]-benzodiazepin-10-carboxamidoxim und N,N-Phthaloylglycin;

1) 10.22. 3-Fluor-10-[5-(imidazol-1-ylmethyl)-1,2,4-oxadiazol-3-yl]-9H-imidazo[1,5-a][1,2,4]triazolo[1,5-d]-[1,4]benzodiazepin,Smp. 219-220°C, aus 3-Fluor-9H-imidazo[1,5-a][1,2,4]triazolo[1,5-d][1,4]-benzodiazepin-10-carboxamidoxim und 1H-Imidazol-1-essigsäure;

1) 10.23. 3-Fluor-10-[5-(2-thenyl)-1,2,4-oxadiazol-3-yl]-9H-imidazo[1,5-a][1,2,4]triazolo[1,5-d][1,4]-benzodiazepin, Smp. 182-183°C, aus 3-Fluor-9H-imidazo[1,5-a][1,2,4]triazolo[1,5-d][1,4]benzodiazepin-10-carboxamidoxim und Thiophen-2-essigsäure;

1) 10.24. 3-Fluor-10-[5-(2-imidazol-1-ylethyl)-1,2,4-oxadiazol-3-yl]-9H-imidazo-[1,5-a][1,2,4]triazolo[1,5-d]-[1,4]benzodiazepin, Smp. 196-198°C, aus 3-Fluor-9H-imidazo[1,5-a][1,2,4]triazolo[1,5-d][1,4]-benzodiazepin-10-carboxamidoxim und 1H-Imidazol-1-propionsäure;

1) 10.25. rac-5-[2-[3-(3-Fluor-9H-imidazo[1,5-a][1,2,4]triazolo[1,5-d][1,4]benzodiazepin-10-yl)-1,2,4-oxadiazol-5-yl]ethyl]-2-pyrrolidinon, Smp. 211-212°C, aus 3-Fluor-9H-imidazo[1,5-a][1,2,4]triazolo[1,5-d]-[1,4]benzodiazepin-10-carboxamidoxim und rac-5-Oxo-2-pyrrolidinpropionsäure;

1) 10.26. N-[2-[3-(3-Fluor-9H-imidazo[1,5-a][1,2,4]triazolo[1,5-d][1,4]benzodiazepin-10-yl)-1,2,4-oxadiazol-5-yl]ethyl]phthalimid, Smp. 258-259°C, aus 3-Fluor-9H-imidazo[1,5-a][1,2,4]triazolo[1,5-d][1,4]-benzodiazepin-10-carboxamidoxim und N,N-Phthaloyl-β-alanin;

1) 10.27. 3-Fluor-10-[5-(2-furyl)-1,2,4-oxadiazol-3-yl]-9H-imidazo[1,5-a][1,2,4]triazolo[1,5-d][1,4]-benzodiazepin, Smp. über 295°C, aus 3-Fluor-9H-imidazo[1,5-a][1,2,4]triazolo[1,5-d][1,4]benzodiazepin-10-carboxamidoxim und Furan-2-carbonsäure;

1) 10.28. 3-Fluor-10-[5-(3-furyl)-1,2,4-oxadiazol-3-yl]-9H-imidazo[1,5-a][1,2,4]triazolo[1,5-d][1,4]-benzodiazepin, Smp. 293-296°C, aus 3-Fluor-9H-imidazo[1,5-a][1,2,4]triazolo[1,5-d][1,4]benzodiazepin-10-carboxamidoxim und Furan-3-carbonsäure;

1) 10.29. 3-Fluor-10-[5-(2-thienyl)-1,2,4-oxadiazol-3-yl]-9H-imidazo[1,5-a][1,2,4]triazolo[1,5-d][1,4]-benzodiazepin, Smp. über 315°C, aus 3-Fluor-9H-imidazo[1,5-a][1,2,4]triazolo[1,5-d][1,4]benzodiazepin-10-carboxamidoxim und Thiophen-2-carbonsäure;

1) 10.30. 3-Fluor-10-[5-(3-thienyl)-1,2,4-oxadiazol-3-yl]-9H-imidazo[1,5-a][1,2,4]triazolo[1,5-d][1,4]-benzodiazepin, Smp. über 315°C, aus 3-Fluor-9H-imidazo[1,5a][1,2,4]triazolo[1,5-d][1,4]benzodiazepin-10-carboxamidoxim und Thiophen-3-carbonsäure;

1) 10.31. rac-3-Fluor-10-[5-(tetrahydro-2-furyl)-1,2,4-oxadiazol-3-yl]-9H-imidazo[1,5-a][1,2,4]triazolo[1,5-d]-[1,4]benzodiazepin, Smp. 181-182°C, aus 3-Fluor-9H-imidazo[1,5-a][1,2,4]triazolo[1,5-d][1,4]-benzodiazepin-10-carboxamidoxim und rac-Tetrahydrofuran-2-carbonsäure;

1) 10.32. 3-Fluor-10-[5-(4-oxazolyl)-1,2,4-oxadiazol-3-yl]-9H-imidazo[1,5-a][1,2,4]triazolo[1,5-d][1,4]-benzodiazepin, Smp. 301-302°C, aus 3-Fluor-9H-imidazo[1,5-a][1,2,4]triazolo[1,5-d][1,4]benzodiazepin-10-carboxamidoxim und Oxazol-4-carbonsäure;

1) 10.33. 3-Fluor-10-[5-(3-methyl-5-isoxazolyl)-1,2,4-oxadiazol-3-yl]-9H-imidazo[1,5-a][1,2,4]triazolo[1,5-d]-[1,4]benzodiazepin, Smp. 303-304°C, aus 3-Fluor-9H-imidazo[1,5-a][1,2,4]triazolo[1,5-d][1,4]-benzodiazepin-10-carboxamidoxim und 3-Methylisoxazol-5-carbonsäure;

1) 10.34. (S)-5-[3-(3-Fluor-9H-imidazo[1,5-a][1,2,4]triazolo[1,5-d][1,4]benzodiazepin-10-yl)-1,2,4-oxadiazol-5-yl]-2-pyrrolidinon, Smp. 229-232°C, aus 3-Fluor-9H-imidazo[1,5-a][1,2,4]triazolo[1,5-d][1,4]-benzodiazepin-10-carboxamidoxim und 5-Oxo-L-prolin;

1) 10.35. 3-Chlor-10-[5-(2-methoxyethyl)-1,2,4-oxadiazol-3-yl]-9H-imidazo[1,5-a][1,2,4]triazolo[1,5-d][1,4]-benzodiazepin, Smp. 190-192°C, aus 3-Chlor-9H-imidazo[1,5-a][1,2,4]triazolo[1,5-d][1,4]benzodiazepin-10-carboxamidoxim und 3-Methoxypropionsäure;

1) 10.36. 10-[5-[2-(Benzyloxy)ethyl]-1,2,4-oxadiazol-3-yl]-3-chlor-9H-imidazo[1,5-a][1,2,4]triazolo[1,5-d]-[1,4]benzodiazepin, Smp. 143-145 ° C, aus 3-Chlor-9H-imidazo[1,5-a][1,2,4]triazolo[1,5-d][1,4]-benzodiazepin-10-carboxamidoxim und 3-(Benzyloxy)propionsäure;

1) 10.37. 3-Chlor-10-[5-[(2,6-xylidino)methyl]-1,2,4-oxadiazol-3-yl]-9H-imidazo[1,5-a][1,2,4]triazolo[1,5-d]-[1,4]benzodiazepin,Smp. 236-237 ° C, aus 3-Chlor-9H-imidazo[1,5-a][1,2,4]triazolo[1,5-d][1,4]-benzodiazepin-10-carboxamidoxim und N-2,6-Xylylglycin;

1) 10.38. 3-Fluor-10-(5-benzyl-1,2,4-oxadiazol-3-yl)--9H-imidazo[1,5-a][1,2,4]triazolo[1,5-d][1,4]-benzodiazepin, Smp. 182-183 ° C, aus 3-Fluor-9H-imidazo[1,5-a][1,2,4]triazolo[1,5-d][1,4]benzodiazepin-10-carboxamidoxim und Phenylessigsäure;

1) 10.39. 3-Fluor-10-(5-phenethyl-1,2,4-oxadiazol-3-yl)-9H-imidazo[1,5-a][1,2,4]triazolo[1,5-d][1,4]-benzodiazepin, Smp. 180-181 ° C, aus 3-Fluor-9H-imidazo[1,5-a][1,2,4]triazolo[1,5-d][1,4]benzodiazepin-10-carboxamidoxim und 3-Phenylpropionsäure;

1) 10.40. 3-Fluor-10-[5-(3-phenylpropyl)-1,2,4-oxadiazol-3-yl]--9H-imidazo[1,5-a][1,2,4]triazolo[1,5-d][1,4]-benzodiazepin, Smp. 180-181 ° C, aus 3-Fluor-9H-imidazo[1,5-a][1,2,4]triazolo[1,5-d][1,4]benzodiazepin-10-carboxamidoxim und 4-Phenylbuttersäure;

1) 10.41. 3-Fluor-10-[5-(2-fluorbenzyl)-1,2,4-oxadiazol-3-yl]--9H-imidazo[1,5-a][1,2,4]triazolo[1,5-d][1,4]-benzodiazepin, Smp. 202-204 ° C, aus 3-Fluor-9H-imidazo[1,5-a][1,2,4]triazolo[1,5-d][1,4]benzodiazepin-10-carboxamidoxim und 2-Fluorphenylessigsäure;

1) 10.42. 3-Fluor-10-[5-(3-fluorbenzyl)-1,2,4-oxadiazol-3-yl]--9H-imidazo[1,5-a][1,2,4]triazolo[1,5-d][1,4]-benzodiazepin, Smp. 188-189 ° C, aus 3-Fluor-9H-imidazo[1,5-a][1,2,4]triazolo[1,5-d][1,4]benzodiazepin-10-carboxamidoxim und 3-Fluorphenylessigsäure;

1) 10.43. 3-Fluor-10-[5-(4-fluorbenzyl)-1,2,4-oxadiazol-3-yl]--9H-imidazo[1,5-a][1,2,4]triazolo[1,5-d][1,4]-benzodiazepin, Smp. 191-192 ° C, aus 3-Fluor-9H-imidazo[1,5-a][1,2,4]triazolo[1,5-d][1,4]benzodiazepin-10-carboxamidoxim und 4-Fluorphenylessigsäure;

1) 10.44. 3-Fluor-10-[5-(2-methoxybenzyl)-1,2,4-oxadiazol-3-yl]-9H-imidazo[1,5-a][1,2,4]triazolo[1,5-d]-[1,4]benzodiazepin, Smp. 237-238 ° C, aus 3-Fluor-9H-imidazo[1,5-a][1,2,4]triazolo[1,5-d][1,4]-benzodiazepin-10-carboxamidoxim und 2-Methoxyphenylessigsäure;

1) 10.45. 3-Fluor-10-[5-(3-methoxybenzyl)-1,2,4-oxadiazol-3-yl]-9H-imidazo[1,5-a][1,2,4]triazolo[1,5-d]-[1,4]benzodiazepin, Smp. 174-175 ° C, aus 3-Fluor-9H-imidazo[1,5-a][1,2,4]triazolo[1,5-d][1,4]-benzodiazepin-10-carboxamidoxim und 3-Methoxyphenylessigsäure;

1) 10.46. 3-Fluor-10-[5-(4-methoxybenzyl)-1,2,4-oxadiazol-3-yl]-9H-imidazo[1,5-a][1,2,4]triazolo[1,5-d]-[1,4]benzodiazepin, Smp. 205-206 ° C, aus 3-Fluor-9H-imidazo[1,5-a][1,2,4]triazolo[1,5-d][1,4]-benzodiazepin-10-carboxamidoxim und 4-Methoxyphenylessigsäure;

1) 10.47. 3-Fluor-10-[5-(3,4-methylendioxybenzyl)-1,2,4-oxadiazol-3-yl]-9H-imidazo[1,5-a][1,2,4]triazolo-[1,5-d][1,4]benzodiazepin,Smp. 237-238 ° C, aus 3-Fluor-9H-imidazo[1,5-a][1,2,4]triazolo[1,5-d][1,4]-benzodiazepin-10-carboxamidoxim und 3,4-Methylendioxyphenylessigsäure;

1) 10.48. 3-Fluor-10-[5-(2-methylbenzyl)-1,2,4-oxadiazol-3-yl]-9H-imidazo[1,5-a][1,2,4]triazolo[1,5-d][1,4]-benzodiazepin, Smp. 203-204 ° C, aus 3-Fluor-9H-imidazo[1,5-a][1,2,4]triazolo[1,5-d][1,4]benzodiazepin-10-carboxamidoxim und o-Tolylessigsäure;

1) 10.49. 3-Fluor-10-[5-(3-methylbenzyl)-1,2,4-oxadiazol-3-yl]-9H-imidazo[1,5-a][1,2,4]triazolo[1,5-d][1,4]-benzodiazepin, Smp. 182-183 ° C, aus 3-Fluor-9H-imidazo[1,5-a][1,2,4]triazolo[1,5-d][1,4]benzodiazepin-10-carboxamidoxim und m-Tolylessigsäure;

1) 10.50. 3-Fluor-10-[5-(4-methylbenzyl)-1,2,4-oxadiazol-3-yl]9H-imidazo[1,5-a][1,2,4]triazolo[1,5-d][1,4]-benzodiazepin, Smp. 233-234 ° C, aus 3-Fluor-9H-imidazo[1,5-a][1,2,4]triazolo[1,5-d][1,4]benzodiazepin-10-carboxamidoxim und p-Tolylessigsäure;

1) 10.51. 3-Fluor-10-[5-(3-trifluormethylbenzyl)-1,2,4-oxadiazol-3-yl]-9H-imidazo[1,5-a][1,2,4]triazolo[1,5-d][1,4]benzodiazepin,Smp. 128-129 ° C, aus 3-Fluor-9H-imidazo[1,5-a][1,2,4]triazolo[1,5-d][1,4]-benzodiazepin-10-carboxamidoxim und 3-Trifluormethyl-phenylessigsäure;

1) 10.52. 3-Fluor-10-[5-(4-trifluormethylbenzyl)-1,2,4-oxadiazol-3-yl]-9H-imidazo[1,5-a][1,2,4]triazolo[1,5-d][1,4]benzodiazepin,Smp. 206-207 ° C, aus 3-Fluor-9H-imidazo[1,5-a][1,2,4]triazolo[1,5-d][1,4]-benzodiazepin-10-carboxamidoxim und 4-Trifluormethyl-phenylessigsäure;

1) 10.53. 3-Fluor-10-[5-(4-trifluormethoxybenzyl)-1,2,4-oxadiazol-3-yl]-9H-imidazo[1,5-a][1,2,4]triazolo[1,5-d][1,4]benzodiazepin,Smp. 175-176 ° C, aus 3-Fluor-9H-imidazo[1,5-a][1,2,4]triazolo[1,5-d][1,4]-benzodiazepin-10-carboxamidoxim und 4-Trifluormethoxy-phenylessigsäure;

1) 10.54. 3-Fluor-10-(5-benzoyl-1,2,4-oxadiazol-3-yl)-9H-imidazo[1,5-a][1,2,4]triazolo[1,5-d][1,4]-benzodiazepin, Smp. 224-226 ° C, aus 3-Fluor-9H-imidazo[1,5-a][1,2,4]triazolo[1,5-d][1,4]benzodiazepin-10-carboxamidoxim und 4-Phenylglyoxylsäure;

1) 10.55. rac-3-Fluor-10-[5-(2-phenylpropyl)-[1,2,4]-oxadiazol-3-yl]-9H-imidazo[1,5-a][1,2,4]triazolo[1,5-d]-

[1,4]benzodiazepin,Smp. ca. 100°C, aus 3-Fluor-9H-imidazo[1,5-a][1,2,4]triazolo[1,5-d][1,4]-benzodiazepin-10-carboxamidoxim und rac-3-Phenylbuttersäure;

1) 10.56. 3-Fluor-10-[5-(1-phenylcyclopropyl)-[1,2,4]-oxadiazol-3-yl]-9H-imidazo[1,5-a][1,2,4]triazolo[1,5-d][1,4]benzodiazepin,Smp. 187-189°C, aus 3-Fluor-9H-imidazo[1,5-a][1,2,4]triazolo[1,5-d][1,4]-benzodiazepin-10-carboxamidoxim und 1-Phenyl-1-cyclopropancarbonsäure;

1) 10.57. 3-Fluor-10-[5-(3-thienylmethyl)-[1,2,4]-oxadiazol-3-yl]-9H-imidazo[1,5-a][1,2,4]triazolo[1,5-d][1,4]-benzodiazepin, Smp. 187-189°C, aus 3-Fluor-9H-imidazo[1,5-a][1,2,4]triazolo[1,5-d][1,4]benzodiazepin-10-carboxamidoxim und Thiophen-3-essigsäure;

1) 10.58. 10-[5-(3-Ethoxypropyl)-[1,2,4]-oxadiazol-3-yl]-3-fluor-9H-imidazo[1,5-a][1,2,4]triazolo[1,5-d][1,4]-benzodiazepin, Smp. 125-126°C, aus 3-Fluor-9H-imidazo[1,5-a][1,2,4]triazolo[1,5-d][1,4]benzodiazepin-10-carboxamidoxim und 4-Ethoxybuttersäure;

1) 10.59. 3-Fluor-10-[5-(phenoxymethyl)-[1,2,4]-oxadiazol-3-yl]-9H-imidazo[1,5-a][1,2,4]triazolo[1,5-d][1,4]-benzodiazepin, Smp. 208-209°C, aus 3-Fluor-9H-imidazo[1,5-a][1,2,4]triazolo[1,5-d][1,4]benzodiazepin-10-carboxamidoxim und Phenoxyessigsäure;

1) 10.60. 10-(5-benzyl-1,2,4-oxadiazol-3-yl)-3-methyl-9H-imidazo[1,5-a][1,2,4]triazolo[1,5-d][1,4]-benzodiazepin, Smp. 184-185°C, aus 3-Methyl-9H-imidazo[1,5-a][1,2,4]triazolo[1,5-d][1,4]benzodiazepin-10-carboxamidoxim und Phenylessigsäure;

1) 10.61. 10-[5-[4-(4-Brombenzyloxy)benzyl]-1,2,4-oxadiazol-3-yl]-3-fluor-9H-imidazo[1,5-a][1,2,4]triazolo-[1,5-d][1,4]benzodiazepin, Smp. 176-177°C, aus 3-Fluor-9H-imidazo[1,5-a][1,2,4]triazolo[1,5-d][1,4]-benzodiazepin-10-carboxamidoxim und 4-(4-Brombenzyloxy)phenylessigsäure;

1) 10.62. N-[3-[3-Fluor-9H-imidazo[1,5-a][1,2,4]triazolo[1,5-d][1,4]benzodiazepin-10-yl]-1,2,4-oxadiazol-5-yl]methyl]-N-methylcarbaminsäure-benzylester, Smp. 171-173°C, aus 3-Fluor-9H-imidazo[1,5-a][1,2,4]-triazolo[1,5-d][1,4]benzodiazepin-10-carboxamidoxim und N-[(Benzyloxy)carbonyl]-N-methylglycin;

1) 10.63. 3-Fluor-10-(5-isobutyl-1,2,4-oxadiazol-3-yl)-9H-imidazo[1,5-a][1,2,4]triazolo[1,5-d][1,4]-benzodiazepin, Smp. 181-182°C, aus 3-Fluor-9H-imidazo[1,5-a][1,2,4]triazolo[1,5-d][1,4]benzodiazepin-10-carboxamidoxim und Isovaleriansäure;

1) 10.64. 3-Fluor-10-(5-ethyl-1,2,4-oxadiazol-3-yl)-9H-imidazo[1,5-a][1,2,4]triazolo[1,5-d][1,4]-benzodiazepin, Smp. 245-247°C, aus 3-Fluor-9H-imidazo[1,5-a][1,2,4]triazolo[1,5-d][1,4]benzodiazepin-10-carboxamidoxim und Propionsäure;

1) 10.65. (S)-1-[3-[3-Fluor-9H-imidazo[1,5-a][1,2,4]triazolo[1,5-d][1,4]benzodiazepin-10-yl]-1,2,4-oxadiazol-5-yl]methyl]carbaminsäure-benzylester, Smp. 119-121°C, aus 3-Fluor-9H-imidazo[1,5-a][1,2,4]triazolo-[1,5-d][1,4]benzodiazepin-10-carboxamidoxim und N-[(Benzyloxy)carbonyl]-L-alanin;

1) 10.66. 3-Fluor-10-[5-(1-pyridin-3-yl-oxy-1-methylethyl)-[1,2,4]-oxadiazol-3-yl]-9H-imidazo[1,5-a][1,2,4]-triazolo[1,5-d][1,4]benzodiazepin, Smp. 104-105°C, aus 3-Fluor-9H-imidazo[1,5-a][1,2,4]triazolo[1,5-d]-[1,4]benzodiazepin-10-carboxamidoxim und 2-Methyl-2-(3-pyridyloxy)propionsäure;

1) 10.67. 3-Fluor-10-[5-(cyclopropylmethyl)-1,2,4-oxadiazol-3-yl]-9H-imidazo[1,5-a][1,2,4]triazolo[1,5-d]-[1,4]benzodiazepin, Smp. 179-181°C, aus 3-Fluor-9H-imidazo[1,5-a][1,2,4]triazolo[1,5-d][1,4]-benzodiazepin-10-carboxamidoxim und Cyclopropanessigsäure.

## Beispiel 2

2) 1. 3,62 g 6-Fluor-2H-1,3-benzoxazin-2,4(1H)-dion werden in 15 ml Formamid suspendiert. Während ca. 10 min leitet man gasförmigen Ammoniak unter Rühren bis zur Sättigung ein, und rührt noch 1 h weiter bei Raumtemperatur. Anschliessend wird während 16 h bei 125°C (Badtemperatur) gerührt. Das Lösungsmittel wird sodann im Hochvakuum abdestilliert. Der Rückstand wird in 50 ml Wasser verrührt und dann leicht abgekühlt. Die entstandenen Kristalle werden abfiltriert, mit eiskaltem Wasser gewaschen, und im Vakuum über Calciumchlorid getrocknet. Man erhält 2.9 g rohes 6-Fluor-3H-chinazolin-4-on. Nach Umkristallisieren aus Essigsäureethylester schmilzt die Substanz bei 252-255°C.

2) 2. 2.7 g rohes 6-Fluor-3H-chinazolin-4-on und 7,5 g 2,4-Bis-(4-methoxyphenyl)-2,4-dithioxo-1,3,2,4-dithiophosphetan ("Lawesson-Reagens") werden in 50 ml Pyridin 8 h bei 110°C Badtemperatur gerührt. Das Reaktionsgemisch wird im Rollverdampfer eingeengt. Der Rückstand wird in 80 ml gesättigter wässeriger Natriumhydrogencarbonat-Lösung aufgenommen und 30 min bei Raumtemperatur gerührt. Man kühlt auf 15°C, filtriert die Kristalle ab, und wäscht sie mit eiskaltem Wasser. Nach Trocknen im Vakuum erhält man 3.0 g rohes 6-Fluor-3H-chinazolin-4-thion. Nach Umkristallisieren aus Essigsäureethylester erhält man gelbe Kristalle vom Smp. 294-297°C.

2) 3. 2,9 g rohes 6-Fluor-3H-chinazolin-4-thion werden in 75 ml Tetrahydrofuran suspendiert. Sodann werden bei Raumtemperatur, unter Rühren, 7,9 ml Hydrazinhydrat zugegeben. Nach ca. 15 min entsteht für kurze Zeit eine Lösung, dann fallt ein Niederschlag aus. Man rührt 3 h bei Raumtemperatur, gibt

dann 75 ml Orthoameisensäure-triethylester dazu und destilliert das Tetrahydrofuran ab. Die zurückbleibende Suspension wird 3 h bei Rückflusstemperatur gerüht. Die Reaktionslösung wird im Vakuum eingedampft. Der Rückstand wird in 300 ml Wasser aufgenommen, 1 h bei Raumtemperatur gerührt, und dann auf 15° C abgekühlt. Die Kristalle werden abfiltriert und im Vakuum getrocknet. Man erhält 2,9 g Rohprodukt. Nach Umkristallisieren aus Ethanol erhält man 2,4 g 9-Fluor-1,2,4-triazolo[1,5-c]chinazolin vom Smp. 193-195° C.

2) 4. 2,3 g 9-Fluor-1,2,4-triazolo[1,5-c]chinazolin werden in 40 ml 6 N Salzsäure 1 h bei 95° C gerührt, und dann im Eisbad abgekühlt. Die Reaktionslösung wird in 30 ml 25%-igem wässerigem Ammoniak gegossen und 10 min im Eisbad verrührt. Die Kristalle werden abfiltriert, mit 30 ml eiskaltem Wasser gewaschen, und im Vakuum getrocknet. Man erhält 2,0 g 4-Fluor-2-(1H-1,2,4-triazol-3-yl)anilin vom Smp. 142,5-144,5° C.

2) 5. 4,3 g 4-Fluor-2-(1H-1,2,4-triazol-3-yl)anilin werden in 200 ml Dioxan und 2,3 ml Pyridin absolut gelöst. Die Lösung wird unter Argon gerührt und auf 12° C abgekühlt. Man tropft dann innerhalb von 5 min, bei 12° bis 15° C, eine Lösung von 2,2 ml Chloracetylchlorid in 8,0 ml Diethylether dazu. Die entstehende Suspension wird 15 min bei 10° bis 12° C gerührt und dann innerhalb von 5 min mit 28,8 ml wässeriger 2 N Natriumhydroxyd-Lösung versetzt. Man rührt über Nacht bei Raumtemperatur. Der pH sinkt dabei auf ca 9. Man stellt mit 3 N Salzsäure auf pH 8, und dampft die Lösung im Vakuum bei ca. 40° C ein. Der Rückstand wird in 150 ml Wasser und 5 ml Essigsäure-ethylester 30 min bei 15° C gerührt. Die Kristalle werden dann abfiltriert, mit kaltem Wasser gewaschen, und im Vakuum bei 50° C getrocknet. Man erhält 3,78 g 10-Fluor-5H-[1,2,4]triazolo[1,5-d][1,4]benzodiazepin6(7H)-on vom Smp. 232,5 bis 238° C. Aus der wässerigen Phase kann noch weitere Substanz wie folgt gewonnen werden: man dampft im Vakuum zur Trockene ein. Der Rückstand wird in 10 ml Trifluoressigsäure über Nacht bei Raumtemperatur gerührt. Man dampft im Vakuum ein, nimmt den Rückstand in 60 ml gesättigter wässeriger Natriumcarbonat-Lösung und 2 ml Essigsäureethylester auf, rührt während 1 h bei Raumtemperatur, und filtriert dann die Kristalle ab. Man erhält weitere 0,5 g 10-Fluor-5H-[1,2,4]triazolo[1,5-d][1,4]-benzodiazepin-6(7H)-on.

2) 6.1. 19,64 g 10-Fluor-5H-[1,2,4]triazolo[1,5-d][1,4]benzodiazepin-6(7H)on werden in 0,62 l alkoholfreiem Chloroform suspendiert. Man gibt 32,8 ml N,N,4-Trimethylanilin und 12,5 ml Phosphoroxychlorid dazu, und rührt das Gemisch 24 h bei Rückflusstemperatur. Man gibt nochmals 6,6 ml N,N,4-Trimethylanilin und 2,5 ml Phosphoroxychlorid hinzu und erhitzt weitere 7 h auf Rückflusstemperatur. Das Reaktionsgemisch wird auf 30° C abgekühlt, in 1,3 l 10%-ige wässerige Natriumhydrogencarbonat-Lösung gegossen, 45 min intensiv gerührt, und dann über Nacht stehen gelassen. Die Chloroform-Phase wird abgetrennt. Die wässerige Phase wird nochmals mit 50 ml Chloroform (alkoholfrei) extrahiert. Diese Chloroform-Phase wird ebenfalls durch das vorher verwendete Dicalite filtriert. Die vereinigten Chloroform-Extrakte werden durch Dicalite filtriert und über 70 g Natriumsulfat getrocknet. Im Rollverdampfer werden dann bei 35° bis 40° C Badtemperatur ca. 650 ml Chloroform abdestilliert. Die zurückbleibende Lösung, enthält ein Gemisch von 6-Chlor-5H-[1,2,4]triazolo[1,5-d][1,4]benzodiazepin und N,N,4-Trimethylanilin.

Eine Lösung von 11,3 g Isocyanessigsäure-ethylester in 2,5 l Tetrahydrofuran wird unter Rühren und unter Argon-Begasung auf-25° C gekühlt. Es werden 11,4 g Kalium-tert-butylat in Portionen so zugegeben, dass die Temperatur nicht über -10° C steigt. Man rührt diese Suspension 45 min bei -10° C, kühlt sie auf -65° C und gibt dann innert 15 bis 30 min die vorher erhaltene Lösung von 6-Chlor-10-fluor-5H-[1,2,4]triazolo[1,5-d][1,4]benzodiazepin und N,N,4-Trimethylanilin in Chloroform so dazu, dass die Temperatur zwischen -35° und -30° C bleibt. Das Reaktionsgemisch wird dann auf 20° C erwärmt, und noch 1 h bei dieser Temperatur weiter gerührt. Man gibt 3,8 ml Essigsäure dazu, rührt weitere 15 min, und giesst in ein Gemisch von 1,0 l 5%-iger wässeriger Natriumhydrogencarbonat-Lösung und 0,2 l Essigsäureethylester. Man rührt 15 min und lässt dann über Nacht stehen. Die in der wässerigen Phase ausgefallenen Kristalle werden abgenutscht und nacheinander mit 50 ml Essigsäureethylester, mit 100 ml Wasser und mit 50 ml Essigsäureethylester gewaschen. Die Kristalle werden im Vakuum getrocknet. Man erhältt 13,65 g 3-Fluor-9H-imidazo[1,5-a][1,2,4]triazolo[1,5-d][1,4]benzodiazepin-10-carbonsäure-ethylester vom Smp. 254-258° C. Die vereinigten organischen Phasen können im Vakuum eingedampft werden. Der Rückstand (ca. 44,0 g) enthält vorwiegend N,N,4-Trimethylanilin, neben geringen Mengen Edukt und 3-Fluor-9H-imidazo[1,5-a][1,2,4]triazolo[1,5-d][1,4]benzodiazepin-10-carbonsäure-ethylester. Im weiteren kann aus den wässerigen Phasen mit Dichlormethan ca. 0,6 g Substanz extrahiert werden. Dieses Extrakt enthält ebenfalls noch 3-Fluor-9H-imidazo[1,5-a][1,2,4]triazolo[1,5-d][1,4]benzodiazepin-10-carbonsäure-ethylester. Insgesamt können aus den organischen und wässerigen Phasen noch 0,57 g 3-Fluor-9H-imidazo[1,5-a][1,2,4]triazolo[1,5-d][1,4]benzodiazepin-10-carbonsäure-ethylester vom Smp. 259-260° C gewonnen werden.

Auf analoge Weise erhält man:

2) 6.2. 9H-Imidazo[1,5-a][1,2,4]triazolo[1,5-d][1,4]benzodiazepin-10-carbonsäure-ethylester, Smp. 233-234 ° C, aus 5H-[1,2,4]Triazolo[1,5-d][1,4]benzodiazepin-6(7H)-on (hergestellt gemäss Beispiel 1) 4.1;

2) 6.3. 3-Chlor-9H-imidazo[1,5-a][1,2,4]triazolo[1,5-d][1,4]benzodiazepin-10-carbonsäure-ethylester, Smp. 255-256 ° C, aus 3-Chlor-5H-[1,2,4]triazolo[1,5-d][1,4]benzodiazepin-6(7H)-on (hergestellt gemäass Beispiel 1) 4.4;

2) 6.4. 4-Chlor-9H-imidazo[1,5-a][1,2,4]triazolo[1,5-d][1,4]benzodiazepin-10-carbonsäure-ethylester, Smp. 227-228 ° C, aus 4-Chlor-5H-[1,2,4]triazolo-[1,5-d][1,4]benzodiazepin-6(7H)-on (hergestellt gemäass Beispiel 1) 4.2.

2) 7.1. 17,0 g 3-Fluor-9H-imidazo[1,5-a][1,2,4]triazolo[1,5-d][1,4]benzodiazepin-10-carbonsäure-ethylester werden unter Rühren in 0,5 l Ethanol absolut auf 80 ° C erwärmt. Man gibt sodann eine Lösung von 2,65 g Natriumhydroxid reinst in 80 ml Wasser dazu und erhitzt 1 h auf Rückflusstemperatur. Das Reaktionsgemisch wird im Vakuum eingedampft. Der Rückstand wird in 520 ml Wasser aufgenommen. Es entsteht eine beinahe klare Lösung. Diese wird mit 0,66 ml konz. Salzsäure angesäuert. Der Kristallbrei wird 1 h bei 10 ° C gerührt und dann filtriert. Die Kristalle werden mit 40 ml eiskaltem entionisiertem Wasser gewaschen und im Vakuum getrocknet. Man erhält 14,5 g 3-Fluor-9H-imidazo[1,5-a][1,2,4]triazolo[1,5-d]-[1,4]benzodiazepin-10-carbonsäure vom Smp. 245-247 ° C (Zers.).

Auf analoge Weise erhält man:

2) 7.2. 9H-Imidazo[1,5-a][1,2,4]triazolo[1,5-d][1,4]benzodiazepin-10-carbonsäure, Smp. 283-285 ° C, aus 9H-Imidazo[1,5-a][1,2,4]triazolo[1,5-d][1,4]benzodiazepin-10-carbonsäure-ethylester;

2) 7.3. 3-Chlor-9H-imidazo[1,5-a][1,2,4]triazolo[1,5-d][1,4]benzodiazepin-10-carbonsäure, Smp. 270-271 ° C, aus 3-Chlor-9H-imidazo[1,5-a][1,2,4]triazolo[1,5-d][1,4]benzodiazepin-10-carbonsäure-ethylester;

2) 7.4. 4-Chlor-9H-imidazo[1,5-a][1,2,4]triazolo[1,5-d][1,4]benzodiazepin-10-carbonsäure, Smp. 276-277 ° C (Zers.), aus 4-Chlor-9H-imidazo[1,5-a][1,2,4]triazolo[1,5-d][1,4]benzodiazepin-10-carbonsäure-ethylester.

2) 8. Zu einer Suspension von 11,0 g 3-Fluor-9H-imidazo[1,5-a][1,2,4]triazolo-[1,5-d][1,4]benzodiazepin-10-carbonsäure in 450 ml Dimethylformamid werden 12,1 g 1,1'-Carbonyldiimidazol zugegeben. Die Suspension geht nach 15 bis 30 min vorübergehend in Lösung, wonach das Imidazolid des Eduktes ausfällt. Dann gibt man 7,1 g Cyclopropylcarboxamidoxim dazu und rührt über Nacht bei 80 ° C. Man dampft das Reaktionsgemisch im Vakuum, dann im Hochvakuum bei 50 ° bis 60 ° C Badtemperatur ein. Der Rückstand wird in 250 ml Essigsäure 2,5 h bei 110 ° C gerührt. Die Lösung wird im Vakuum und dann im Hochvakuum zur Trockene eingedampft. Der Rückstand wird in 80 ml Essigsäureethylester kurz erwärmt, unter Rühren auf Raumtemperatur abgekühlt und dann langsam, unter Rühren in 200 ml 7%-iger wässeriger Natriumhydrogencarbonat-Lösung gegossen. Man rührt 1 h bei Raumtemperatur und filtriert dann das Gemisch durch eine Glassinternutsche. Der Filterkuchen wird zuerst mit 20 ml kaltem Essigsäure-ethylester, dann mit 2,0 l Wasser gewaschen. Die Kristalle werden im Vakuum getrocknet. Man erhält 11,05 g 10-(3-Cyclopropyl-1,2,4-oxadiazol-5-yl)-3-fluor-9H-imidazo[1,5-a][1,2,4]triazolo[1,5-d]-[1,4]benzodiazepin vom Smp. 215-218 ° C (Sintern bei 208-215 ° C). Die organische Phase wird im Vakuum auf ca. 30 ml konzentriert und dann auf eine Säule aus 20g Kieselgel (Korngrösse 0,063-0,2 mm) gegeben. Man eluiert mit Essigester. Der Rückstand aus den Eluaten, welche nach DC 10-(3-Cyclopropyl-1,2,4-oxadiazol-5-yl)-3-fluor-9H-imidazo[1,5-a][1,2,4]triazolo[1,5-d][1,4]benzodiazepin enthalten, wird aus Essigsäure-ethylester kristallisiert. Man erhält weitere 0,48 g 10-(3-Cyclopropyl-1,2,4-oxadiazol-5-yl)-3-fluor-9H-imidazo[1,5-a][1,2,4]triazolo[1,5-d][1,4]benzodiazepin vom Smp. 217-218 ° C.

Auf analoge Weise erhält man:

2) 8.2 3-Fluor-10-(3-(2-methoxyethyl)-1,2,4-oxadiazol-5-yl)-9H-imidazo[1,5-a][1,2,4]triazolo[1,5-d][1,4]-benzodiazepin, Smp. 181-182 ° C, aus 3-Fluor-9H-imidazo[1,5-a][1,2,4]triazolo[1,5-d][1,4]benzodiazepin-10-carbonsäure und 3-Methoxypropionamidoxim;

2) 8.3. 10-(3-Benzyl-1,2,4-oxadiazol-5-yl)-3-fluor-9H-imidazo[1,5-a][1,2,4]triazolo[1,5-d][1,4]benzodiazepin, Smp. 157-158 ° C, aus 3-Fluor-9H-imidazo[1,5-a][1,2,4]triazolo[1,5-d][1,4]benzodiazepin-10-carbonsäure und 2-Phenylacetamidoxim;

2) 8.4. 3-Chlor-10-(3-cyclopropyl-1,2,4-oxadiazol-5-yl)-9H-imidazo[1,5-a][1,2,4]triazolo[1,5-d][1,4]-benzodiazepin, Smp. 255-256 ° C, aus 3-Chlor-9H-imidazo[1,5-a][1,2,4]triazolo[1,5-d][1,4]benzodiazepin-10-carbonsäure (hergestellt gemäss Beispiel 1) und Cyclopropancarboxamidoxim;

2) 8.5. 3-Chlor-10-(3-(2-methoxyethyl)-1,2,4-oxadiazol-5-yl)-9H-imidazo[1,5-a][1,2,4]triazolo[1,5-d][1,4]-benzodiazepin, Smp. 239-240 ° C, aus 3-Chlor-9H-imidazo[1,5-a][1,2,4]triazolo[1,5-d][1,4]benzodiazepin-10-carbonsäure (hergestellt gemäss Beispiel 1) und 3-Methoxypropionamidoxim;

2) 8.6. 10-(3-Cyclopropyl-1,2,4-oxadiazol-5-yl)-9H-imidazo[1,5-a][1,2,4]triazolo[1,5-d][1,4]benzodiazepin, Smp. 230-233 ° C (Zers.), aus 9H-imidazo[1,5-a][1,2,4]-triazolo[1,5-d][1,4]benzodiazepin-10-carbonsäure

und Cyclopropancarboxamidoxim.

2) 8.7. 4-Chlor-10-(3-cyclopropyl-1,2,4-oxadiazol-5-yl)-9H-imidazo[1,5-a][1,2,4]triazolo[1,5-d][1,4]-benzodiazepin, Smp. 280-282 °C, aus 4-Chlor-9H-imidazo[1,5-a][1,2,4]triazolo[1,5-d][1,4]benzodiazepin-10-carbonsäure (hergestellt gemäss Beispiel 1) und Cyclopropancarboxamidoxim.

Beispiel 3

3) 1.1. 88 g 4-Chlor-2-(chlormethyl)chinazolin werden in 1 l Tetrahydrofuran suspendiert, auf 10 °C abgekühlt und unter Rühren mit 92 ml Aminoacetaldehyddimethylacetal versetzt. Man rührt 2,5 h bei Raumtemperatur und kühlt sodann das Reaktionsgemisch auf 0 °C ab. Die ausgefallenen Kristalle werden abfiltriert. Das Filtrat wird eingeengt und in 3 l Essigsäureethylester aufgenommen. Der unlösliche Teil wird abfiltriert und das Filtrat wird im Vakuum eingedampft. Der Rückstand (2-(Chlormethyl)-4-(dimethoxyethylamino)chinazolin enthaltend) wird in 1,2 l Essigsäure 3 h auf 100 °C erhitzt und dann im Vakuum eingeengt. Der Rückstand wird zwischen Chloroform und gesättigter wässeriger Natriumhydrogencarbonat-Lösung verteilt. Der Chloroform-Extrakt wird durch eine Kieselgel-Säule chromatographiert. Mit Chloroform/Ethanol 99,5:0,5 wird das gewünschte Produkt eluiert. Dieses wird aus Essigsäureethylester/Diisopropylether umkristallisiert. Man erhält 30,2 g 5-(Chlormethyl)imidazo-[1,2-c]chinazolin vom Smp. 153-154 °C.

Auf analoge Weise erhält man:

3) 1.2. 5-(Chlormethyl)-9-fluorimidazo[1,2-c]chinazolin, Smp. 171-172 °C, aus 4-Chlor-6-fluor-2-(chlormethyl)chinazolin;

3) 1.3. 10-Chlor-5-(chlormethyl)imidazo[1,2-c]chinazolin, Smp. 224-225 °C, aus 4,5-Dichlor-2-(chlormethyl)chinazolin.

3) 2.1. Eine Lösung von 21,4 g 5-(Chlormethyl)imidazo[1,2-c]chinazolin in 500 ml Dioxan wird bei 15 °C unter Rühren in ein Gemisch von 197 ml 1 N wässeriger Natriumhydroxid-Lösung und 100 ml Dioxan zugetropft. Man rührt 2,5 h bei Raumtemperatur und giesst dann das Reaktionsgemisch in 4 l gesättigte wässerige Natriumchlorid-Lösung. Man extrahiert mehrmals mit Chloroform. Die getrockneten Chloroform-Extrakte werden im Vakuum eingedampft. Der Rückstand (18,5 g) wird aus Chloroform-Diethylether umkristallisiert. Man erhält 15,0 g 5H-Imidazo[1,2-d][1,4]benzodiazepin-6(7H)-on vom Smp. 265-266 °C.

Auf analoge Weise erhält man:

3) 2.2. 10-Fluor-5H-imidazo[1,2-d][1,4]benzodiazepin-6(7H)-on, Smp. 282-283 °C, aus 5-(Chlormethyl)-9-fluor-imidazo[1,2-c]chinazolin.

3) 2.3. 11-Chlor-5H-imidazo[1,2-d][1,4]benzodiazepin-6(7H)-on, Smp. 176-177 °C, aus 10-Chlor-5-(chlormethyl)imidazo[1,2-c]chinazolin.

3) 3.1. Zu einer Lösung von 1,8 g Kalium-tert-butylat in 20 ml Dimethylformamid werden bei -15 °C 2,2 g Isocyanessigsäure-tert-butylester zugetropft. Dann wird 1 h bei -15° bis -10 °C gerührt.

Andererseits wird eine Lösung von 2,7 g 5H-Imidazo[1,2-d][1,4]benzodiazepin-6(7H)-on in 55 ml Dimethylformamid bei -15 °C mit 0,76 g einer ca. 55%-igen Dispersion von Natriumhydrid in Mineralöl versetzt. Das Gemisch wird 1 h bei -15 °C gerührt. Dann wird auf -40 °C gekühlt, 3,6 ml Phosphorsäure-diphenylester-chlorid innert 15 min zugetropft und 20 min bei -20 °C weiter gerührt. Dann wird auf -60 °C gekühlt und die Lösung des Isocyanessigsäure-tert-butylester-kaliumsalzes zugegeben. Man rührt das Reaktionsgemisch anschliessend 3 h bei Raumtemperatur. Dann kühlt man auf 10 °C und versetzt mit 0,5 ml Essigsäure. Das Reaktionsgemisch wird in 5 l gesättigte wässerige Natriumhydrogencarbonat-Lösung gegossen. Man extrahiert 2 mal mit Essigsäureethylester und 2 mal mit Chloroform. Die organischen Extrakte werden mit gesättigter wässeriger Natriumhydrogencarbonat-Lösung gewaschen, getrocknet und im Vakuum eingedampft. Der Rückstand wird in Chloroform gelöst und durch Kieselgel chromatographiert. Mit Chloroform/Ethanol 98,5:1,5 eluiert man 2,3 g rohen 9H-Diimidazo[1,5-a:1',2'-d]-[1,4]benzodiazepin-10-carbonsäure-tert-butylester. Dieser wird aus Essigsäureethylester/Diethylether/Diisopropylether umkristallisiert. Man erhält 1,8 g reinen 9H-Diimidazo-[1,5-a:1',2'-d][1,4]benzodiazepin-10-carbonsäure-tert-butylester vom Smp. 188-190 °C.

Auf analoge Weise erhält man:

3) 3.2. 3-Fluor-9H-diimidazo[1,5-a:1',2'-d][1,4]benzodiazepin-10-carbonsäureethylester, Smp. 213-215 °C, aus 10-Fluor-5H-imidazo[1,2-d][1,4]benzodiazepin-6(7H)-on, durch Umsetzung mit Isocyanessigsäureethylester statt mit Isocyanessigsäure-tert-butylester. Dieser Ethylester wird gemäss Beispiel 5) 4. zur 3-Fluor-9H-diimidazo[1,5-a:1',2'-d][1,4]benzodiazepin-10-carbonsäure hydrolysiert.

3) 3.3. 4-Chlor-9H-diimidazo[1,5-a:1',2'-d][1,4]benzodiazepin-10-carbonsäureethylester, Smp. 217-218 °C °C, aus 11-Chlor-5H-imidazo[1,2-d][1,4]benzodiazepin-6(7H)-on, durch Umsetzung mit Isocyanessigsäureethylester statt mit Isocyanessigsäure-tert-butylester. Die Hydrolyse zur 4-Chlor-9H-diimidazo-[1,5-a,1',2'-d][1,4]benzodiazepin-10-carbonsäure erfolgt gemäss Beispiel 5) 4.

3) 4. 10 g 9H-Diimidazo[1,5-a:1',2'-d][1,4]benzodiazepin-10-carbonsäure-tert-butylester werden in 170 ml Trifluoressigsäure gelöst und 15 h bei Raumtemperatur stehen gelassen. Das Reaktionsgemisch wird sodann im Hochvakuum eingedampft. Der Rückstand wird aus Essigsäureethylester umkristallisiert. Man erhält 13,5 g 9H-Diimidazo[1,5-a:1',2'-d][1,4]benzodiazepin-10-carbonsäure, die zum Teil als Trifluoracetat vorliegt.

3) 5.1. 5,7 g rohe 9H-Diimidazo[1,5-a:1',2'-d][1,4]benzodiazepin-10-carbonsäure werden in 80 ml Dimethylformamid gelöst. Man gibt 6,5 g 1,1'-Carbonyldiimidazol dazu und rührt bei 30° C während 3 h. Dann werden 4,5 g Cyclopropancarboxamidoxim zugegeben und über Nacht bei 80° C gerührt. Das Reaktionsgemisch wird im Vakuum eingedampft und der Rückstand in Essigsäure 2,5 h bei 115° C gerührt. Man dampft im Vakuum ein, verteilt den Rückstand zwischen Chloroform und gesättigter wässeriger Natriumhydrogencarbonat-Lösung. Das Chloroform-Extrakt wird getrocknet und durch Kieselgel chromatographiert. Mit Chloroform/Ethanol 99:1 werden 3,15 g rohes 10-(3-Cyclopropyl-1,2,4-oxadiazol-5-yl)-9H-diimidazo[1,5-a:1',2'-d][1,4]benzodiazepin eluiert. Durch Umkristallisieren aus Essigsäureethylester erhält man 2,7 g reines 10-(3-Cyclopropyl-1,2,4-oxadiazol-5-yl)-9H-diimidazo[1,5-a:1',2'-d][1,4]benzodiazepin vom Smp. 224-226° C.

Auf analoge Weise erhält man:

3) 5.2. 3-Fluor-10-(3-cyclopropyl-1,2,4-oxadiazol-5-yl)-9H-diimidazo[1,5-a:1',2'-d][1,4]benzodiazepin, Smp. 226-227° C, aus 3-Fluor-9H-diimidazo[1,5-a:1',2'-d][1,4]benzodiazepin-10-carbonsäure.

3) 5.3. 4-Chlor-10-(3-cyclopropyl-1,2,4-oxadiazol-5-yl)-9H-diimidazo[1,5-a:1',2'-d][1,4]benzodiazepin, Smp. 247-248° C, aus 4-Chlor-9H-diimidazo[1,5-a:1',2'-d][1,4]benzodiazepin-10-carbonsäure.

## Beispiel 4

10-(3-Cyclopropyl-1,2,4-oxadiazol-5-yl)-9H-diimidazo[1,5-a:1',2'-d][1,4]benzodiazepin wird in Ethanol gelöst und mit ethanolischer Salzsäure versetzt. Nach Zugabe von Diethylether kristallisiert 10-(3-Cyclopropyl-1,2,4-oxadiazol-5-yl)-9H-diimidazo[1,5-a:1',2'-d][1,4]benzodiazepin-monohydrochlorid aus. Dieses wird abfiltriert, mit Ethanol/Diethylether gewaschen und getrocknet. Smp. 289-290° C (Zers.).

## Beispiel 5

5) 1.1. 10 g 5,6-Dihydro-6-oxo-4H-imidazo[1,5-a][1,4]benzodiazepin-3-carbonsäureethylester (hergestellt nach Eur. Pat. Anm. Nr. 27'214 vom 22. April 1981) werden in 110 ml Pyridin suspendiert und mit 15 g 2,4-Bis-(4-methoxyphenyl)-2,4-dithioxo-1,3,2,4-dithiadiphosphetan ("Lawesson-Reagens") versetzt. Das Gemisch wird 48 h auf 100° C erhitzt und sodann im Vakuum eingedampff. Der Rückstand wird in 120 ml Methanol suspendiert und 20 min am Rückfluss gekocht. Nach dem Abkühlen werden die Kristalle abfiltriert. Man erhält 9,2 g rohen 5,6-Dihydro-6-thioxo-4H-imidazo[1,5-a][1,4]benzodiazepin-3-carbonsäureethylester. Durch Einengen des Filtrates erhält man weitere 0,5 g dieses Produktes. Durch Umkristallisieren aus Ethanol erhält man 6,7 g reinen 5,6-Dihydro-6-thioxo-4H-imidazo[1,5-a]benzodiazepin-3-carbonsäureethylester vom Smp. 273-274° C.

Auf analoge Weise erhält man:

5) 1.2. 8-Fluor-5,6-dihydro-6-thioxo-4H-imidazo[1,5-a][1,4]benzodiazepin-3-carbonsäureethylester, Smp. 301-302° C (Zers.), aus 8-Fluor-5,6-dihydro-6-oxo-4H-imidazo[1,5-a][1,4]benzodiazepin-3-carbonsäureethylester;

5) 1.3. 8-Chlor-5,6-dihydro-6-thioxo-4H-imidazo[1,5-a][1,4]benzodiazepin-3-carbonsäureethylester, Smp. 285-287° C, aus 8-Chlor-5,6-dihydro-6-oxo-4H-imidazo[1,5-a][1,4]benzodiazepin-3-carbonsäureethylester; dieser wird in analoger Weise, wie in Eur. Pat. Anm. Nr. 27'214 für den 8-Fluor-5,6-dihydro-6-oxo-4H-imidazo[1,5-a][1,4]benzodiazepin-3-carbonsäureethylesterbeschrieben, hergestellt;

5) 1.4. 5,6-Dihydro-8-methyl-6-thioxo-4H-imidazo[1,5-a][1,4]benzodiazepin-3-carbonsäureethylester, Smp. 274-275° C, aus 5,6-Dihydro-8-methyl-6-oxo-4H-imidazo[1,5-a][1,4]benzodiazepin-3-carbonsäureethylester.

5) 2.1. 6,0 g 5,6-Dihydro-6-thioxo-4H-imidazo[1,5-a][1,4]benzodiazepin-3-carbonsäureethylester werden in 40 ml Aminoacetaldehyddimethylacetal 48 h bei 85° C gerührt. Man verdünnt mit 150 ml Wasser und extrahiert viermal mit Essigsäureethylester. Die organischen Extrakte werden mit gesättigter wässeriger Natriumchlorid-Lösung gewaschen, und im Vakuum eingedampft. Der Rückstand wird durch Kieselgel chromatographiert. Man eluiert mit Dichlormethan/Ethanol 99:1, 98:2 und 97:3. Das Roheluat wird aus Essigsäureethylester/Diisopropylether umkristallisiert. Man erhält 4,3 g reinen 6-[(2,2-Dimethoxyethyl)-

amino]-4H-imidazo[1,5-a][1,4]benzodiazepin-3-carbonsäureethylester vom Smp. 121-122 ° C.

Auf analoge Weise erhält man:

5) 2.2. 6-[(2,2-Diethoxyethyl)amino]-8-fluor-4H-imidazo[1,5-a][1,4]benzodiazepin-3-carbonsäureethylester, aus 8-Fluor-5,6-dihydro-6-thioxo-4H-imidazo[1,5-a][1,4]benzodiazepin-3-carbonsäureethylester und Aminoacetaldehyd-diethylacetal;

5) 2.3. 8-Chlor-6-[(2,2-dimethoxyethyl)amino]-4H-imidazo[1,5-a][1,4]benzodiazepin-3-carbonsäureethylester, Smp. 167-168 ° C, aus 8-Chlor-5,6-dihydro-6-thioxo-4H-imidazo[1,5-a][1,4]benzodiazepin-3-carbonsäureethylester und Aminoacetaldehyd-dimethylacetal;

5) 2.4. 6-[(2,2-Diethoxyethyl)amino]-8-methyl-4H-imidazo[1,5-a][1,4]benzodiazepin-3-carbonsäureethylester (als Oel), aus 5,6-Dihydro-8-methyl-6-thioxo-4H-imidazo[1,5-a][1,4]benzodiazepin-3-carbonsäureethylester und Aminoacetaldehyd-diethylacetal.

5) 3.1. Eine Lösung von 19,3 g 6-[(2,2-Dimethoxyethyl)amino]-4H-imidazo[1,5-a][1,4]benzodiazepin-3-carbonsäureethylester in 180 ml Essigsäure wird 66 h auf 110 ° C erhitzt. Das Reaktionsgemisch wird im Vakuum eingedampft und der Rückstand zwischen Dichlormethan und gesättigter wässeriger Natriumhydrogencarbonat-Lösung verteilt. Die organischen Phasen werden mit gesättigter wässeriger Natriumchlorid-Lösung gewaschen, getrocknet und im Vakuum eingedampft. Der Rückstand wird aus Essigsäureethylester umkristallisiert. Man erhält 12,1 g 9H-Diimidazo[1,5-a:1',2'-d][1,4]benzodiazepin-10-carbonsäureethylester vom Smp. 193-194 ° C.

Auf analoge Weise erhält man:

5) 3.2. 3-Fluor-9H-diimidazo[1,5-a:1',2'-d][1,4]benzodiazepin-10-carbonsäureethylester, Smp. 213-215 ° C, aus 6-[(2,2-Diethoxyethyl)amino]-8-fluor-4H-imidazo[1,5-a][1,4]benzodiazepin-3-carbonsäureethylester;

5) 3.3. 3-Chlor-9H-diimidazo[1,5-a:1',2'-d][1,4]benzodiazepin-10-carbonsäureethylester, Smp. 228-229 ° C, aus 8-Chlor-6-[(2,2-dimethoxyethyl)amino]-4H-imidazo[1,5-a][1,4]benzodiazepin-3-carbonsäureethylester;

5) 3.4. 3-Methyl-9H-diimidazo[1,5-a:1',2'-d][1,4]benzodiazepin-10-carbonsäureethylester, Smp. 221-222 ° C, aus 6-[(2,2-Dimethoxyethyl)amino]-8-methyl-4H-imidazo[1,5-a][1,4]benzodiazepin-3-carbonsäureethylester.

5) 4.1. 5 g 9H-Diimidazo[1,5-a:1',2'-d][1,4]benzodiazepin-10-carbonsäureethylester werden in einem Gemisch von 1,5 g Kaliumhydroxid, 35 ml Wasser und 250 ml Ethanol 2 h auf 80 ° C erhitzt. Das Reaktionsgemisch wird im Vakuum eingedampff. Man löst den Rückstand in 100 ml Wasser und stellt ihn mit Salzsäure auf pH 5. Man lässt einige Stunden stehen und filtriert sodann die ausgefallenen Kristalle ab. Nach Trocknen im Vakuum erhält man 4,0 g 9H-Diimidazo[1,5-a:1',2'-d][1,4]benzodiazepin-10-carbonsäure.

Auf analoge Weise erhält man:

5) 4.2. 3-Fluor-9H-diimidazo[1,5-a:1',2'-d][1,4]benzodiazepin-10-carbonsäure, aus 3-Fluor-9H-diimidazo-[1,5-a:1',2'-d][1,4]benzodiazepin-10-carbonsäureethylester (nach Beispiel 3 oder 5 hergestellt);

5) 4.3. 3-Chlor-9H-diimidazo[1,5-a:1',2'-d][1,4]benzodiazepin-10-carbonsäure, Smp. 277-278 ° C, aus 3-Chlor-9H-diimidazo[1,5-a:1',2'-d][1,4]benzodiazepin-10-carbonsäureethylester;

5) 4.4. 4-Chlor-9H-diimidazo[1,5-a:1',2'-d][1,4]benzodiazepin-10-carbonsäure, Smp. 300-302 ° C (Zers.), aus 4-Chlor-9H-diimidazo[1,5-d:1',2'-d][1,4]benzodiazepin-10-carbonsäureethylester (nach Beispiel 3 hergestellt);

5) 4.5. 3-Methyl-9H-diimidazo[1,5-a:1',2'-d][1,4]benzodiazepin-10-carbonsäure, Smp. 259-261 ° C, aus 3-Methyl-9H-diimidazo[1,5-d:1',2'-d][1,4]benzodiazepin-10-carbonsäureethylester;

5) 5.1. 4,0 g 9H-Diimidazo[1,5-a:1',2'-d][1,4]benzodiazepin-10-carbonsäure werden in 70 ml Dimethylformamid suspendiert. Man gibt 5 g 1,1'-Carbonyldiimidazol dazu und rührt 2 h bei Raumtemperatur. Man versetzt dann mit 250 ml 25%-iger wässeriger Ammoniak-Lösung und 0,5 l Wasser und rührt 1 h bei 10 ° C. Man filtriert die entstandenen Kristalle ab und trocknet sie im Vakuum. Man erhält 3,94 g 9H-Diimidazo[1,5-a:1',2'-d][1,4]benzodiazepin-10-carboxamid. Umkristallisation aus Essigsäureethylester. Smp. 276-279 ° C.

Auf analoge Weise erhält man:

5) 5.2. 3-Fluor-9H-diimidazo[1,5-a:1',2'-d][1,4]benzodiazepin-10-carboxamid, Smp. 287-289 ° C, aus 3-Fluor-9H-diimidazo[1,5-a:1',2'-d][1,4]benzodiazepin-10-carbonsäure;

5) 5.3. 3-Chlor-9H-diimidazo[1,5-a:1',2'-d][1,4]benzodiazepin-10-carboxamid, Smp. über 295 ° C, aus 3-Chlor-9H-diimidazo[1,5-a:1',2'-d][1,4]benzodiazepin-10-carbonsäure;

5) 5.4. 4-Chlor-9H-diimidazo[1,5-a:1',2'-d][1,4]benzodiazepin-10-carboxamid, Smp. 342-343 ° C, aus 4-Chlor-9H-diimidazo[1,5-a:1',2'-d][1,4]benzodiazepin-10-carbonsäure;

5) 5.5. 3-Methyl-9H-diimidazo[1,5-a:1',2'-d][1,4]benzodiazepin-10-carboxamid, Smp. über 295 ° C, aus 3-Methyl-9H-diimidazo[1,5-a:1',2'-d][1,4]-benzodiazepin-10-carbonsäure;

5) 5.6. 4-Methyl-9H-diimidazo[1,5-a:1',2'-d][1,4]benzodiazepin-10-carboxamid, Smp. 320-321 ° C, aus 4-Methyl-9H-diimidazo[1,5-a:1',2'-d][1,4]-benzodiazepin-10-carbonsäure (hergestellt gemäss Beispiel 21) 9.);

5) 5.7. 4-Brom-9H-diimidazo[1,5-a:1',2'-d][1,4]benzodiazepin-10-carboxamid, aus 4-Brom-9H-diimidazo-[1,5-a:1',2'-d][1,4]benzodiazepin-10-carbonsäure (hergestellt gemäss Beispiel 20) 9..

5) 6.1. 2,9 g 9H-Diimidazo[1,5-a:1',2'-d][1,4]benzodiazepin-10-carboxamid werden in 70 ml Dioxan und 2,3 ml Pyridin suspendiert. Man kühlt auf 10°C und gibt 2,0 ml Trifluoressigsäureanhydrid dazu. Man rührt 3 h bei Raumtemperatur, gibt dann nochmals 1,65 ml Pyridin und 1,0 ml Trifluressigsäureanhydrid dazu. Nach 15 h bei Raumtemperatur giesst man in 600 ml gesättigte wässerige Natriumhydrogencarbonat-Lösung und extrahiert viermal mit Chloroform. Nach Abdampfen des Chloroforms erhält man 3,0 g Kristalle, welche durch Kieselgel chromatographiert werden. Das gewünschte Produkt wird mit Chloroform/Ethanol 98:2 eluiert. Nach Umkristallisation aus Essigsäureethylester erhält man 2,3 g 9H-Diimidazo-[1,5-a:1',2'-d][1,4]benzodiazepin-10-carbonitril vom Smp. 226-228°C.

Auf analoge Weise erhält man:

5) 6.2. 3-Fluor-9H-diimidazo[1,5-a:1',2'-d][1,4]benzodiazepin-10-carbonitril, Smp. 211-212°C, aus 3-Fluor-9H-diimidazo[1,5-a:1',2'-d][1,4]benzodiazepin-10-carboxamid;

5) 6.3. 3-Chlor-9H-diimidazo[1,5-a:1',2'-d][1,4]benzodiazepin-10-carbonitril, Smp. 231-232°C, aus 3-Chlor-9H-diimidazo[1,5-a:1',2'-d][1,4]benzodiazepin-10-carboxamid.

5) 6.4. 4-Chlor-9H-diimidazo[1,5-a:1',2'-d][1,4]benzodiazepin-10-carbonitril, Smp. 207-208°C, aus 4-Chlor-9H-diimidazo[1,5-a:1',2'-d][1,4]benzodiazepin-10-carboxamid.

5) 6.5. 3-Methyl-9H-diimidazo[1,5-a:1',2'-d][1,4]benzodiazepin-10-carbonitril, Smp. 267-269°C, aus 3-Methyl-9H-diimidazo[1,5-a:1',2'-d][1,4]benzodiazepin-10-carboxamid.

5) 6.6. 4-Methyl-9H-diimidazo[1,5-a:1',2'-d][1,4]benzodiazepin-10-carbonitril, Smp. 220-221°C, aus 4-Methyl-9H-diimidazo[1,5-a:1',2'-d][1,4]benzodiazepin-10-carboxamid.

5) 6.7. 4-Brom-9H-diimidazo[1,5-a:1',2'-d][1,4]benzodiazepin-10-carbonitril, aus 4-Brom-9H-diimidazo[1,5-a:1',2'-d][1,4]benzodiazepin-10-carboxamid.

5) 7.1. 3,4 g 9H-Diimidazo[1,5-a:1',2'-d][1,4]benzodiazepin-10-carbonitril werden in 65 ml Ethanol suspendiert. Man gibt 1,1 g Hydroxylamin-hydrochlorid, 1,4 g Natriumhydrogencarbonat und 15 ml Wasser dazu, und erhitzt das Gemisch 1,5 h auf Rückflusstemperatur. Nach Abkühlen werden die entstandenen Kristalle abfiltriert und getrocknet. Man erhält 3,11 g 9H-Diimidazo[1,5-a:1',2'-d][1,4]benzodiazepin-10-carboxamidoxim.

Auf analoge Weise erhält man:

5) 7.2. 3-Fluor-9H-diimidazo[1,5-a:1',2'-d][1,4]benzodiazepin-10-carboxamidoxim, aus 3-Fluor-9H-diimidazo[1,5-a:1',2'-d][1,4]benzodiazepin-10-carbonitril;

5) 7.3. 3-Chlor-9H-diimidazo[1,5-a:1',2'-d][1,4]benzodiazepin-10-carboxamidoxim, aus 3-Chlor-9H-diimidazo[1,5-a:1',2'-d][1,4]benzodiazepin-10-carbonitril;

5) 7.4. 4-Chlor-9H-diimidazo[1,5-a:1',2'-d][1,4]benzodiazepin-10-carboxamidoxim, Smp 258-259°C, aus 4-Chlor-9H-diimidazo[1,5-a:1',2'-d][1,4]benzodiazepin-10-carbonitril.

5) 7.5. 3-Methyl-9H-diimidazo[1,5-a:1',2'-d][1,4]benzodiazepin-10-carboxamidoxim, Smp 260-261°C (Zers.), aus 3-Methyl-9H-diimidazo[1,5-a:1',2'-d][1,4]benzodiazepin-10-carbonitril;

5) 7.6. 4-Methyl-9H-diimidazo[1,5-a:1',2'-d][1,4]benzodiazepin-10-carboxamidoxim, Smp 230-232°C, aus 4-Methyl-9H-diimidazo[1,5-a:1',2'-d][1,4]benzodiazepin-10-carbonitril;

5) 7.7. 4-Brom-9H-diimidazo[1,5-a:1',2'-d][1,4]benzodiazepin-10-carboxamidoxim, aus 4-Brom-9H-diimidazo[1,5-a:1',2'-d][1,4]benzodiazepin-10-carbonitril.

5) 8.1. 1,42 g Cyclopropancarbonsäure werden in 100 ml Dimethylformamid gelöst und mit 2,7 g 1,1'-Carbonyldiimidazol versetzt. Man rührt 2 h bei Raumtemperatur und gibt dann 3,11 g 9H-Diimidazo[1,5-a:1',2'-d][1,4]benzodiazepin-10-carboxamidoxim dazu. Man rührt 16 h bei Raumtemperatur und dampff anschliessend im Hochvakuum ein. Der Rückstand wird in 25 ml Cyclopropancarbonsäure gelöst und 4 h auf 130°C erhitzt. Dann wird im Hochvakuum eingedampft. Der Rückstand wird zwischen Dichlormethan und gesättigter wässeriger Natriumhydrogencarbonat-Lösung verteilt. Die wässerige Phase wird noch 4 mal mit Dichlormethan extrahiert; die organischen Extrakte werden mit gesättigter wässeriger Natriumchlorid-Lösung gewaschen, getrocknet und durch Kieselgel chromatographiert. Mit Dichlormethan/Ethanol 98:2 wird das gewünschte Produkt eluiert (3,7 g roh). Nach Umkristallisieren aus Essigsäureethylester erhält man 1,9 g 10-(5-Cyclopropyl-1,2,4-oxadiazol-3-yl)-9H-diimidazo[1,5-a:1',2'-d]-[1,4]benzodiazepin vom Smp. 187-189°C.

Auf analoge Weise erhält man:

5) 8.2. 10-(5-Cyclopropyl-1,2,4-oxadiazol-3-yl)-3-fluor-9H-diimidazo[1,5-a:1',2'-d][1,4]benzodiazepin, Smp. 181-183°C, aus 3-Fluor-9H-diimidazo[1,5-a:1',2'-d][1,4]benzodiazepin-10-carboxamidoxim;

5) 8.3. 3-Chlor-10-(5-cyclopropyl-1,2,4-oxadiazol-3-yl)-9H-diimidazo[1,5-a:1',2'-d][1,4]benzodiazepin, Smp.

213-215 °C, aus 3-Chlor-9H-diimidazo[1,5-a:1',2'-d][1,4]benzodiazepin-10-carboxamidoxim;
5) 8.4. 4-Chlor-10-(5-cyclopropyl-1,2,4-oxadiazol-3-yl)-9H-diimidazo[1,5-a:1',2'-d][1,4]benzodiazepin, Smp. 176-177 °C, aus 4-Chlor-9H-diimidazo[1,5-a:1',2'-d][1,4]benzodiazepin-10-carboxamidoxim;
5) 8.5. 10-(5-Cyclopropyl-1,2,4-oxadiazol-3-yl)-3-methyl-9H-diimidazo[1,5-a:1',2'-d][1,4]benzodiazepin, Smp. 255-257 °C, aus 3-Methyl-9H-diimudazo[1,5-a:1',2'-d][1,4]benzodiazepin-10-carboxamidoxim;
5) 8.6. 10-(5-Benzyl-1,2,4-oxadiazol-3-yl)-3-methyl-9H-diimidazo[1,5-a:1',2'-d][1,4]benzodiazepin, Smp. 156-157 °C, aus 3-Methyl-9H-diimidazo[1,5-a:1',2'-d][1,4]benzodiazepin-10-carboxamidoxim und Phenylessigsäure;
5) 8.7. 3-Chlor-10-[5-(2-methoxyethyl)-1,2,4-oxadiazol-3-yl]-9H-diimidazo[1,5-a:1',2'-d][1,4]benzodiazepin, Smp. 169-170 °C, aus 3-Chlor-9H-diimidazo[1,5-a:1',2'-d][1,4]benzodiazepin-10-carboxamidoxim und 3-Methoxypropionsäure;
5) 8.8. 4-Chlor-10-(5-isopropyl-1,2,4-oxadiazol-3-yl)-9H-diimidazo[1,5-a:1',2'-d][1,4]benzodiazepin, Smp. 156-157 °C, aus 4-Chlor-9H-diimidazo[1,5-a:1',2'-d][1,4]benzodiazepin-10-carboxamidoxim und Isobuttersäure;
5) 8.9. 4-Chlor-10-(5-ethoxymethyl-1,2,4-oxadiazol-3-yl)-9H-diimidazo[1,5-a:1',2'-d][1,4]benzodiazepin, Smp. 178-180 °C, aus 4-Chlor-9H-diimidazo[1,5-a:1',2'-d][1,4]benzodiazepin-10-carboxamidoxim und Ethoxyessigsäure;.
5) 8.10. rac-4-Chlor-10-[5-(2-methyl-cyclopropyl)-1,2,4-oxadiazol-3-yl]-9H-diimidazo-[1,5-a:1',2'-d][1,4]-benzodiazepin, Smp. 183-185 °C, aus 4-Chlor-9H-diimidazo[1,5-a:1',2'-d][1,4]benzodiazepin-10-carboxamidoxim und dem Amidoxim der rac-trans-2-Methylcyclopropancarbonsäure;
5) 8.11. 4-Methyl-10-(5-cyclopropyl-1,2,4-oxadiazol-3-yl)-9H-diimidazo[1,5-a:1',2'-d][1,4]benzodiazepin, Smp. 173-174 °C, aus 4-Methyl-9H-diimidazo[1,5-a:1',2'-d][1,4]benzodiazepin-10-carboxamidoxim;
5) 8.12. 4-Brom-10-(5-cyclopropyl-1,2,4-oxadiazol-3-yl)-9H-diimidazo[1,5-a:1',2'-d][1,4]benzodiazepin, aus 4-Brom-9H-diimidazo[1,5-a:1',2'-d][1,4]benzodiazepin-10-carboxamidoxim.

## Beispiel 6

6) 1.1. 3,0 g 3-Fluor-9H-diimidazo[1,5-a:1',2'-d][1,4]benzodiazepin-10-carbonsäure werden in 80 ml Dimethylformamid suspendiert. Man gibt 3,0 g 1,1'-Carbonyldiimidazol dazu und rührt 3 h bei Raumtemperatur. Dann werden 2,0 g Cyclopropancarboxamidoxim zugegeben und 16 h bei 80 °C gerührt. Das Reaktionsgemisch wird im Vakuum eingeengt und der Rückstand in 100 ml Essigsäure 1,5 h bei 100 °C gerührt. Man dampft im Vakuum ein, verteilt den Rückstand zwischen Chloroform und gesättigter wässeriger Natriumhydrogencarbonat-Lösung. Das Chloroform-Extrakt wird getrocknet und durch Kieselgel chromatographiert. Mit Chloroform/Ethanol 98,5:1,5 werden 3,1 g rohes 10-(5-Cyclopropyl-1,2,4-oxadiazol-3-yl)-3-fluor-9H-diimidazo[1,5-a:1',2'-d][1,4]benzodiazepin eluiert. Durch Umkristallisieren aus Essigsäureethylester erhält man 2,7 g reines 10-(3-Cyclopropyl-1,2,4-oxadiazol-5-yl)-3-fluor-9H-diimidazo[1,5-a:1',2'-d][1,4]benzodiazepin vom Smp. 226-227 °C.

Auf analoge Weise erhält man:
6) 1.2. 3-Chlor-10-(3-Cyclopropyl-1,2,4-oxadiazol-5-yl)-9H-diimidazo[1,5-a:1',2'-d][1,4]benzodiazepin, Smp. 222 °C, aus 3-Chlor-9H-diimidazo[1,5-a:1',2'-d][1,4]benzodiazepin-10-carbonsäure;
6) 1.3. 10-(3-Cyclopropyl-1,2,4-oxadiazol-5-yl)-3-methyl-9H-diimidazo[1,5-a:1',2'-d][1,4]benzodiazepin, Smp. 270-271 °C, aus 3-Methyl-9H-diimidazo-[1,5-a:1',2'-d][1,4]benzodiazepin-10-carbonsäure.

## Beispiel 7

3,0 g 3-Fluor-9H-imidazo[1,5-a][1,2,4]triazolo[1,5-d][1,4]benzodiazepin-10-carboxamidoxim (hergestellt gemäss Beispiel 1) 9.3.) werden in 300 ml Dichlormethan suspendiert und mit 2,1 ml Triethylamin versetzt. Unter Rühren tropft man bei Raumtmeperatur eine Lösung von 1,0 mi Chloracetylchlorid in 10 ml Dichlormethan zu und rührt 18 h am Rückfluss. Das Reaktionsgemisch wird im Vakuum eingedampft. Der Rückstand wird mit 50 ml Essigsäure gelöst und 4 h auf Rückflusstemperatur erhitzt. Danach wird im Vakuum eingedampft. Der Rückstand wird in Dichlormethan gelöst und mit gesättigter wässeriger Natriumhydrogencarbonat-Lösung 30 min gerührt. Die organische Phase wird abgetrennt. Die wässerige Phase wird nochmals mit Dichlormethan extrahiert. Die vereinigten organischen Phasen werden getrocknet, filtriert und im Vakuum eingedampft. Der Rückstand (2 g) wird durch Kieselgel chromatographiert. Mit Dichlormethan/Methanol 99:1 bis 97:3 eluiert man 1,9 g rohes Produkt, welches aus Essigsäureethylester umkristallisiert wird. Man erhält 1,4 g reines 10-[5-(Chlormethyl)-1,2,4-oxadiazol-3-yl]-3-fluor-9H-imidazo[1,5-a][1,2,4]triazolo[1,5-d][1,4]benzodiazepin vom Smp. 238-239 °C.

Beispiel 8

8) 1. 10 g 3-Fluor-9H-imidazo[1,5-a][1,2,4]triazolo[1,5-d][1,4]benzodiazepin-10-carbonsäureethylester werden unter Argon in 400 ml Tetrahydrofuran gelöst. Bei 45°C gibt man 0,8 g Lithiumborhydrid (95%) dazu und rührt anschliessend 7 h bei Rückflusstemperatur. Man kühlt auf 20°C und tropft langsam ca. 20 ml 6 N Salzsäure dazu (bis pH 2), verdünnt dann mit 50 ml Wasser und rührt während 16 h. Man dampft das Tetrahydrofuran im Vakuum ab, stellt mit wässerigem Ammoniak schwach basisch und filtriert die Kristalle ab. Nach dem Trocknen erhält man 6,9 g 3-Fluor-9H-imidazo[1,5-a][1,2,4]triazolo[1,5-d][1,4]benzodiazepin-10-methanol vom Smp. 240-242°C.

8) 2. 14,5 g 3-Fluor-9H-imidazo[1,5-a][1,2,4]triazolo[1,5-d][1,4]benzodiazepin-10-methanol werden in 1,4 l Dichlormethan gelöst, mit 145 g Mangandioxid versetzt und 3 h bei Raumtemperatur gerührt. Man filtriert den unlöslichen Anteil ab, rührt ihn 1 h in 1 l siedendem Dichlormethan und filtriert abermals. Die Filtrate werden im Vakuum eingedampft und die Kristalle abfiltriert. Man erhält 10,3 g 3-Fluor-9H-imidazo[1,5-a]-[1,2,4]triazolo[1,5-d][1,4]benzodiazepin-10-carboxaldehyd vom Smp. 209-211°C. Aus dem Filtrat können weitere 1,1 g gewonnen werden.

8) 3. 13,8 g 3-Fluor-9H-imidazo[1,5-a][1,2,4]triazolo[1,5-d][1,4]benzodiazepin-10-carboxaldehyd werden in 1 l Tetrahydrofuran gelöst und mit 4,3 g Hydroxylamin-hydrochlorid und 9,3 ml Triethylamin versetzt. Man erwärmt 6 h auf Rückflusstemperatur und fügt sodann nochmals 1,0 g Hydroxylaminhydrochlorid und 1,7 ml Triethylamin hinzu. Man erwärmt nochmals 24 h auf Rückflusstemperatur und fügt dann wieder 0,5 g Hydroxylaminhydrochlorid und 0,9 ml Triethylamin hinzu. Man dampft das Reaktionsgemisch im Vakuum ein. Der Rückstand wird in Wasser suspendiert, 1 h gerührt und dann filtriert. Die Kristalle werden in Aceton suspendiert, kurz auf Rückflusstemperatur erwärmt, wieder abgekühlt und abfiltriert. Man erhält 13,2 g 3-Fluor-9H-imidazo[1,5-a][1,2,4]triazolo[1,5-d][1,4]benzodiazepin-10-carboxaldehydoxim vom Smp. 265°C.

8) 4. 3,2 g 3-Fluor-9H-imidazo[1,5-a][1,2,4]triazolo[1,5-d][1,4]benzodiazepin-10-carboxaldehydoxim werden in 60 ml Dimethylformamid gelöst, mit 0,1 g N-Chlorsuccinimid versetzt und 10 min bei Raumtemperatur gerührt. Danach leitet man über die Reaktionslösung ca. 20 ml Chlorwasserstoff und rührt 15 min bei 30°C Badtemperatur. Man gibt nochmals 1,4 g N-Chlorsuccinimid hinzu und rührt weitere 2,5 h bei 30°C. Man giesst dann das Gemisch in 300 ml Eiswasser, rührt 1 h bei 5°C und filtriert. Die Kristalle werden mit Wasser gewaschen und im Vakuum getrocknet. Man erhält 3,5 g (3-Fluor-9H-imidazo[1,5-a]-[1,2,4]triazolo[1,5-d][1,4]benzodiazepin-10-yl)carbonylchloridoxim vom Smp. über 165°C (Zers.).

8) 5. 2,7 g(3-Fluor-9H-imidazo[1,5-a][1,2,4]triazolo[1,5-d][1,4]benzodiazepin-10-yl)carbonylchloridoxim werden in 90 ml 1,2-Dimethoxyethan suspendiert und mit 4,1 g (2,6-Dimethylxylidino)acetonitril versetzt. Man erwärmt auf Rückflusstemperatur und tropft dann innert 25 min eine Lösung von 1,2 ml Triethylamin in 15 ml 1,2-Dimethoxyethan zu. Man erhitzt weitere 2 h auf Rückflusstemperatur und dampft dann im Vakuum ein. Der Rückstand wird durch Kieselgel chromatographiert. Mit Dichlormethan/Methanol (99:1 bis 97:3) eluiert man 0,7 g Produkt, welches aus Essigsäureethylester umkristallisiert wird. Man erhält 0,4 g 3-Fluor-10-[5-(2,6-xylidinomethyl)-1,2,4-oxadiazol-3-yl]-9H-imidazo[1,5-a][1,2,4]triazolo[1,5-d]-[1,4]benzodiazepin vom Smp. 201-205°C.

Beispiel 9

9) 1.1. 2,0 g N-[[3-[3-Fluor-9H-imidazo[1,5-a][1,2,4]triazolo[1,5-d][1,4]benzodiazepin-10-yl]-1,2,4-oxadiazol-5-yl]methyl]phthalimid (hergestellt gemäss Beispiel 1) 10.21.) werden in 100 ml Ethanol suspendiert und mit 0,25 ml Hydrazinhydrat 2 h auf Rückflusstemperatur erhitzt. Danach werden 10,0 ml 37%ige wässerige Salzsäure zugefügt. Das Gemisch wird 2,5 h auf Rückflusstemperatur erhitzt und anschliessend im Vakuum eingedampft. Der Rückstand wird in 100 ml Wasser suspendiert und 30 min bei 80°C gerührt. Nach dem Abkühlen auf 0° bis 5°C wird der Niederschlag abfiltriert und mit Wasser gewaschen. Das saure Filtrat wird mit 2N wässeriger Natriumhydroxid-Lösung alkalisch gestellt und fünfmal mit Dichlormethan extrahiert. Die vereinigten organischen Phasen werden mit gesättigter wässeriger Natriumchlorid-Lösung gewaschen, dann getrocknet und im Vakuum eingedampft. Der Rückstand wird aus Essigsäureethylester umkristallisiert. Man erhält 0,7 g 10-[5-(Aminomethyl)-1,2,4-oxadiazol-3-yl]-3-fluor-4H-imidazo[1,5-a][1,2,4]triazolo[1,5-d][1,4]benzodiazepin vom Smp. 257-259°C.

Auf analoge Weise erhält man:

9) 1.2. 10-[5-(Aminoethyl)-1,2,4-oxadiazol-3-yl]-3-fluor-4H-imidazo[1,5-a][1,2,4]triazolo[1,5-d][1,4]-benzodiazepin-hydrochlorid,Smp. 200-203°C. aus N-[2-[3-[3-Fluor-9H-imidazo[1,5-a][1,2,4]triazolo[1,5-d]-[1,4]benzodiazepin10-yl]-1,2,4-oxadiazol-5-yl]ethyl]phthalimid (hergestellt gemäss Beispiel 10) 1.26.).

Beispiel 10

10) 1.1. 1,4 g 10-[5-(Chlormethyl)-1,2,4-oxadiazol-3-yl]-3-fluor-9H-imidazo[1,5-a][1,2,4]triazolo[1,5-d][1,4]-benzodiazepin (hergestellt gemäss Beispiel 7) und 1,0 g Morpholin werden in 50 ml Dimethylformamid 7 h bei 80° Badtemperatur gerührt. Danach wird das Dimethylformamid im Hochvakuum abdestilliert. Der Rückstand wird in Dichlormethan gelöst und zweimal mit gesättigter wässeriger Natriumchlorid-Lösung gewaschen. Die wässerigen Phasen werden noch dreimal mir Dichlormethan extrahiert. Die vereinigten organischen Phasen werden getrocknet und im Vakuum eingedampft. Der Rückstand wird durch Kieselgel chromatographiert. Mit Dichlormethan/ Methanol 99:1 bis 96:4 eluiert man 1,7 g Produkt, welches aus Essigsäureethylester/Hexan umkristallisiert wird. Man erhält 1,4 g 3-Fluor-10-[5-(4-morpholinomethyl)-1,2,4-oxadiazol-3-yl]-9H-imidazo[1,5-a][1,2,4]triazolo[1,5-d][1,4]benzodiazepin vom Smp. 150-151°C.

Auf analoge Weise erhält man:

10) 1.2. 3-Fluor-10-[5-[(4-methyl-1-piperazinyl)methyl]-1,2,4-oxadiazol-3-yl]-9H-imidazo[1,5-a][1,2,4]-triazolo[1,5-d][1,4]benzodiazepin, Smp. 160-162°C, aus 10-[5-(Chlormethyl)-1,2,4-oxadiazol-3-yl]-3-fluor-9H-imidazo[1,5-a][1,2,4]triazolo[1,5-d][1,4]benzodiazepin und 1-Methylpiperazin;

10) 1.3. 3-Fluor-10-[5-[[4-(o-methoxyphenyl)-1-piperazinyl]methyl]-1,2,4-oxadiazol-3-yl]-9H-imidazo[1,5-a]-[1,2,4]triazolo[1,5-d][1,4]benzodiazepin, Smp. 209-210°C, aus 10-[5-(Chlormethyl)-1,2,4-oxadiazol-3-yl]-3-fluor-9H-imidazo[1,5-a][1,2,4]triazolo[1,5-d][1,4]benzodiazepin und 1-(o-Methoxyphenyl)piperazin;

10) 1.4. 3-Fluor-10-[5-[[4-(2-propinyl)-1-piperazinyl]methyl]-1,2,4-oxadiazol-3-yl]-9H-imidazo[1,5-a][1,2,4]-triazolo[1,5-d][1,4]benzodiazepin, Smp. 134-135°C, aus 10-[5-(Chlormethyl)-1,2,4-oxadiazol-3-yl]-3-fluor-9H-imidazo[1,5-a][1,2,4]triazolo[1,5-d][1,4]benzodiazepin und 1-(2-Propinyl)piperazin;

10) 1.5. 10-[5-[(Benzylmethylamino)methyl]-1,2,4-oxadiazol-3-yl]-3-fluor-9H-imidazo[1,5-a][1,2,4]triazolo-[1,5-d][1,4]benzodiazepin-methansulfonat, (1:1,2), Smp. ca. 150°C, aus 10-[5-(Chlormethyl)-1,2,4-oxadiazol-3-yl]-3-fluor-9H-imidazo[1,5-a][1,2,4]triazolo[1,5-d][1,4]benzodiazepin und N-Methylbenzylamin;

10) 1.6. 10-[5-[(Cyclohexylamino)methyl]-1,2,4-oxadiazol-3-yl]-3-fluor-9H-imidazo[1,5-a][1,2,4]triazolo[1,5-d][1,4]benzodiazepin, Smp. 169-171°C, aus 10-[5-(Chlormethyl)-1,2,4-oxadiazol-3-yl]-3-fluor-9H-imidazo-[1,5-a][1,2,4]triazolo[1,5-d][1,4]benzodiazepin und N-Methylcyclohexylamin;

10) 1.7. N-[3-(3-Fluor-9H-imidazo[1,5-a][1,2,4]triazolo[1,5-d][1,4]benzodiazepin-10-yl)-[1,2,4]oxadiazol-5-ylmethyl]-N-methyl-2-(morpholin-4-yl)ethylamin, Smp. 100-102°C, aus 10-[5-(Chlormethyl)-1,2,4-oxadiazol-3-yl]-3-fluor-9H-imidazo[1,5-a][1,2,4]triazolo[1,5-d][1,4]benzodiazepin und 4-[2-(Methylamino)-ethyl]morpholin;

10) 1.8. 3-Fluor-10-[5-[(2-methoxyethyl)methylaminomethyl]-1,2,4-oxadiazol-3-yl]-3-fluor-9H-imidazo[1,5-a][1,2,4]triazolo[1,5-d][1,4]benzodiazepin, Smp. 144-145°C, aus 10-[5-(Chlormethyl)-1,2,4-oxadiazol-3-yl]-3-fluor-9H-imidazo[1,5-a][1,2,4]triazolo[1,5-d][1,4]benzodiazepin und (2-Methoxyethyl)methylamin;

10) 1.9. 3-Fluor-10-[5-[[(2-hydroxyethyl)methylamino]methyl]-1,2,4-oxadiazol-3-yl]-3-fluor-9H-imidazo[1,5-a][1,2,4]triazolo[1,5-d][1,4]benzodiazepin, Smp. 154-155°C, aus 10-[5-(Chlormethyl)-1,2,4-oxadiazol-3-yl]-3-fluor-9H-imidazo[1,5-a][1,2,4]triazolo[1,5-d][1,4]benzodiazepin und 2-Methylaminoethanol;

10) 1.10. rac-[2,2-Dimethyl-[1,3]dioxolan-4-yl-methyl]-[3-(3-fluor-9H-imidazo[1,5-a][1,2,4]triazolo[1,5-d]-[1,4]benzodiazepin-10-yl)-[1,2,4]oxadiazol-5-yl-methyl]methylamin, Smp. 104-107°C, aus 10-[5-(Chlormethyl)-1,2,4-oxadiazol-3-yl]-3-fluor-9H-imidazo[1,5-a][1,2,4]triazolo[1,5-d][1,4]benzodiazepin und rac-N,2,2-Trimethyl-1,3-dioxolan-4-methanamin;

10) 1.11. N-[[3-[3-Fluor-9H-imidazo[1,5-a][1,2,4]triazolo[1,5-d][1,4]benzodiazepin-10-yl]-1,2,4-oxadiazol-5-yl]methyl]-N-methylglycin-ethylester, Smp. 114-117°C, aus 10-[5-(Chlormethyl)-1,2,4-oxadiazol-3-yl]-3-fluor-9H-imidazo-[1,5-a][1,2,4]triazolo[1,5-d][1,4]benzodiazepin, Sarcosinethylester-hydrochlorid und Triethylamin;

10) 1.12. 2-[[[3-[3-Fluor-9H-imidazo[1,5-a][1,2,4]triazolo[1,5-d][1,4]benzodiazepin-10-yl]-1,2,4-oxadiazol-5-yl]methyl]methylamino]acetamid, Smp. 206-207°C, aus N-[[3-[3-Fluor-9H-imidazo[1,5-a][1,2,4]triazolo-[1,5-d][1,4]benzodiazepin-10-yl]-1,2,4-oxadiazol-5-yl]methyl]-N-methylglycin ethylester und einer 25%-igen Lösung von Ammoniak in Methanol;

10) 1.13. [2-[[[3-[3-Fluor-10-imidazo[1,5-a][1,2,4]triazolo[1,5-d][1,4]benzodiazepin-10-yl]-1,2,4-oxadiazol-5-yl]methyl]methylamin]ethyl-carbaminsäure-tert-butylester, Smp. 138-140°C, aus 10-[5-(Chlormethyl)-1,2,4-oxadiazol-3-yl]-3-fluor-9H-imidazo[1,5-a][1,2,4]triazolo[1,5-d][1,4]benzodiazepin und [2-(Methylamino)ethyl]carbaminsäure-tert-butylester.

Beispiel 11

3,6 g 10-[5-[2-Benzyloxy)ethyl]-1,2,4-oxadiazol-3-yl]-3-fluor-9H-imidazo[1,5-a][1,2,4]triazolo[1,5-d][1,4]-

benzodiazepin (hergestellt gemäss Beispiel 1) 10.36.) werden mit 30 ml einer 30 %-igen Lösung von Bromwasserstoff in Essigsäure 3 h bei Raumtemperatur gerührt. Das Reaktionsgemisch wird in 300 ml Wasser gegossen und unter Rühren mit Natriumhydrogencarbonat auf pH 7 bis 8 gestellt. Danach wird mit Dichlormethan extrahiert. Die organischen Phasen werden getrocknet und im Vakuum eingedampft. Der Rückstand wird aus Essigsäureethylester umkristallisiert. Man erhält 3,0 g Essigsäure-[2-[3-(3-Fluor-9H-imidazo[1,5-a][1,2,4]triazolo[1,5-d][1,4]benzodiazepin-10-yl)-1,2,4-oxadiazol-5-yl]ethyl]ester vom Smp. 188-189 ° C.

Beispiel 12

3,0 g Essigsäure-[2-[3-(3-Fluor-9H-imidazo[1,5-a][1,2,4]triazolo[1,5-d][1,4]benzodiazepin-10-yl)-1,2,4-oxadiazol-5-yl]ethyl]ester werden in 150 ml Methanol suspendiert und nacheinander mit 20 ml Natriummethylat-Lösung (aus 20 ml Methanol und 0,1 g Natrium) und 0,2 ml Wasser versetzt. Man rührt 3 h bei Raumtemperatur, und dampft anschliessend im Vakuum ein. Der Rückstand wird durch Kieselgel chromatographiert. Mit Dichlormethan/Methanol 98:2 bis 95:5 eluiert man 0,9 g Produkt, welches aus Essigsäureethylester umkristallisiert wird. Man erhält 0,7 g 3-[3-Fluor-9H-imidazo[1,5-a][1,2,4]triazolo[1,5-d]-[1,4]benzodiazepin-10-yl]-1,2,4-oxadiazol-5-ethanol vom Smp. 242 ° C.

Beispiel 13

13) 1. 157 g 3-Aminothiophen-2-carbonsäure-methylester werden in 1,5 l Dioxan gelöst. Man gibt 36 ml Chloracetonitril dazu, kühlt auf 5 ° C ab, und leitet Chlorwasserstoff während 6,5 h ein. Nach 3,5 h gibt man nochmals 36 ml Chloracetonitril dazu und rührt 15 h bei Raumtemperatur. Nach nochmaliger Zugabe von 36 ml Chloracetonitril leitet man Chlorwasserstoff während 7,5 h ein, und lässt 15 h bei Raumtemperatur stehen. Die Suspension wird dann im Vakuum eingeengt. Der Rückstand wird in 1,5 l Wasser aufgenommen und mit Ammoniak auf pH 8 gestellt. Der Niederschlag wird abfiltriert, mit Wasser gewaschen und getrocknet. Man erhält 153 g rohes 2-(Chlormethyl)thieno[3,2-e]pyrimidin-4(1H)-on. Dieses wird durch Aufschlämmen in warmem Essigsäureethylester gereinigt. Man erhält 137,7 g reines 2-(Chlormethyl)thieno[3,2-e]pyrimidin-4(1H)-on vom Smp. 234-236 ° C.

13) 2. 40 g 2-(Chlormethyl)thieno[3,2-e]pyrimidin-4(1H)-on werden in 780 ml Chloroform suspendiert und mit 6,2 ml Phosphoroxychlorid versetzt. Man rührt 6 h bei 65 ° C, gibt nochmals 6,2 ml Phosphoroxychlorid dazu und rührt weitere 15 h bei 65 ° C. Man gibt 6,2 ml Phosphoroxychlorid und 12,2 ml N,N,4-Trimethylanilin dazu, rührt 3 h bei 65 ° C, gibt nochmals 9,5 ml N,N,4-Trimethylanilin dazu und rührt weitere 15 h bei 65 ° C. Man gibt 3,1 ml Phosphoroxychlorid und 4,75 ml N,N,4-Trimethylanilin dazu, rührt 4h bei 65 ° C, giesst alsdann das abgekühlte Reaktionsgemisch in 5 l gesättigte wässerige Natriumhydrogencarbonat-Lösung und rührt 30 min. Man extrahiert die wässerige Phase noch fünfmal mit Chloroform. Die Chloroform-Extrakte werden getrocknet und im Vakuum eingedampft. Der Rückstand wird mit Hexan gewaschen. Man erhält 32,2 g 4-Chlor-2-(chlormethyl)thieno[3,2-d]pyrimidin vom Smp. 101-102 ° C.

13) 3. 8,2 g 4-Chlor-2-(chlormethyl)thieno[3,2-d]pyrimidin werden in 500 ml absolutem Tetrahydrofuran gelöst. Die Lösung wird auf 5 ° C abgekühlt. Es werden 7 ml Hydrazinhydrat innert 5 min zugetropft, wobei eine Lösung entsteht und die Temperatur auf 15 bis 20 ° C ansteigt. Man rührt 15 h bei Raumtemperatur und dampft dann das Reaktionsgemisch im Vakuum ein. Der Rückstand wird mit 100 ml gesättigter wässeriger Natriumhydrogencarbonat-Lösung 30 min gerührt und dann abfiltriert. Die Kristalle werden mit Wasser neutral gewaschen und im Vakuum getrocknet. Man erhält 14,7 g 2-(Chlormethyl)-4-hydrazinothieno[3,2-d]pyrimidin. Durch Extrahieren des Filtrates mit Chloroform gewinnt man nochmals 1,0 g 2-(Chlormethyl)-4-hydrazinothieno[3,2-d]pyrimidin.

13) 4. 15,7 g 2-(Chlormethyl)-4-hydrazinothieno[3,2-d]pyrimidin werden in 280 ml Orthoameisensäure-triethylester suspendiert. Die Suspension wird unter Rühren aufgeheizt (Badtemp. 125 ° C) und das entstehende Ethanol abdestilliert. Nach 3 h kühlt man auf 0 ° C ab, filtriert den Niederschlag ab, und wäscht ihn mit Ethanol. Das Produkt wird im Vakuum getrocknet. Man erhält 15,2 g 5-(Chlormethyl)-thieno[2,3-e][1,2,4]triazolo[4,3-c]pyrimidin. Smp. über 400 ° C (Zers.).

13) 5. 15,2 g 5-(Chlormethyl)thieno[2,3-e][1,2,4]triazolo[4,3-c]pyrimidin werden in 650 ml Dioxan und 43 ml Dimethylformamid suspendiert und auf 5 ° C abgekühlt. Man gibt 86 ml 1 N Natriumhydroxid-Lösung innert 3 min dazu, und rührt 17 h bei Raumtemperatur. Das Reaktionsgemisch wird dann zwischen 1,5 l gesättigter wässeriger Natriumchlorid-Lösung und 0,7 l Chloroform verteilt. Die wässerige Phase wird alsdann mit 12 N Salzsäure schwach sauer (pH 6) gestellt. Man extrahiert noch fünfmal mit Chloroform. Die organischen Extrakte werden im Vakuum eingeengt. Der kristalline Rückstand wird aus Ethanol

umkristallisiert. Man erhält 5H-Thieno[2,3-f][1,2,4]triazolo[1,5-d][1,4]diazepin-6(7H)-on vom Smp. 213-215 °C.

13) 6. 12,5 g 5H-Thieno[2,3-f][1,2,4]triazolo[1,5-d][1,4]diazepin-6(7H)-on werden in 760 ml Chloroform und 87 ml N,N,4-Trimethylanilin suspendiert, und 17,4 ml Phosphoroxychlorid zugegeben. Das Gemisch wird 16 h bei Rückflusstemperatur gerührt. Man gibt nochmals 3,6 ml N,N,4-Trimethylanilin und 1,7 ml Phosphoroxychlorid hinzu und erhitzt weitere 4 h auf Rückflusstemperatur. Man gibt nochmals 3,6 ml N,N,4-Trimethylanilin und 1,7 ml Phosphoroxychlorid hinzu und erhitzt weitere 2,5 h auf Rückflusstemperatur. Das abgekühlte Reaktionsgemisch wird in 2 l gesättigte wässerige Natriumhydrogencarbonat-Lösung gegossen und 30 min intensiv gerührt. Die wässerige Phase wird abgetrennt und noch 4 mal mit Chloroform extrahiert. Die vereinigten Chloroform-Extrakte werden im Vakuum eingedampft. Der Rückstand besteht aus einem Gemisch von 6-Chlor-5H-thieno[2,3-f][1,2,4]triazolo[1,5-d][1,4]diazepin und N,N,4-Trimethylanilin, das in 100 ml Tetrahydrofuran gelöst wird.

Eine Lösung von 10,2 g Isocyanessigsäureethylester in 360 ml Tetrahydrofuran wird auf-25 °C gekühlt. Es werden 10,4 g Kalium-tert-butylat zugegeben und sodann 1 h bei -10 °C gerührt. Diese Lösung wird auf-60 °C gekühlt und mit der Lösung von 6-Chlor-5H-thieno[2,3-f][1,2,4]triazolo[1,5-d][1,4]-diazepin und N,N,4-Trimethylanilin versetzt, wobei die Temperatur auf -15 °C ansteigt. Man rührt 2 h bei Raumtemperatur und stellt dann mit Essigsäure auf pH 7. Man verteilt das Reaktionsgemisch zwischen gesättigter wässeriger Natriumhydrogencarbonat-Lösung und Chloroform. Die wässerige Phase wird fünfmal mit Chloroform extrahiert. Die organischen Extrakte werden im Vakuum eingedampft, der Rückstand in Ethanol aufgenommen und mit Hexan verdünnt. Man erhält 12,0 g 8H-Imidazo[1,5-a]thieno-[2,3-f][1,2,4]triazolo[1,5-d][1,4]diazepin-7-carbonsäureethylester vom Smp. 231-232 °C.

13) 7. 11,9 g 8H-Imidazo[1,5-a]thieno[2,3-f][1,2,4]triazolo[1,5-d][1,4]diazepin-7-carbonsäureethylester werden in einer Lösung aus 125 ml Ethanol, 3,8 g Kaliumhydroxid und 37,5 ml Wasser 2,5 h bei 80 °C gerührt. Man dampft die Lösung im Vakuum ein, löst den Rückstand in 800 ml Wasser und neutralisiert auf pH 6 bis 7 mit verdünnter Salzsäure. Der Niederschlag wird abfiltriert und getrocknet. Man erhält 9,65 g 8H-Imidazo[1,5-a]thieno[2,3-f][1,2,4]triazolo[1,5-d][1,4]diazepin-7-carbonsäure.

13) 8. Zu einer Suspension von 9,5 g 8H-Imidazo[1,5-a]thieno[2,3-f][1,2,4]triazolo[1,5-d][1,4]diazepin-7-carbonsäure in 300 ml Dimethylformamid werden 11,4 g 1,1'-Carbonyldiimidazol zugegeben. Man rührt dieses Gemisch 5 h bei Raumtemperatur und gibt dann 450 ml konzentrierte wässerige Ammoniak-Lösung dazu. Nach 30 min Rühren wird die klare Lösung mit 1200 ml Wasser versetzt. Der entstandene Niederschlag wird abfiltriert, mit Wasser gewaschen und im Vakuum getrocknet. Man erhält 8,6 g 8H-Imidazo[1,5-a]thieno[2,3-f][1,2,4]triazolo[1,5-d][1,4]diazepin-7-carboxamid vom Smp. 347-349 °C.

13) 9. Zu einer Suspension von 8,6 g 8H-Imidazo[1,5-a]thieno[2,3-f][1,2,4]triazolo[1,5-d][1,4]diazepin-7-carboxamid in 215 ml Dimethylformamid und 6,1 ml Pyridin werden 6,1 ml Trifluoressigsäureanhydrid innert 15 min bei 5° bis 7 °C zugegeben, und 4 h bei Raumtemperatur gerührt. Man gibt nochmals 6,1 ml Pyridin und 6,1 ml Trifluoressigsäureanhydrid dazu. Nach 15 h Rühren bei Raumtemperatur wird das Gemisch in 1,3 l eiskalte gesättigte wässerige Natriumhydrogencarbonat-Lösung gegossen. Man extrahiert mehrmals mit Dichlormethan. Die Dichlormethan-Extrakte werden mit gesättigter wässeriger Natriumchlorid-Lösung gewaschen, getrocknet, und im Vakuum eingedampft. Der Rückstand wird aus Methanol/Chloroform umkristallisiert. Man erhält 4,7 g 8H-Imidazo[1,5-a]thieno[2,3-f][1,2,4]triazolo[1,5-d]-[1,4]diazepin-7-carbonitril vom Smp. 275-276 °C.

13) 10. Zu einer Suspension von 4,5 g 8H-Imidazo[1,5-a]thieno[2,3-f][1,2,4]triazolo[1,5-d][1,4]diazepin-7-carbonitril in 85 ml Ethanol werden zunächst 1,45 g Hydroxylaminhydrochlorid, dann eine Lösung von 1,85 g Natriumhydrogencarbonat in 22,5 ml Wasser zugegeben. Man rührt 1,5 h bei Rückflusstemperatur. Der entstandene Niederschlag wird abfiltriert und mit Diethylether gewaschen. Man erhält 5,15 g 8H-Imidazo[1,5-a]thieno[2,3`f][1,2,4]triazolo[1,5-d][1,4]diazepin-7-carboxamidoxim vom Smp. 267-269 °C. Durch Einengen des Filtrates lassen sich weitere 0,1 g dieser Verbindung gewinnen.

13) 11.1. 0,52 ml Cyclopropancarbonsäure werden in 20 ml Dimethylformamid gelöst und mit 0,97 g 1,1'-Carbonyldlimidazol versetzt. Man rührt 2,5 h bei Raumtemperatur, dann gibt man 1,15 g 8H-Imidazo-[1,5-a]thieno[2,3-f][1,2,4]triazolo[1,5-d][1,4]diazepin-7-carboxamidoxim dazu und rührt 16 h bei 80 °C. Das Reaktionsgemisch wird im Hochvakuum eingedampft. Der Rückstand wird in 10 ml Cyclopropancarbonsäure gelöst und 3 h auf 130 °C erhitzt. Die Lösung wird im Vakuum eingedampft. Der Rückstand wird zwischen Dichlormethan und gesättigter wässeriger Natriumhydrogencarbonat-Lösung verteilt. Die wässerige Phase wird noch dreimal mit Dichlormethan extrahiert. Die organischen Extrakte werden eingedampft und der Rückstand durch 50 g Kieselgel chromatographiert. Mit Dichlormethan/Ethanol 99:1 eluiert man 7-(5-Cyclopropyl-1,2,4-oxadiazol-3-yl)8H-imidazo[1,5-a]thieno[2,3-f][1,2,4]triazolo[1,5-d][1,4]-diazepin. Dieses wird aus Ethanol umkristallisiert. Man erhält 0,94 g reines 7-(5-Cyclopropyl-1,2,4-oxadiazol-3-yl)-8-imidazo[1,5-a]thieno[2,3-f][1,2,4]triazolo[1,5-d][1,4]diazepin vom Smp. 196 °C.

Auf analoge Weise erhält man:
13) 11,2. 7-(5-Isopropyl-1,2,4-oxadiazol-3-yl)-8H-imidazo[1,5-a]thieno[2,3-f][1,2,4]triazolo[1,5-d][1,4]-diazepin vom Smp. 180-181 ° C, aus 8H-Imidazo[1,5-a]thieno[2,3-f][1,2,4]-triazolo[1,5-d][1,4]diazepin-7-carboxamidoxim und Isobuttersäure (anstatt Cyclopropancarbonsäure).

Beispiel 14

14) 1. 17,3 g 2-Aminothiophen-3-carbonitril werden in 150 ml Dioxan gelöst. Man gibt 13,3 g Chloracetonitril dazu, kühlt auf 2 ° C ab, leitet während 7 h Chlorwasserstoff ein und rührt anschliessend 15 h bei Raumtemperatur. Das Reaktionsgemisch wird im Vakuum eingedampft. Der Rückstand wird in 500 ml Wasser aufgeschlämmt und die Kristalle abfiltriert. Man erhält 26,2 g rohes 4-Chlor-2-(chlormethyl)-thieno[2,3-d]pyrimidin.Nach Umkristallisieren aus Hexan erhält man reines Produkt vom Smp. 66-68 ° C.
Auf analoge Weise, wie im Beispiel 13 beschrieben, erhält man:
14) 2. 5-(Chlormethyl)thieno[3,2-e][1,2,4]triazolo[4,3-c]pyrimidin, Smp. über 120 ° C (Zers.), aus 4-Chlor-2-(chlormethyl)thieno[2,3-d]pyrimidin durch Umsetzung mit Hydrazinhydrat und anschliessend mit Orthoameisensäure-triethylester;
14) 3. 5H-Thieno[3,2-f][1,2,4]triazolo[1,5-d][1,4]diazepin-6(7H)-on, Smp. 260 ° C, aus 5-(Chlormethyl)-thieno[3,2-e][1,2,4]triazolo[4,3-c]pyrimidin durch Umsetzung mit Natriumhydroxid in wässerigem Dioxan.
Auf analoge Weise, wie im Beispiel 1 beschrieben, erhält man:
14) 4. 8H-Imidazo[1,5-a]thieno[3,2-f][1,2,4]triazolo[1,5-d][1,4]diazepin-9-carbonsäure-tert-butylester, Smp. 217 ° C, aus 5H-Thieno[3,2-f][1,2,4]triazolo[1,5-d][1,4]diazepin-6(7H)-on. Dieses wird mittels Phosphoroxychlorid und N,N,4-Trimethylanilin in Chloroform in 6-Chlor-5H-thieno[3,2-f][1,2,4]triazolo[1,5-d]diazepin überführt, welches mit deprotoniertem Isocyanessigsäure-tert-butylester umgesetzt wird;
14) 5. 8H-Imidazo[1,5-a]thieno[3,2-f][1,2,4]triazolo[1,5-d][1,4]diazepin9-carbonsäure, Smp. 266-267 ° C, aus 8H-Imidazo[1,5-a]thieno[2,3-f][1,2,4]triazolo[1,5-d][1,4]diazepin-9-carbonsäure-tert-butylester durch Umsetzung mit Trifluoressigsäure.
14) 6. Zu einer Suspension von 2,0 g 8H-Imidazo[1,5-a]thieno[3,2-f][1,2,4]triazolo[1,5-d][1,4]diazepin-9-carbonsäure in 100 ml Dimethylformamid werden 2,45 g 1,1'-Carbonyldiimidazol zugegeben. Man rührt 2 h bei Raumtemperatur, gibt dann 1,7 g Cyclopropylcarboxamidoxim dazu und rührt 16 h bei 80 ° C. Man dampft das Reaktionsgemisch im Hochvakuum ein, löst den Rückstand in 80 ml Essigsäure und erhitzt 1,5 h auf 110 ° C. Die Lösung wird im Vakuum eingedampft. Der Rückstand wird in Dichlormethan und gesättigter wässeriger Natriumhydrogencarbonat-Lösung aufgenommen; die wässerige Phase wird viermal mit Dichlormethan extrahiert. Der Rückstand aus den Dichlormethan-Extrakten wird in Essigsäureethylester gelöst und durch 200 g Kieselgel chromatographiert. Das gewünschte Produkt wird mit Essigsäureethylester/Ethanol 97:3 eluiert (1,55 g) und aus Essigsäureethylester umkristallisiert. Man erhält 1,3 g 9-(3-Cyclopropyl-1,2,4-oxadiazol-5-yl)-8H-imidazo[1,5-a]thieno[3,2-f][1,2,4]triazolo[1,5-d][1,4]-diazepin vom Smp. 219-220 ° C.

Beispiel 15

15) 1.1. 10,0 g 4-Chinolinol werdenin 30 g Hydrazinhydrat in einem Autoilaven 6 h auf 170 ° C erhitzt. Nach dem Abkühlen wird das Reaktionsgemisch in wässeriger Salzsäure gelöst (Gesamtvolumen der Lösung ca. 300 ml, pH ca. 1). Dann wird mit konzentrierter wässeriger Ammoniak-Lösung schwach alkalisch gestellt (pH ca. 9). Die ausgefallenen Kristalle werden abfiltriert und getrocknet. Man erhält 8,0 g 2-(1H-Pyrazol-3-yl)anilin vom Smp. 122-123 ° C.
Auf analoge Weise erhält man:
15) 1.2. 4-Fluor-2-(1H-pyrazol-3-yl)anilin, Smp. 91-92 ° C, aus 6-Fluor-4-chinolinol.
15) 2.1. 8,6 g 2-(1H-Pyrazol-3-yl)anilin werden in 800 ml Tetrahydrofuran gelöst. Man gibt 75,3 g Kaliumcarbonat dazu, kühlt auf 5 ° C ab und tropft dann innert 10 min eine Lösung von 6,7 ml Chloracetylchlorid in 40 ml Diethylether dazu. Man rührt 30 min bei 5 ° C, und tropft dann nochmals eine Lösung von 0,67 ml Chloracetylchlorid in 5 ml Diethylether dazu. Man rührt weitere 15 min und filtriert sodann den unlöslichen Teil ab. Das Filtrat wird im Vakuum eingedampft. Der Rückstand wird zwischen gesättigter wässeriger Natriumhydrogencarbonat-Lösung und Dichlormethan verteilt. Die wässerige Phase wird noch mehrmals mit Dichlormethan extrahiert. Die organischen Extrakte werden getrocknet und im Vakuum eingedampft. Der Rückstand wird in 560 ml einer 0.41 N Lösung von Chlorwasserstoff in Dioxan aufgenommen und 1 h bei 90 ° C gerührt. Die Lösung wird im Vakuum eingedampft und der Rückstand zwischen gesättigter wässeriger Natriumhydrogencarbonat-Lösung und Dichlormethan verteilt. Das Dichlormethan-Extrakt wird im Vakuum eingedampft. Man erhält 11,4 g 5-(Chlormethyl)pyrazolo[1,5-c]-chinazolin. Nach Umkristallisation aus Diisopropylether beträgt der Smp.135-136 ° C.

Auf analoge Weise erhält man:

15) 2.2. 5-(Chlormethyl)-9-flourpyrazolo[1,5-c]chinazolin, Smp. 155-157 ° C, aus 4-Fluor-2-(1H-pyrazol-3-yl)anilin.

15) 3.1. Eine Lösung von 11,4 g 5-(Chlormethyl)pyrazolo[1,5-c]chinazolin in 160 ml Dioxan wird innert 15 min in ein auf 0 ° bis 5 ° C gekühltes Gemisch von 130 ml 1 N wässeriger Natriumhydroxid-Lösung und 130 ml Dioxan getropft. Man entfernt die Kühlung und rührt 2,5 h bei Raumtemperatur. Man giesst sodann das Reaktionsgemisch in 600 ml gesättigte wässerige Natriumchlorid-Lösung und extrahiert viermal mit Dichlormethan. Die Dichlormethan-Extrakte werden im Vakuum eingedampft. Der Rückstand (5,4 g) wird aus Dichlormethan/Diethylether kristallisiert. Man erhält 4,15 g 5H-Pyrazolo[1,5-d][1,4]-benzodiazepin-6(7H)-on vom Smp. 236-240 ° C.

Auf analoge Weise erhält man:

15) 3.2. 10-Fluor-5H-pyrazolo[1,5-d][1,4]benzodiazepin-6(7H)-on, Smp. 258-260 ° C, aus 5-(Chlormethyl)-9-flourpyrazolo[1,5-c]chinazolin.

15) 4.1. 2,03 g Isocyanessigsäureethylester werden in 85 ml Tetrahydrofuran gelöst. Man kühlt auf -15 ° C und gibt 1,97 g Kalium-tert-butylat dazu. Man rührt noch 1 h bei -10 ° C.

Andererseits löst man 2,8 g 5H-Pyrazolo[1,5-d][1,4]benzodiazepin-6(7H)-on in 70 ml Dimethylformamid. Bei -15 ° C gibt man 0,8 g einer ca. 55%-igen Dispersion von Natriumhydrid in Mineralöl dazu und rührt 1 h bei -10 ° C. Alsdann kühlt man die Lösung auf -40 ° C, tropft 3,8 ml Diphenylphosphorylchlorid innerhalb von 10 min dazu, rührt 30 min bei -40 ° C und kühlt sodann auf -60 ° C ab. Bei dieser Temperatur gibt man die vorher zubereitete Suspension des Isocyanessigsäureethylester-kaliumsalzes dazu und entfernt sodann die Kühlung. Man rührt 2,5 h bei Raumtemperatur und versetzt dann mit 0.55 ml Essigsäure. Das Gemisch wird in 1,25 l gesättigte wässerige Natriumhydrogencarbonat-Lösung gegossen und dreimal mit Essigsäureethylester extrahiert. Die organischen Phasen werden noch je einmal mit gesättigter wässeriger Natriumhydrogencarbonat- und Natriumchlorid-Lösung gewaschen und dann im Vakuum eingedampft. Der Rückstand wird im 300 ml Essigsäureethylester und 300 ml Hexan gelöst und durch eine Kieselgelsäule chromatographiert. Mit Hexan/Essigsäureethylester 1:1 werden zunächst unverändertes Ausgangsmaterial (0,41 g) und Nebenprodukte eluiert. Das gewünschte Produkt wird mit Hexan/Essigsäureethylester 3:7 und 1:9 eluiert. Nach Umkristallisieren aus Essigsäureethylester/Diisopropylether/ Diethylether erhält man 2,95 g 9H-Imidazo[1,5-a]pyrazolo[1,5-d]-[1,4]benzodiazepin-8-carbonsäureethylester vom Smp. 180-182 ° C.

Auf analoge Weise erhält man:

15) 4.2. 2-Fluor-9H-Imidazo[1,5-a]pyrazolo[1,5-d][1,4]benzodiazepin-8-carbonsäureethylester Smp. 213-215 ° C, aus 10-Fluor-5H-pyrazolo[1,5-d][1,4]benzodiazepin-6(7H)-on.

15) 5.1. 2,9 g 9H-Imidazo[1,5-a]pyrazolo[1,5-d][1,4]benzodiazepin-8-carbonsäureethylester werden in 250 ml Ethanol suspendiert. Man gibt eine Lösung von 1,0 g Kaliumhydroxid in 25 ml Wasser dazu und erhitzt 45 min auf 80 ° C. Die Lösung wird im Vakuum eingedampft; der Rückstand in 100 ml Wasser gelöst und mit 20 ml 1 N Salzsäure versetzt. Man rührt 1 h im Eisbad und filtriert dann die Kristalle ab. Nach dem Trocknen erhält man 2,5 g 9H-Imidazo[1,5-a]pyrazolo[1,5-d][1,4]benzodiazepin-8-carbonsäure vom Smp. 256-257 ° C (Zers.).

Auf analoge Weise erhält man:

15) 5.2. 2-Fluor-9H-Imidazo[1,5-a]pyrazolo[1,5-d][1,4]benzodiazepin-8-carbonsäure, Smp. 258-260 ° C, aus 2-Fluor-9H-Imidazo[1,5-a]pyrazolo[1,5-d][1,4]benzodiazepin-8-carbonsäureethylester.

15) 6.1. Zu einer Lösung von 2,5 g 9H-Imidazo[1,5-a]pyrazolo[1,5-d][1,4]benzodiazepin-8-carbonsäure in 30 ml Dimethylformamid werden 3,0 g 1,1'-Carbonyldiimidazol zugegeben. Man rührt 4 h bei Raumtemperatur. Dan gibt man 2,1 g Cyclopropancarboxamidoxim dazu und rührt 20 h bei 80 ° C. Das Reaktionsgemisch wird im Hochvakuum eingedampft. Der Rückstand wird in 50 ml Essigsäure gelöst und 2,5 h auf 110 ° C erhitzt. Man dampft im Vakuum ein und verteilt den Rückstand zwischen Essigsäureethylester und gesättigter wässeriger Natriumhydrogencarbonat-Lösung. Die wässerige Phase wird dreimal mit Essigsäureethylester extrahiert, die organischen Extrakte je einmal mit gesättigter Natriumhydrogencarbonat- und mit Natriumchlorid-Lösung gewaschen und im Vakuum eingeengt. Der Rückstand (2,6 g) wird aus Essigsäureethylester und aus Ethanol umkristallisiert. Man erhält 2,15 g 8-(3-Cyclopropyl-1,2,4-oxadiazol-5-yl)-9H-imidazo[1,5-a]pyrazolo[1,5-d][1,4]benzodiazepin vom Smp. 208-210 ° C.

Auf analoge Weise erhält man:

15) 6.2. 8-(3-Cyclopropyl-1,2,4-oxadiazol-5-yl)-2-fluor-9H-imidazo[1,5-a]pyrazolo[1,5-d][1,4]benzodiazepin vom Smp. 217-218 ° C, aus 2-Fluor-9H-imidazo[1,5-a]pyrazolo[1,5-d][1,4]benzodiazepin-8-carbonsäure.

Beispiel 16

16) 1.1. Zu einer Suspension von 4,0 g 9H-Imidazo[1,5-a]pyrazolo[1,5-d][1,4]benzodiazepin-8-carbonsäure in 35 ml Dimethylformamid werden 4,8 g 1,1'-Carbonyldiimidazol zugegeben. Man rührt dieses Gemisch 3 h bei Raumtemperatur und gibt dann 150 ml konzentrierte wässerige Ammoniak-Lösung und 300 ml Wasser dazu. Nach 1 h Rühren bei 5 ° C wird der entstandene Niederschlag abfiltriert, mit Wasser gewaschen und im Vakuum getrocknet. Man erhält 4,0 g 9H-Imidazo[1,5-a]pyrazolo[1,5-d][1,4]-benzodiazepin-8-carboxamid vom Smp. 316-320 ° C.

Auf analoge Weise erhält man:

16) 1.2. 2-Fluor-9H-Imidazo[1,5-a]pyrazolo[1,5-d][1,4]benzodiazepin-8-carboxamid, Smp. 342-344 ° C, aus 2-Fluor-9H-imidazo[1,5-a]pyrazolo[1,5-d][1,4]benzodiazepin-8-carbonsäure.

16) 2.1. Zu einer Suspension von 3,9 g 9H-Imidazo[1,5-a]pyrazolo[1,5-d][1,4]benzodiazepin-8-carboxamid in 80 ml Dimethylformamid und 10 ml Pyridin werden 4,3 ml Trifluoressigsäureanhydrid bei 15 ° C zugegeben, und dann 16 h bei Raumtemperatur gerührt. Das Reaktionsgemisch wird in ein Gemisch von 1 l gesättigter wässeriger Natriumhydrogencarbonat-Lösung und 400 ml Essigsäureethylester gegossen. Man extrahiert noch einmal mit Essigsäureethylester. Die organischen Extrakte werden mit gesättigter wässeriger Natriumchlorid-Lösung gewaschen, getrocknet, und im Vakuum eingedampft. Der Rückstand wird aus Essigsäureethylester und Dietylether umkristallisiert. Man erhält 3,15 g 9H-Imidazo[1,5-a]-pyrazolo[1,5-d][1,4]benzodiazepin-8-carbonitril vom Smp. 248-250 ° C.

Auf analoge Weise erhält man:

16) 2.2. 2-Fluor-9H-Imidazo[1,5-a]pyrazolo[1,5-d][1,4]benzodiazepin-8-carbonitril, Smp. 261-262 ° C, aus 2-Fluor-9H-imidazo[1,5-a]pyrazolo[1,5-d][1,4]benzodiazepin-8-carboxamid.

16) 3.1. Zu einer Suspension von 3,0 g 9H-Imidazo[1,5-a]pyrazolo[1,5-d][1,4]benzodiazepin-8-carbonitril in 56 ml Ethanol werden zunächst 1,04 g Hydroxylaminhydrochlorid, dann eine Lösung von 1,32 g Natriumhydrogencarbonat in 16 ml Wasser zugegeben. Man rührt 1,25 h bei 80 ° C. Man kühlt ab und gibt 40 ml Wasser dazu. Nach 15 min wird der entstandene Niederschlag abfiltriert. Man erhält 2,6 g 9H-Imidazo[1,5-a]pyrazolo[1,5-d][1,4]benzodiazepin-8-carboxamidoxim. Durch Einengen des Filtrates und Umkristallisieren des Rückstandes aus Ethanol/Wasser erhält man weitere 0,4 g dieser Verbindung.

Auf analoge Weise erhält man:

16) 3.2. 2-Fluor-9H-Imidazo[1,5-a]pyrazolo[1,5-d][1,4]benzodiazepin-8-carboxamidoxim aus 2-Fluor-9H-imidazo[1,5-a]pyrazolo[1,5-d][1,4]benzodiazepin-8-carbonitril.

16) 4.1. 1,3 ml Cyclopropancarbonsäure werden in 100 ml Dimethylformamid gelöst und mit 2,6 g 1,1'-Carbonyldiimidazol versetzt. Man rührt 3 h bei Raumtemperatur, dann gibt man 3,0 g 9H-Imidazo[1,5-a]-pyrazolo[1,5-d][1,4]benzodiazepin-8-carbamidoxim dazu und rührt 18 h bei 80 ° C. Das Gemisch wird im Hochvakuum eingedampft. Der Rückstand wird in 25 ml Cyclopropancarbonsäure gelöst und 3 h auf 130 ° C erhitzt. Die Lösung wird im Hochvakuum eingedampft. Der Rückstand wird zwischen Dichlormethan und gesättigter wässeriger Natriumhydrogencarbonat-Lösung verteilt. Die wässerige Phase wird noch zweimal mit Dichlormethan extrahiert. Die organischen Extrakte werden eingedampft und der Rückstand durch 300 g Kieselgel chromatographiert. Mit Dichlormethan/Ethanol 99:1 eluiert man 2,65 g rohes 8-(5-Cyclopropyl-1,2,4-oxadiazol-3-yl)-9H-imidazo[1,5a]pyrazolo[1,5-d][1,4]benzodiazepin. Dieses wird aus Essigsäureethylester umkristallisiert. Man erhält 2,1 g reines 8-(5-Cyclopropyl-1,2,4-oxadiazol-3-yl)-9H-imidazo[1,5-a]pyrazolo[1,5-d][1,4]benzodiazepin vom Smp. 207-209 ° C.

Auf analoge Weise erhält man:

16) 4.2. 8-(5-Cyclopropyl-1,2,4-oxadiazol-3-yl)-2-fluor-9H-imidazo[1,5-a]pyrazolo[1,5-d][1,4]benzodiazepin, Smp. 200-202 ° C, aus2-Fluor-9H-imidazo[1,5-a]pyrazolo[1,5-d][1,4]benzodiazepin-8-carboxamidoxim.

Beispiel 17

17) 1. 84.6 g 2H-Thieno[3,2-d][1,3]oxazin-2,4(1H)-dion und 192,3 g N-(2,4-dimethoxybenzyl)glycin werden in 450 ml Dimethylformamid und 150 ml Wasser während 5 Tagen bei 50 ° C gerührt. Das Lösungsmittel wird im Vakuum abgedampft und der Rückstand in 600 ml Essigsäure gelöst. Das Reaktionsgemisch wird 2 h auf Rückflusstemperatur erhitzt und anschliessend im Vakuum eingedampft. Der Rückstand wird in 1 l Dichlormethan aufgekocht. Die enstandenen Kristalle werden abfiltriert und in heissem Dimethylformamid gelöst. Es wird Wasser zugegeben bis zu Beginn der Kristallisation. Man erhält 72,5 g 7-(2,4-Dimethoxybenzyl)-6,7-dihydro-5H-thieno[3,2-e][1,4]diazepin-5,8-dion.

17) 2. 89,2 g 7-(2,4-Dimethoxybenzyl)-6,7-dihydro-5H-thieno[3,2-e][1,4]diazepin-5,8-dion werden in 350 ml 1,2-Dichlorethan und 295 ml N,N,4-Trimethylanilin suspendiert und auf Rückflusstemperatur erhitzt und dann 46 ml Phosphoroxychlorid zugetropft. Die dunkle Reaktionslösung wird 1 h auf Rückflusstemperatur erhitzt, auf Raumtemperatur abgekühlt und vorsichtig in ein Gemisch von 240 g Natriumhydrogencarbonat und 700 ml Wasser gegossen. Die wässerige Phase wird abgetrennt und mit Dichlormethan extrahiert. Die vereinigten organischen Phasen werden mit Wasser nachgewaschen, getrocknet (Magnesiumsulfat), und im Vakuum eingedampft. Man erhält so die Iminchlorid-Lösung I.

Andererseits werden 48,4 g Kalium-tert-butylat in 190 ml Dimethylformamid gelöst und bei -30° bis -20°C tropfenweise mit 46 ml Isocyanessigsäureethylester versetzt Die erhaltene Lösung wird 0,5 h bei -30° bis -20°C nachgerührt. Dann wird bei -20° bis -10°C die Iminchloridlösung I zugetropft. Das Reaktionsgemisch wird auf Raumtemperatur erwärmt, mit 30 ml Essigsäure neutralisiert, und in 700 ml Wasser gegossen. Man extrahiert mehrmals mit Dichlormethan. Die organischen Phasen werden getrocknet und im Vakuum eingedampft. Der feste Rückstand wird aus Essigsäureethylester umkristallisiert. Man erhält 73,2 g 5-(2,4-Dimethoxybenzyl)-5,6-dihydro-6-oxo-4H-imidazo[1,5-a]thieno[2,3-f][1,4]diazepin-3-carbonsäureethylester vom Smp. 168°C.

17) 3. 3,6 g 5-(2,4-Dimethoxybenzyl)-5,6-dihydro-6-oxo-4H-imidazo[1,5-a]thieno[2,3-f][1,4]diazepin-3-carbonsäureethylester werden in 12,5 ml Trifluoressigsäure während 2 h auf Rückflusstemperatur erhitzt. Das Reaktionsgemisch wird dann im Vakuum eingedampft, und der Rückstand zwischen Dichlormethan und Wasser verteilt. Man neutralisiert durch Zugabe von Natriumhydrogencarbonat. Die erhaltene Emulsion wird durch Dicalit filtriert und das Dicalit mit Dichlormethan nachgewaschen. Die Phasen des Filtrates werden getrennt, und die wässerige Phase nochmals mit Dichlormethan extrahiert. Die vereinigten organischen Phasen werden getrocknet und im Vakuum eingedampft. Der Rückstand wird in Essigsäureethylester aufgekocht; die erhaltenen Kristalle werden abfiltriert. Man erhält 1,6 g 5,6-Dihydro-6-oxo-4H-imidazo[1,5-a]thieno[2,3-f][1,4]diazepin-3-carbonsäureethylester vom Smp. 263-264°C.

17) 4. 24,07 g 5,6-Dihydro-6-oxo-4H-imidazo[1,5-a]thieno[2,3-f][1,4]diazepin-3-carbonsäureethylester werden in 260 ml Toluol suspendiert und mit 22,73 g Lawesson-Reagenz versetzt. Das Gemisch wird 1,25 h auf Rückflusstemperatur erhizt, dann abgekühlt und filtriert. Der Filterkuchen wird getrocknet Man erhält 21,78 g 5,6-Dihydro-6-thioxo-4H-imidazo[1,5-a]thieno[2,3-f][1,4]diazepin-3-carbonsäureethylester.

17) 5. Durch Umsetzung von 5,6-Dihydro-6-thioxo-4H-imidazo[1,5-a]thieno[2,3-f][1,4]diazepin-3-carbonsäureethylester mit Aminoacetaldehyddimethylacetal, in analoger Weise wie in Beispiel 5 beschrieben, erhält man 6-[(2,2-Dimethoxyethyl)amino]-4H-imidazo[1,5-a]thieno[2,3-f][1,4]diazepin-3-carbonsäureethylester vom Smp. 134°C.

17) 6. Durch Erhitzen von 6-[(2,2-Dimethoxyethyl)amino]-4H-imidazo[1,5-a]thieno[2,3-f][1,4]diazepin-3-carbonsäureethylester in Essigsäure während 1,5 h, in analoger Weise wie in Beispiel 5 beschrieben, erhält man 8H-Diimidazo[1,5-a:1',2'-d]thieno[2,3-f][1,4]diazepin-7-carbonsäureethylester vom Smp. 203-204°.

17) 7. Durch Hydrolyse von 8H-Diimidazo[1,5-a:1',2'-d]thieno[2,3-f][1,4]diazepin-7-carbonsäureethylester mit Kaliumhydroxid in Ethanol/Wasser, in analoger Weise wie in Beispiel 5 beschrieben, erhält man 8H-Diimidazo[1,5-a:1',2'-d]thieno[2,3-f][1,4]diazepin-7-carbonsäure vom Smp. 225-227°C.

17) 8. Durch Umsetzung von 8H-Diimidazo[1,5-a:1',2'-d]]thieno[2,3-f][1,4]diazepin-7-carbonsäure mit 1,1'-Carbonyldiimidazol, dann mit Cyclopropansäureamidoxim und anschliessendem Erhitzen in Essigsäure, in analoger Weise wie in Beispiel 6 beschrieben, erhält man 7-(3-Cyclopropyl-1,2,4-oxadiazol-5-yl)-8H-diimidazo[1,5-a:1',2'-d]thieno[2,3-f][1,4]diazepin vom Smp. 231-233°C.

Beispiel 18

18) 1. Eine Lösung von 12,0 mg (0,08 mmol) 3-Cyclopropyl-5-(isocyanomethyl)-1,2,4-oxadiazol (hergestellt gemäss U.S.-Patentschrift Nr. 4'622'320 vom 11. Nov. 1986) in 0,1 ml Dimethylformamid wird mit einer Lösung von 0.24 mmol 6-Chlor-10-fluor-5H-triazolo[1,5-d][1,4]benzodiazepin und N,N,4-Trimethylanilin in Dimethylformamid (hergestellt gemäss Beispiel 1) 5.1) vermischt. Bei -20°C werden 4,5 mg einer ca. 50%-igen Dispersion von Natriumhydrid in Mineralöl zuguegeben. Man rührt 45 min bei dieser Temperatur und gibt dann nochmals 0,5 mg Natriumhydrid-Suspension dazu. Man lässt die Temperatur innerhalb von 30 min auf -9°C ansteigen und versetzt dann mit 1 ml gesättigter Natriumchlorid-Lösung und 0,012 ml Essigsäureethylester. Man extrahiert dann dreimal mit je 10 ml Dichlormethan, vereinigt die organischen Extrakte und wäscht sie mit gesättigter wässeriger Natriumchlorid-Lösung, dann mit Wasser und trocknet sie. Die organischen Phasen werden dann eingedampft und mittels Hochdruckflüssigchromatographie (HPLC) durch Kieselgel gereinigt. Man eluiert mit Hexan/Essigester 5:1 bis 5:6. Man erhält 37,4 mg vorgereinigtes Produkt, welches nochmals mittels HPLC gereinigt wird. Man erhält 19,3 mg reines 10-(3-Cyclopropyl-1,2,4-oxadiazol-5-yl)-3-fluor-9H-imidazo[1,5-a][1,2,4]triazolo[1,5-d][1,4]benzodiazepin.

Auf analoge Weise erhält man:

18) 2. 10-(5-Cyclopropyl-1,2,4-oxadiazol-3-yl)-3-fluor-9H-imidazo[1,5-a][1,2,4]triazolo[1,5-d][1,4]-benzodiazepin aus 6-Chlor-10-fluor-5H-triazolo[1,5-d][1,4]benzodiazepin und 5-Cyclopropyl-3-(isocyanomethyl)-1,2,4-oxadiazol (hergestellt gemäss Eur. Pat.-Anm. Nr. 241'682 vom 21. Okt. 1987 oder U.S.-Patentschrift Nr. 4'622'320 vom 11. Nov. 1986).

Beispiel 19

19) 1. 91,4 g 2-Amino-5-methylbenzoesäure-methylester und 23,4 ml Chloracetonitril werden in 1,0 l absolutem Dioxan gelöst und die Lösung auf 10 °C abgekühlt. Anschliessend wird während 8 h ein schwacher Strom von trockenem Chlorwasserstoff bei 5° bis 15 °C eingeleitet. Nach 4 h fügt man nochmals 23,4 ml Chloracetonitril zu. Man rührt 18 h bei Raumtemperatur weiter. Die Suspension wird anschliessend im Vakuum eingedampft. Der kristalline Rückstand wird mit 2,5 l Eis/Wasser suspendiert, mit ca. 100 ml 25%-igem Ammoniak auf pH 8 bis 9 gestellt, 1 h bei 5 °C gerührt und dann filtriert. Die Kristalle werden mit Wasser gewaschen und im Vakuum getrocknet. Man erhält 114,1 g 2-Chlormethyl-6-methyl-3H-chinazolin-4-on vom Smp. 263-265 °C.

19) 2. 114,1 g 2-Chlormethyl-6-methyl-3H-chinazotin-4-on werden in 1,1 l Chloroform und 120 ml N,N,4-Trimethylanilin gelöst, mit 53 ml Phosphoroxychlorid versetzt, und 20 h auf Rückflusstemperatur erhitzt. Das Reaktionsgemisch wird im Vakuum eingedampft. Der Rückstand wird in 1,0 l Essigsäure-ethylester gelöst und nacheinander mit 2 N Salzsäure, Wasser, gesättigter wässeriger Natriunthydrogencarbonat-Lösung und gesättigter wässeriger Natriumchlorid-Lösung gewaschen. Die organischen Extrakte werden im Vakuum auf ca. 0,5 l eingeengt. Man lässt über Nacht bei -25 °C kristallisieren. Der Kristallbrei wird filtriert, die Kristalle mit Essigsäure-ethylester (-25 °C) gewaschen, und im Vakuum getrocknet. Man erhält 89,9 g 4-Chlor-2-(chlormethyl)-6-methylchinazolin vom Smp. 111-112 °C. Aus der Mutterlauge können weitere 11,1 g 4-Chlor-2-(chlormethyl)-6-methylchinazolin vom Smp. 114-116 °C gewonnen werden.

19) 3. 63,5 g 4-Chlor-2-(chlormethyl)-6-methylchinazolin werden in 630 ml absolutem Tetrahydrofuran bei Raumtemperatur gelöst, und sodann auf 10 °C abgekühlt. Es werden 27,2 ml Hydrazinhydrat innerhalb von 8 min zugetropft, wobei eine Lösung entsteht, und die Temperatur auf 15 °C anstelgt. Man rührt 4 h bei 5 bis 15 °C und giesst dann das Reaktionsgemisch in eine Lösung von 40 g Natriumhydrogencarbonat in 5 l Wasser. Man extrahiert fünimal mit je 1,5 l Dichlormethan. Die vereinigten organischen Phasen werden mit Wasser gewaschen, getrocknet, und im Vakuum auf ein Volumen von 0,5 l eingeengt (T < 40 °C). Man fügt 0,5 l Essigsäure-ethylester hinzu und dampft das Dichlormethan im Vakuum vollständig ab, wobei das Produkt auskristallisiert. Man lässt über Nacht bei -25 °C stehen und filtriert die Kristalle ab, wäscht sie mit Diethylether, und trocknet sie im Vakuum bei Raumtemperatur. Man erhält 38 g 2-(Chlormethyl)-4-hydrazino-6-methylchinazolin vom Smp. über 140 °C (Zers.).

19) 4. 58,2 g 2-(Chlormethyl)-4-hydrazino-6-methylchinazolin werden in 870 ml Orthoameisensäuretriethylester in einem auf 100 °C vorgeheiztem Oelbad unter Rühren auf Rückflusstemperatur erwärmt, und weitere 0,5 h bei dieser Temperatur gerührt (Badtemp. 140-145 °C). Während des Aufheizens werden ca. 40 ml Ethanol abdestilliert. Danach wird im Vakuum auf ein Volumen von ca. 150 ml eigeengt. Man fügt dann 150 ml Diethylether hinzu, kühlt auf 0 bis 5 °C ab, und filtriert die Kristalle ab. Das Produkt wird mit Ethanol/Diethylether 1:2 und mit Diethylether gewaschen, und im Vakuum getrocknet. Man erhält 55,1 g rohes 5-(Chlormethyl)-9-methyl-1,2,4-triazolo[4,3-c]chinazolin vom Smp. 175 °C.

19) 5. 62,1 g 5-(Chlormethyl)-9-methyl-1,2,4-triazolo[4,3-c]chinazolin werden in 1,1 l Dioxan suspendiert und auf 8 °C abgekühlt. Man tropft innert 10 min bei 7° bis 10 °C, 320 ml 1 N Natriumhydroxid-Lösung zu. Man rührt 24 h bei Raumtemperatur. Das Reaktionsgemisch wird dann mit 2 N Salzsäure schwach sauer (pH 5-6) gestellt und im Vakuum eingeengt. Der Rückstand wird in Dichlormethan gelöst und mit Natriumchlorid-Lösung gewaschen. Die organische Phase wird getrocknet, mit Norit-Kohle entfärbt, klar filtriert, und im Vakuum bis auf ein Volumen von ca. 400 ml eingeengt. Nach Zugabe von 400 ml Essigsäure-ethylester wird wiederum auf ein Volumen von ca. 200 ml eingeengt. Der Kristallbrei wird über Nacht bei -25 °C stehen gelassen, und die Kristalle sodann abfiltriert. Nach Trocknen im Vakuum erhält man 35,5 g 10-Methyl-5H-[1,2,4]triazolo[1,5-d][1,4]benzodiazepin-6(7H)-on vom Smp. 206-208 °C.

19) 6. 34,5 g 10-Methyl-5H-[1,2,4]triazolo[1,5-d][1,4]benzodiazepin-6(7H)-on werden in 0,8 l Chloroform und 50 ml N,N,4-Trimethylanilin suspendiert. Man gibt 19,2 ml Phosphoroxychlorid dazu, und rührt das Gemisch 20 h bei Rückflusstemperatur. Das abgekühlte Reaktionsgemisch wird in 1 l gesättigte wässerige Natriumhydrogencarbonat-Lösung gegossen und 0,5 h intensiv gerührt. Die wässerige Phase wird abgetrennt und noch 2 mal mit je 0,5 l Chloroform extrahiert. Die vereinigten Chloroform-Extrakte werden getrocknet und im Vakuum eingedampft. Der Rückstand (300 ml) besteht aus einem Gemisch von 6-Chlor-10-methyl-5H-[1,2,4]triazolo[1,5-d][1,4]benzodiazepin und N,N,4-Trimethylanilin.

Eine Lösung von 21,7 g Kalium-tert-butylatin 100 ml Dimethylformamid wird auf -50 °C gekühlt. Es werden 21,0 ml Isocyanessigsäure-ethylester innert 8 min bei einer Temperatur von -50° bis -35 °C zugetropft. Danach kühlt man auf -50 °C und tropft die Lösung von 6-Chlor-10-methyl-5H-[1,2,4]triazolo-[1,5-d][1,4]benzodiazepin und N,N,4-Trimethylanilin dazu, wobei die Temperatur auf -15 °C ansteigt. Das Reaktionsgemisch wird noch 1 h bei Raumtemperatur weiter gerührt und sodann in 1 l gesättigte wässerige Natriumchlorid-Lösung gegossen. Man extrahiert viermal mit Chloroform. Die organischen Extrakte werden im Vakuum auf 300 ml eingeengt. Diese Lösung wird mit Essigsäureethylester verdünnt, und abermals im Vakuum auf 200 eingeengt. Das gewünschte Produkt kristallisiert aus. Man lässt über Nacht bei -25 °C stehen, und filtriert dann die Kristalle ab. Man erhält 36,8 g 3-Methyl-9H-imidazo[1,5-a]-[1,2,4]triazolo[1,5-d][1,4]benzodiazepin-10-carbonsäure-ethylester vom Smp. 204-206 °C.

19) 7. 35,6 g 3-Methyl-9H-imidazo[1,5-a][1,2,4]triazolo[1,5-d][1,4]benzodiazepin-10-carbonsäure-ethylester werden in einem Gemisch von 9,7 g Kaliumhydroxid, 180 ml Wasser und 1,5 l Ethanol 3 h auf 80 °C erhitzt. Das Reaktionsgemisch wird im Vakuum eingedampft. Man löst den Rückstand in 2 l Wasser und stellt ihn mit Salzsäure auf pH 2. Man lässt einige Stunden stehen und filtriert sodann die ausgefallenen Kristalle ab. Nach Trocknen im Vakuum erhält man 31,2 g 3-Methyl-9H-imidazo[1,5-a][1,2,4]triazolo[1,5-d][1,4]benzodiazepin-10-carbonsäure vom Smp. 281-282 °C (Zers.).

19) 8.1. 3,0 g 3-Methyl-9H-imidazo[1,5-a][1,2,4]triazolo[1,5-d][1,4]benzodiazepin-10-carbonsäure werden in 60 ml Dimethylformamid suspendiert. Man gibt 2,1 g 1,1'-Carbonyldiimidazol dazu und rührt 3 h bei Raumtemperatur. Dann werden 1,5 g Cyclopropancarboxamidoxim zugegeben und 2 h bei 90 °C gerührt. Das Reaktionsgemisch wird im Vakuum eingeengt und der Rückstand in 30 ml Essigsäure 3 h bei 100 °C gerührt. Man dampft im Vakuum ein, verteilt den Rückstand zwischen Chloroform und gesättigter wässeriger Natriumhydrogencarbonat-Lösung. Das Chloroform-Extrakt wird getrocknet und durch Kieselgel chromatographiert. Mit Chloroform/Essigsäureethylester 9:1 werden 3,3 g rohes 10-(3-Cyclopropyl-1,2,4-oxadiazol-5-yl)-3-methyl-9H-imidazo[1,5-a][1,2,4]triazolo[1,5-d][1,4]benzodiazepin eluiert. Durch Unikristallisieren aus Essigsäureethylester erhält man 2,8 g reines 10-(3-Cyclopropyl-1,2,4-oxadiazol-5-yl)-3-methyl-9H-imidazo[1,5-a][1,2,4]triazolo[1,5-d][1,4]benzodiazepin vom Smp. 236-237 °C.

Auf analoge Weise erhält man:

19) 8.2. 10-(3-Benzyl-1,2,4-oxadiazol-5-yl)-3-methyl-9H-imidazo[1,5-a][1,2,4]triazolo[1,5-d][1,4]-benzodiazepin, Smp. 188-189 °C, aus 3-Methyl-9H-imidazo[1,5-a][1,2,4]triazolo[1,5-d][1,4]benzodiazepin-10-carbonsäure und 2-Phenylacetamidoxim.

Beispiel 20

20) 1. In eine Suspension von 43 g 5-Brom-2H-1,3-benzoxazin-2,4(1H)dionin 190 ml Formamid wird bei 15 bis 25 °C gasförmiger Ammoniak eingeleitet. Es entsteht dabei eine Lösung. Man rührt 3 h bei Raumtemperatur, und vertreibt dann den überschüssigen Ammoniak mittels Durchleiten von Stickstoff. Die Lösung wird dann 15 h bei 175 °C gerührt. Die Reaktionslösung wird dann abgekühlt und unter Rühren mit 250 ml Wasser versetzt. Die Kristalle werden abfiltriert, mit Wasser gewaschen, und im Vakuum getrocknet. Man erhält 26 g 5-Brom-3H-chinazolin-4-on vom Smp. 239-240 °C.

Aus dem Filtrat lassen sich, durch Abdestillieren des Wassers, nochmaligem Erhitzen der zurückbleibenden Formamid-Lösung, 15 h auf 175 °C, und Aufarbeitung wie oben beschrieben, weitere 7,2 g 5-Brom-3H-chinazolin-4-on gewinnen.

20) 2. 33 g 5-Brom-3H-chinazolin-4-on werden in 1,2 l Chloroform suspendiert. Bei Raumtemperatur werden 106 ml N,N,4-Trimethylanilin und 41 ml Phosphoroxychlorid zugegeben und 16 h bei Rückflusstemperatur gerührt. Die abgekühlte Reaktionslösung wird in 5 l gesättigte wässerige Natriumhydrogencarbonat-Lösung gegossen. Nach 30 min Rühren wird die organische Phase abgetrennt, und die wässerige Phase mit Dichlormethan extrahiert. Die vereinigten organischen Phasen werden über Natriumsulfat getrocknet und sodann durch eine Säule aus 2 kg Kieselgel chromatographiert. Zunächst wird mit Dichlormethan und Dichlormethan/Essigsäureethylester 98:2 N,N,4-Trimethylanilin eluiert. Dann werden mit Dichlormethan/Essigsäureethylester 96:4 29,0 g 5-Brom-4-chlorchinazolin vom Smp. 124-126 °C eluiert.

20) 3. Eine Lösung von 5,63 g 5-Brom-4-chlorchinazolin in 300 ml Tetrahydrofuran wird mit 10,5 g Natriumhydrogencarbonat und 5,76 g 2-Bromethylamin versetzt, und 66 h bei Raumtemperatur gerührt. Das Reaktionsgemisch wird im Vakuum eingedampft, und der Rückstand in 300 ml Wasser gerührt. Die Kristalle werden abfiltriert, mit Wasser gewaschen und im Vakuum getrocknet. Man erhält 5,7 g 10-Brom-2,3-dihydroimidazo[1,2-c]chinazolin vom Smp. 177-179 °C .

20) 4. In einem Reaktionsgefäss, das mit einem Wasserabscheider versehen ist, werden zu einer warmen Lösung von 22 g 10-Brom-2,3-dihydroimidazo[1,2-c]chinazolin in 2 l Benzol 242 g Mangandioxid

zugegeben. Das Gemisch wird 15 h auf Rückflusstemperatur erhitzt. Dann gibt man weitere 8 g Mangandioxid dazu, und erhitzt nochmals 1 h auf Rückflusstemperstur. Das heisse Reaktionsgemisch wird durch Dicalite filtriert. Der Filterkuchen wird mit 1 l heissem Benzol nachgewaschen.dann nochmals in 1,5 l Dichlormetha/Ethanol 199:1 ausgekocht, und abermals filtriert. Die filtrierten Lösungen werden im Vakuum auf ca. 1,5 l eingeengt. Es kristallisiert ein Nebenprodukt, das abfiltriert wird (1,65 g). Das Filtrat wird duch eine Säule aus 2 kg Kieselgel chromatographiert. Mit Dichlormethan/Ethanol 99:1 werden insgesamt 12,0 g des gewünschten Produktes eluiert. Nach Kristallisieren aus Essigsäure-ethylester erhält man 11,7 g 10-Bromimidazo[1,2-c]chinazolin vom Smp. 215-216 °C.

20) 5. 11,6 g 5,10-Bromimidazo[1,2-c]chinazolin werden in 170 ml 6 N Salzsäure 6,5 h bei 90 °C gerührt, und dann im Eisbad abgekühlt. Die Reaktionslösung wird in 30 ml 25%-igen wässerigem Ammoniak und 50 g Eis gegossen und 10 min gerührt. Die Kristalle werden abfiltriert, mit Wasser gewaschen, und im Vakuum getrocknet. Man erhält 9,85 g 3-Brom-2-(1H-2-imidazolyl)anilin. Die wässerige Phase wird mit Natruimchlorid gesättigt und dreimal mit Chloroform extrahiert. Nach Eindampfen der organischen Extrakte im Vakuum erhält man weitere 1,52 g 3-Brom-2-(1H-2-imidazolyl)anilin. Die Substanz kann aus Wasser umkristallisiert werden. Smp. 164-165 °C.

20) 6. Zu einer Lösung von 6,0 g 3-Brom-2-(1H-2-imidazolyl)anilin in 150 ml Dioxan und 6,1 ml Pyridin wird innert 15 min bei 5 °C eine Lösung von 2,4 ml Chloracetylchlorid in 10 ml Diethylether zugetropft. Man rührt 10 min bei 5 °C, und tropft dann nochmals eine Lösung von 0,25 ml Chloracetylchlorid in 5 ml Diethylether dazu. Man rührt weitere 30 min bei 10 ° bis 12 °C und versetzt dann innerhalb von 5 min mit einem Gemisch von 75 ml wässeriger 1 N Natriumhydroxyd-Lösung und 150 ml Dioxan. Man rührt 45 min bei Raumtemperatur und giesst dann in 1 l Wasser. Man extrahiert viermal mit Chloroform. Die Chloroform-Extrakte werden im Vakuum eingedampft. Der ölige Rückstand wird aus Dichlormethan/Diethylether kristallisiert. Man erhält 0,99 g 11-Brom-5H-imidazo[1,2-d][1,4]benzodiazepin-6(7H)-on vom Smp. 234-235 °C. Die wässerige Phase wird im Vakuum eingedampft. Der Rückstand wird mehrmals in Ethanol und Toluol aufgenommen und wieder im Vakuum eingedampft. Der Rückstand wird in 150 ml Trifluoressigsäure gelöst und 16 h bei Raumtemperatur stehen gelassen. Das Reaktionsgemisch wird im Vakuum eingedampft, und der Rückstand zwischen gesättigter wässeriger Natriumhydrogencarbonat-Lösung und Chloroform verteilt. Die organischen Extrakte werden im Vakuum eingedampft, und der Rückstand aus Dichlormethan/Diethylether kristallisiert. Man erhält weitere 3,37 g 11-Brom-5H-imidazo[1,2-d][1,4]benzodiazepin-6(7H)-on.

20) 7. 2,86 g Isocyanessigsäureethylester werden in 120 ml Tetrahydrofuran gelöst. Man kühlt auf -15 °C und gibt 2,8 g Kalium-tert-butylat dazu. Man rührt noch 1 h bei -10 °C.

Andererseits löst man 5,56 g 11-Brom-5H-[1,2,4]imidazo[[1,2-d][1,4]benzodiazepin-6(7H)-on in 100 ml Dimethylformamid. Bei -15 °C gibt man 0,85 g einer ca. 80%-igen Dispersion von Natriumhydrid in Mineralöl dazu und rührt 1 h bei -10 °C. Alsdann kühlt man die Lösung auf -40 °C, tropft 5,66 ml Diphenylphosphorylchlorid innerhalb von 10 min dazu, rührt 30 min bei -40 °C und kühlt sodann auf -60 °C ab. Bei dieserTemperatur gibt man die vorher zubereitete Suspension des Isocyanessigsäureethylester-kaliumsalzes dazu und entfernt sodann die Kühlung. Man rührt 2 h bei Raumtemperatur und stellt dann mit Essigsäure auf pH 6 bis 7. Das Gemisch wird in 2 l gesättigte wässerige Natriumhydrogencarbonat-Lösung gegossen und viermal mit Chloroform extrahiert. Die organischen Phasen werden noch je einmal mit gesättigter wässeriger Natriumhydrogencarbonat- und Natriumchlorid-Lösung gewaschen und dann im Vakuum eingedampft. Der Rückstand wird aus Essigsäureethylester und Diethylether kristallisiert. Man erhält 4,8 g 4-Brom-9H-diimidazo[1,5-a:1',2'-d][1,4]-benzodiazepin-10-carbonsäure-ethylester vom Smp. 246-247 °C.

20) 8. 3,72 g 4-Brom-9H-diimidazo[1,5-a:1',2'-d][1,4]benzodiazepin-10-carbonsäure-ethylester werden in 250 ml Ethanol suspendiert. Man gibt eine Lösung von 1,0 g Kaliumhydroxid in 25 ml Wasser dazu und erhitzt 1 h auf 80 °C. Die Lösung wird im Vakuum eingedampft; der Rückstand in Wasser gelöst und mit 20 ml 1 N Salzsäure versetzt. Man rührt 1 h im Eisbad und filtriert dann die Kristalle ab. Nach dem Trocknen erhält man 3,35 g 4-Brom-9H-diimidazo[1,5-a:1',2'-d][1,4]benzodiazepin-10-carbonsäure vom Smp. 304-306 °C (Zers.).

20) 9. Zu einer Lösung von 1,38 g 4-Brom-9H-diimidazo[1,5-a:1',2'-d][1,4]benzodiazepin-10-carbonsäure in 30 ml Dimethylformamid werden 1,08 g 1,1'-Carbonyldiimidazol zugegeben. Man rührt 4 h bei Raumtemperatur. Dan gibt man 0,68 g Cyclopropancarboxamidoxim dazu und rührt 20 h bei 80 °C. Das Reaktionsgemisch wird im Hochvakuum eingedampft. Der Rückstand wird in 20 ml Essigsäure gelöst und 2,5 h auf 110 °C erhitzt. Man dampft im Vakuum ein und verteilt den Rückstand zwischen Dichlormethan und gesättigter wässeriger Natriumhydrogencarbonat-Lösung. Die wässerige Phase wird dreimal mit Dichlormethan extrahiert, die organischen Extrakte je einmal mit gesättigter Natriumhydrogencarbonat- und mit Natriumchlorid-Lösung gewaschen und im Vakuum eingeengt. Der

Rückstand wird aus Essigsäureethylester kristallisiert. Man erhält 1,26 g 10-(3-Cyclopropyl-1,2,4-oxadiazol-5-yl)-4-brom-9H-diimidazo[1,5-a:1',2'-d][1,4]benzodiazepin vom Smp. 243-244 °C.

Beispiel 21

21) 1. 66,2 g 2-Amino-6-methylbenzonitril und 22 g Acetonitril werden in 750 ml absolutem Dioxan gelöst und die Lösung auf 5 °C abgekühlt. Anschliessend wird während 8 h ein schwacher Strom von trockenem Chlorwasserstoff bei 5 bis 7 °C eingeleitet. Man rührt 15 h bei Raumtemperatur weiter und kühlt sodann wieder auf 5 °C ab. Man gibt nochmals 11 g Acetonitril hinzu, leitet abermals während 8 h Salzsäure-Gas ein, und rührt weitere 15 h bei Raumtemperatur. Die Suspension wird anschliessend im Vakuum bei 30 °C eingedampft. Der kristalline Rückstand wird mit 0,7 l Wasser verrührt, auf 0 ° bis 5 °C abgekühlt, mit gesättigter Natriumhydrogencarbonat-Lösung neutralisiert, und filtriert. Die Kristalle werden mit Wasser gewaschen und im Vakuum getrocknet. Man erhält 107 g rohes 4-Amino-2,5-dimethylchinazolin, das noch anorganische Salze enthält. Nach Umkristallisation aus Ethanol schmilzt diese Verbindung bei 198-199 °C.

21) 2. 42 g rohes 4-Amino-2,5-dimethylchinazolin werden in 1,0 l 6 N Salzsäure 20 h auf 95 °C erhitzt. Das Reaktionsgemisch wird im Vakuum eingeengt, und sodann mit gesättigter wässeriger Natriumhydrogencarbonat-Lösung neutralisiert. Die Kristalle werden filtriert, mit Wasser gewaschen, und im Vakuum getrocknet. Man erhält 34 g 2,5-Dimethyl-3H-chinazolin-4-on vom Smp. 255-257 °C.

21) 3. 17,4 g 2,5-Dimethyl3H-chinazolin-4-on werden in 300 ml Chloroform und 25 ml N,N,4-Trimethylanilin gelöst, mit 8 ml Phosphoroxychlorid versetzt, und 20 h auf Rückflusstemperatur erhitzt. Das Reaktionsgemisch wird dann mit 1,5 l gesättigter wässeriger Natriumhydrogencarbonat-Lösung während 20 min gerührt. Die organische Phase wird abgetrennt, und die wässerige Phase noch dreimal mit Chloroform extrahiert. Die organischen Extrakte werden mit 1 N Salzsäure und mit Wasser gewaschen und sodann im Vakuum eingedampft. Der kristalline Rückstand (14,15 g) besteht aus rohem 4-Chlor-2,5-dimethylchinazolin vom Smp. 76-77 °C.

21) 4. 14,15 g rohes 4-Chlor-2,5-dimethylchinazolin werden in 300 ml Tetrahydrofuran suspendiert, auf 5 °C abgekühlt und unter Rühren mit 16,5 ml 2,2-Dimethoxyethylamin versetzt. Man rührt 24 h bei Raumtemperatur, gibt nochmals 10 ml 2,2-Dimethoxyethylamin hinzu, und rührt weitere 40 h. Das Reaktionsgemisch wird im Vakuum eingedampft, und der Rückstand zwischen Chloroformund gesättigter wässeriger Natriumhydrogencarbonat-Lösung verteilt. Die Chloroform-Lösung wird im Vakuum eingedampft. Der Rückstand (21 g) wird durch eine aus 150 g Kieselgel chromatographiert. Mit Essigsäureethylester werden 18,5 g 4-(2,2-Dimethoxyethylamino)-2,5-dimethylchinazolin eluiert. Diese werden aus Hexan kristallisiert. Man erhält 18,3 g 4-(2,2-Dimethoxyethylamino)-2,5-dimethylchinazolin vom Smp. 48-49 °C.

21) 5. 15,8 g 4-(2,2-Dimethoxyethylamino)-2,5-dimethyichinazolin werden in 300 g Polyphosphorsäure 24 h auf 155 °C erhitzt und dann in eiskalte wässerige Ammoniak-Lösung gegossen. Man extrahiert viermal mit Chloroform. Die Chloroform-Extrakte werden im Vakuum eiggengt. Als Rückstand erhält man 12,1 g kristallisiertes 5,10-Dimethylimidazo[1,2-c]chinazolin. Nach Umkristallisieren aus Essigsäureethylester liegt der Smp. bei 159 °C.

21) 6. 13 g 5,10-Dimethylimidazo[1,2-c]chinazolin werden in 350 ml 6 N Salzsäure 5 h bei 95 °C gerührt, und dann im Eisbad abgekühlt. Die Reaktionslösung wird in 30 ml 25%-igen Ammoniak gegossen und 10 min im Eisbad verrührt. Die Kristalle werden abfiltriert, mit Wasser gewaschen, und im Vakuum getrocknet. Man erhält 10,1 g 3-Methyl-2-(1H-2-imidazolyl)anilin. Das Filtrat wird dreimal mit Chloroform extrahiert. Die Extrakte werden im Vakuum eingedampft. Man erhält weitere 1,4 g 3-Methyl-2-(1H-2-imidazolyl)anilin. Nach Umkristallisieren aus Essigsäureethylester liegt der Smp. bei 174-175 °C.

21) 7. Zu einer Lösung von 11,5 g 3-Methyl-2-(1H-2-imidazolyl)anilin in 0,35 l Tetrahydrofuran und 21 ml Pyridin werden bei 0 °C innert 15 min eine Lösung von 8,25 ml Chloracetylchlorid in 25 ml Diethylether zugetropft. Man rührt 1 h bei 0 °C, und giesst dann das Reaktionsgemisch in gesättigte wässerige Natriumhydrogencarbonat-Lösung. Man extrahiert dreimal mit Chloroform. Die organischen Extrakte werden im Vakuum eingedampft. Der Rückstand (13 g) wird in 300 ml Dioxan gelöst und mit 200 ml 1 N wässeriger Natriumhydroxyd-Lösung versetzt. Man rührt 16 h bei Raumtemperatur, stellt dann mit Salzsäure auf pH 6, und dampft im Vakuum ein. Der Rückstand wird in absolutem Ethanol suspendiert, und die unlöslichen anorganischen Salze abfiltriert. Das Filtrat wird im Vakuum eingedampft, der Rückstand in Trifluoressigsäure gelöst, und 16 h bei Raumtemperatur gerührt. Das Reaktionsgemisch wird im Vakuum eingedampft, und der Rückstand zwischen Chloroform und gesättigter wässeriger Natriumhydrogencarbonat-Lösung verteilt. Die organischen Phasen werden im Vakuum eingedampft. Der Rückstand wird durch eine Säule aus 500 g Kieselgel chromatographiert. Mit Essigsäureethylester

werden nacheinander 1,4 g 10-Methylimidazo[1,2-c]chinazolin-5(6H)-on (Smp. 308-310 ° C) und 9,0 g 11-Methyl-5H-imidazo[1,2-d][1,4]benzodiazepin-6(7H)-on vom Smp. 188-189 ° C eluiert.

21) 8. Eine Lösung von 5,57 g Isocyanessigsäure-ethylester in 250 ml Tetrahydrofuran wird auf -15 ° C gekühlt. Es werden 5,48 g Kalium-tertbutylat zugegeben, und die Lösung noch 1 h bei - 10 ° bis - 15 ° C weiter gerührt.

Andererseits wird eine Lösung von 9,0 g 11-Methyl-5H-imidazo[1,2-d][1,4]benzodiazepin-6(7H)-on in 195 ml Dimethylformamid bei -15 ° C mit 1,5 g einer ca. 80%-igen Dispersion von Natriumhydrid in Mineralöl versetzt. Das Gemisch wird 2 h bei -12 ° bis -8 ° C gerührt. Dann wird auf -45 ° C gekühlt, 11,1 ml Phosphorsäure-diphenylester-chlorid innert 15 min zugetropft und 30 min bei -40 ° C weiter gerührt. Dann wird auf-65 ° C gekühlt und die Lösung des Isocyanessigsäure-ethylester-kaliumsalzes zugegeben. Die Temperatur steigt dabei auf -30 ° C. Man rührt das Reaktionsgemisch anschliessend 1,5 h bei Raumtemperatur. Dann kühlt man auf 10 ° C und stellt mit Essigsäure auf pH 6 bis 7. Das Reaktionsgemisch wird in 2 l gesättigte wässerige Natriumhydrogencarbonat-Lösung gegossen. Man extrahiert viermal mit Chloroform. Die organischen Extrakte werden mit gesättigter wässeriger Natriumhydrogencarbonat-Lösung gewaschen, getrocknet und im Vakuum eingedampft. Der Rückstand wird in 150 ml Essigsäureethylester aufgenommen, wobei das Produkt kristallisiert, Nach einigen Stunden Stehenlassen werden die Kristalle abfiltriert. Man erhält 8,8 g 4-Methyl-9H-diimidazo[1,5-a:1',2'-d][1,4]benzodiazepin-10-carbonsäure-ethylester vom Smp. 220 ° C. Das Filtrat wird eingedampft. Der Rückstand wird in Dichlormethan gelöst und durch eine Säule aus 60 g Kieselgel chromatographiert. Mit Dichlormethan eluiert man zuerst ein Nebenprodukt, dann weitere 1,1 g 4-Methyl-9H-diimidazo[1,5-a:1',2'-d][1,4]benzodiazepin-10-carbonsäure-ethylester.

21) 9. 10,6 g 4-Methyl-9H-diimidazo[1,5-a:1',2'-d][1,4]benzodiazepin-10-carbonsäure-ethylester werden in einem Gemisch von 3,2 g Kaliumhydroxid, 42,5 ml Wasser und 115 ml Ethanol 1 h auf 80 ° C erhitzt. Das Reaktionsgemisch wird im Vakuum eingedampft. Man löst den Rückstand in 15 ml Wasser und stellt ihn mit Salzsäure auf pH < 5. Man lässt einige Stunden stehen und filtriert sodann die Kristalle ab. Nach Trocknen im Vakuum erhält man 9,3 g 4-Methyl-9H-diimidazo[1,5-a:1',2'-d][1,4]benzodiazepin-10-carbonsäure vom Smp. 305 ° C (Zers.).

21) 10. 0,5 g 4-Methyl-9H-dimidazo[1,5-a:1',2'-d][1,4]benzodiazepin-10-carbonsäure werden in 15 ml Dimethylformamid suspendiert. Man gibt 0,45 g 1,1'-Carbonyldiimidazol dazu und rührt 4 h bei Raumtemperatur. Dann werden 274 mg Cyclopropancarboxamidoxim zugegeben und 20 h bei 80 ° C gerührt. Das Reaktionsgemisch wird im Vakuum eingeengt und der Rückstand in 40 ml Essigsäure 1,5 h bei 110 ° C gerührt. Man dampft im Vakuum ein, verteilt den Rückstand zwischen Chloroform und gesättigter wässeriger Natriumhydrogencarbonat-Lösung. Das Chloroform-Extrakt wird getrocknet und im Vakuum eingedampft. Der Rückstand (0,62 g) wird in Chloroform gelöst und durch 35 g Kieselgel chromatographiert. Mit Chloroform wird rohes 10-(3-Cyclopropyl-1,2,4-oxadiazol-5-yl)-4-methyl-9H-diimidazo[1,5-a:1',2'-d][1,4]benzodiazepin eluiert. Durch Umkristallisieren aus Ethanol erhält man 0,43 g reines 10-(3-Cyclopropyl-1,2,4-oxadiazol-5-yl)-4-methyl-9H-diimidazo[1,5-a:1',2'-d][1,4]benzodiazepin vom Smp. 200 ° C.

Beispiel 22

4,0 g 10-[5-[4-(4-Brombenzyloxy)benzyl]-1,2,4-oxadiazol-3-yl]-3-fluor-9H-imidazo[1,5-a][1,2,4]triazolo-[1,5-d][1,4]benzodiazepin (hergestellt gemäss Beispiel 1) 10.61.) werden in 40 ml Essigsäure und 10,4 ml 30%-igem Bromwasserstoffin Essigsäure gelöst und 21 h bei 80 ° C gerührt. Danach giesst man auf Eiswasser und stellt mit 25%-igem Ammoniak auf pH 8 bis 9. Man rührt 1 h bei 0 ° bis 5 ° C. Danach filtriert man die Kristalle ab, wäscht sie und trocknet sie im Vakuum. Das Rohprodukt wird aus Methanol kristallisiert. Man erhält 2,25 g 4-[3-[3-Fluor-9H-imidazo[1,5-a][1,2,4]triazolo[1,5-d][1,4]benzodiazepin-10-yl]-1,2,4-oxadiazol-5-ylmethyl]phenol vom Smp. 245-246 ° C.

Beispiel 23

2,0 g [2-[[[3-[3-Fluor-9H-imidazo[1,5-a][1,2,4]triazolo[1,5-d][1,4]benzodiazepin-10-yl]-1,2,4-oxadiazol-5-yl]methyl]methylamino]ethylcarbamin säure-tert-butylester (hergestellt gemäss Beispiel 10) 1.13.) werden in 40 ml Dichlormethan und 1,54 ml Trifluoressigsäure gelöst und 18 h bei Raumtemperatur und 1 h bei Rückflusstemperatur gerührt. Das Reaktionsgemisch wird im Vakuum eingedampft. Der Rückstand wird in Methanol gelöst, mit 0,7 ml einer 21%-igen Lösung von Chlorwasserstoffin Ethanol versetzt, und im Vakuum aufein Kleines Volumen eingeengt. Die Kristalle werden abfiltriert, gewaschen und getrocknet. Man erhält 1,55 g 10-[5-[[(2-Aminoethyl)methylamino]methyl]-1,2,4-oxadiazol-3-yl]-3-fluor-9H-imidazo[1,5-a]-

[1,2,4]triazolo[1,5-d][1,4]benzodiazepin-monohydrochlorid vom Smp. 218-219° C.

Beispiel 24

1,7 g N-[[3-[3-Fluor-9H-imidazo[1,5-a][1,2,4]triazolo[1,5-d][1,4]benzodiazepin-10-yl]-1,2,4-oxadiazol-5-yl]-methyl-N-methylcarbaminsäure-benzylester (hergestellt gemäss Beispiel 1) 10.62.) werden in 5 ml Essigsäure und 5 ml 30%-igem Bromwasserstoff in Essigsäure während 1 h bei Raumtemperatur gerührt. Die feste Masse wird in Wasser gelöst, und mit Diethylether extrahiert. Die wässerige Phase wird mit Natriumhydrogencarbonat auf pH 8 gestellt und dann mit Dichlormethan extrahiert. Die organische Phase wird getrocknet, flitriert, mit 50 ml Essigsäureethylester verdünnt und im Vakuum aufein kleines Volumen eingedampft, wobei das Produkt kristallisiert. Man lässt 16 h bei 2° C stehen und filtriert die Kristalle ab. Man erhält 1,0 g 3-Fluor-10-[5-(methylamino)methyl]-1,2,4-oxadiazol-3-yl]-3-fluor-9H-imidazo[1,5-a][1,2,4]-triazolo[1,5-d][1,4]benzodiazepin vom Smp. 230-231° C.

Beispiel A

Es werden in üblicher Weise Tabletten folgender Zusammensetzung hergestellt:

| | | mg/Tablette |
|---|---|---|
| Wirkstoff | | 5 |
| Milchzucker | | 45 |
| Maisstärke | | 15 |
| Mikrokristalline Cellulose | | 34 |
| Magnesiumstearat | | 1 |
| | Tablettengewicht | 100 |

Beispiel B

Es werden Kapseln folgender Zusammensetzung hergestellt:

| | | mg/Kapsel |
|---|---|---|
| Wirkstoff | | 10 |
| Milchzucker | | 155 |
| Maisstärke | | 30 |
| Talk | | 5 |
| | Kapselfüllgewicht | 200 |

Der Wirkstoff, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

Beispiel C

Es werden Suppositorien folgender Zusammensetzung hergestellt:

| | | mg/Supp. |
|---|---|---|
| Wirkstoff | | 15 |
| Suppositorienmasse | | 1285 |
| | Total | 1300 |

Die Suppositorienmasse wird in einem Glas- oder Stahlgefäss geschmolzen, gründlich vermengt und auf 45° abgekühlt. Hierauf gibt man den fein pulverisierten Wirkstoffzu und rührt, bis er vollständig dispergiert ist. Man giesst die Mischung in Suppositorienformen geeigneter Grösse, lässt abkühlen, nimmt

dann die Suppositorien aus den Formen und verpackt sie einzeln in Wachspapier oder Metallfolien.

**Patentansprüche**

**1.** Verbindungen der allgemeinen Formel

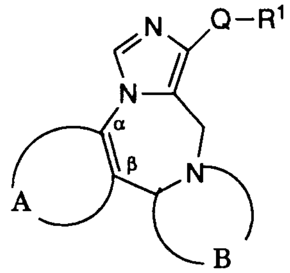

I

worin A zusammen mit den beiden mit $\alpha$: und $\beta$ bezeichneten Kohlenstoffatomen eine der Gruppen

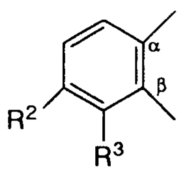

,

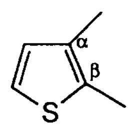

,

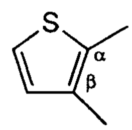

,

(a) (b) (c)

B einen der Reste

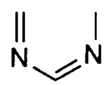

,

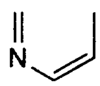

,

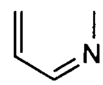

,

(d) (e) (f)

Q eine der Gruppen

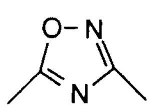

,

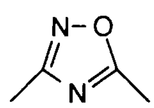

,

(g) (h)

$R^1$ eine gegebenenfalls durch Hydroxy, niederes Alkoxy, Aryl, $C_{3-6}$-Cycloalkyl, Aroyl, Aryloxy, Heteroaroyloxy, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, eine Aroylgruppe, einen über ein Kohlenstoffatom gebundenen fünfgliedrigen Heterocyclus oder gegebenenfalls durch Acyl oder niederes Alkyl

substituiertes $C_{3-6}$-Cycloalkyl,

$R^2$ und $R^3$ je Wasserstoff, Halogen oder niederes Alkyl,

$R^4$ Wasserstoff oder niederes Alkyl,

$R^5$ Wasserstoff, Aryl, Acyl, $C_{3-6}$-Cycloalkyl, Aralkoxycarbonyl oder gegebenenfalls durch Aryl, Morpholino, niederes Alkoxy, Hydroxy, 2,2-Dimethyl-1,3-dioxolan-4-yl, Alkoxycarbonyl, Carbamoyl, Alkoxycarbonylamino, Aralkoxycarbonyl oder Amino substituiertes niederes Alkyl oder $R^4$ und $R^5$ zusammen mit dem Stickstoffatom entweder Phthalimino oder einen sechsgliedrigen gesättigten Heterocyclus bedeuten,

und pharmazeutisch annehmbare Säureadditionssalze von basischen Verbindungen der Formel I.

**2.** Verbindungen der in Anspruch 1 definierten Formel I, worin A, B und Q die dort angegebene Bedeutung haben und worin $R^1$ eine gegebenenfalls durch Hydroxy, niederes Alkoxy, Aryl, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, einen über ein Kohlenstoffatom gebundenen fünfgliedrigen Heterocyclus oder $C_{3-6}$-Cycloalkyl, $R^2$ und $R^3$ je Wasserstoff oder Halogen, $R^4$ Wasserstoff oder niederes Alkyl, $R^5$ Wasserstoff, niederes Alkyl, Aryl oder Acyl oder $R^4$ und $R^5$ zusammen mit dem Stickstoffatom entweder Phthalimino oder einen sechsgliedrigen gesättigten Heterocyclus bedeuten.

**3.** Verbindungen nach Anspruch 1 oder 2, dadurch gekennzeichnet, dass

a) der fünfgliedrige, über ein Kohlenstoffatom gebundene Heterocyclus aromatisch oder gesättigtist, ein Stickstoff-, Sauerstoff- oder Schwefelatom und gegebenenfalls zusätzlich ein Stickstoffatom als Ringglieder enthält, und unsubstituiert oder durch niederes Alkyl substituiert ist oder in Nachbarschaft zu einem nichtaromatischen Stickstoffatom eine Oxogruppe enthält;

b) der fünfgliedrige, über ein Stickstoffatem gebundene Heterocyclus aromatisch ist und gegebenenfalls ein zweites Stickstoffatom als zusätzliches Ringglied enthält;

c) der sechsgliedrige gesättigte Heterocyclus als Ringglied zusätzlich ein Sauerstoffatom oderdie Gruppe $>N-R^6$ enthalten kann, wobei $R^6$ niederes Alkyl, Aryl, niederes Alkenyl oder niederes Alkinyl bedeutet.

**4.** Verbindungen nach Anspruch 3, dadurch gekennzeichnet, dass

a) der fünfgliedrige, über ein Kohlenstoffatom gebundene Heterocyclus eine 2-Thienyl-, 3-Thienyl-, 2-Furyl-, 3-Furyl-, 5-Oxazolyl- oder 2-Tetrahydrofurylgruppe

b) der fünfgliedrige, über ein Stickstoffatom gebundene Heterocyclus eine 1-Imidazolylgruppe darstellt

c) der sechsgliedrige, gesättigte Heterocyclus eine 4-Morpholino- oder eine 1-Piperazinylgruppe ist, welche in 4-Stellung durch niederes Alkyl, Aryl, niederes Alkenyl oder niederes Alkinyl substituiert ist.

**5.** Verbindungen nach Anspruch 1 oder 2, worin $R^1$ Cyclopropyl bedeutet.

**6.** Verbindungen nach einem der Ansprüche 1-5, worin A die Gruppe a) bedeutet.

**7.** Verbindungen nach einem der Ansprüche 1-6, worin $R^3$ Wasserstoff und $R^2$ Wasserstoff, Fluor oder Chlor bedeuten.

**8.** Verbindungen nach einem der Ansprüche 1-7, worin B den Rest d) oder e) bedeutet.

**9.** 10-(3-Cyclopropyl-1,2,4-oxadiazol-5-yl)-3-fluor-9H-imidazo[1,5-a][1,2,4]triazolo[1,5-d][1,4]benzodiazepin.

**10.** 10-(5-Cyclopropyl-1,2,4-oxadiazol-3-yl)-3-fluor-9H-imidazo[1,5-a][1,2,4]triazolo[1,5-d][1,4]benzodiazepin.

**11.** 10-(5-Cyclopropyl-1,2,4-oxadiazol-3-yl)-9H-imidazo[1,5-a][1,2,4]triazolo[1,5-d][1,4]benzodiazepin.

**12.** 10-(3-Cyclopropyl-1,2,4-oxadiazol-5-yl)-9H-imidazo[1,5-a][1,2,4]triazolo[1,5-d][1,4]benzodiazepin.

**13.** 10-(3-Cyclopropyl-1,2,4-oxadiazol-5-yl)-3-fluor-9H-diimidazo[1,5-a:1',2'-d][1,4]benzodiazepin.

**14.** 10-(5-Cyclopropyl-1,2,4-oxadiazol-3-yl)-3-fluor-9H-diimidazo[1,5-a:1',2'-d][1,4]benzodiazepin.

**15.** 10-(5-Cyclopropyl-1,2,4-oxadiazol-3-yl)-9H-diimidazo[1,5-a:1',2'-d][1.4]benzodiazepin.

**16.** 3-Chlor-10-(5-cyclopropyl-1,2,4-oxadiazol-3-yl)-9H-diimidazo[1,5-a:1',2'-d][1,4]benzodiazepin.

**17.** 4-Chlor-10-(3-cyclopropyl-1,2,4-oxadiazol-5-yl)-9H-diimidazo[1,5-a:1',2'-d][1,4]benzodiazepin.

**18.** 4-Chlor-10-(5-cyclopropyl-1,2,4-oxadiazol-3-yl)-9H-diimidazo[1,5-a:1',2'-d][1,4]benzodiazepin.

**19.** Verbindungen der allgemeinen Formel

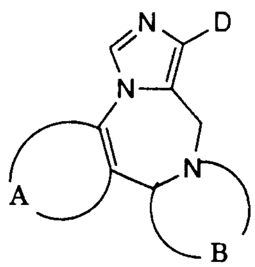

XXIV

worin A und B die in Anspruch 1 angegebene Bedeutung besitzen und D eine der Gruppen -COOH, $-C(NH_2)=NOH$, -C(Cl)=NOH, $-CONH_2$, -CN, $-CH_2-OH$, -CHO, -CH=NOH, oder $-COOR^9$ bedeuten, wobei $R^9$ niederes Alkyl bedeutet.

**20.** Verbindungen der allgemeinen Formel

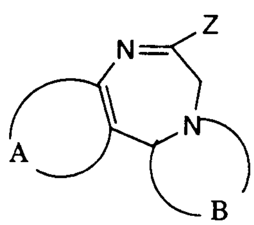

X

worin A und B die in Anspruch 1 angegebene Bedeutung besitzen und Z eine Abgangsgruppe bedeutet.

**21.** Verbindungen der allgemeinen Formel

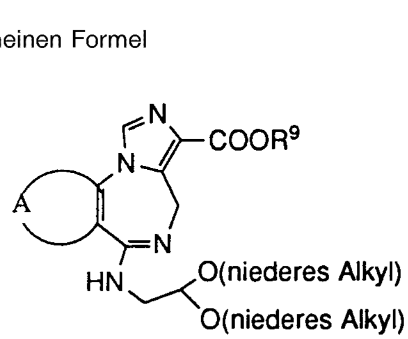

XVIII

worin A die in Anspruch 1 angegebene Bedeutung besitzt und $R^9$ niederes Alkyl bedeutet.

**22.** Verbindungen nach einem der Ansprüche 1-18 zur Anwendung als therapeutische Wirkstoffe.

**23.** Verbindungen nach einem der Ansprüche 1-18 zur Anwendung bei der Bekämpfung oder Verhütung

von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

**24.** Verfahren zur Herstellung von Verbindungen nach einem der Ansprüche 1-18 und von pharmazeutisch annehmbaren Säureadditionssalzen von basischen Verbindungen der Formel I, dadurch gekennzeichnet, dass man

a) ein reaktionsfähiges funktionelles Derivat einer Carbonsäure der allgemeinen Formel

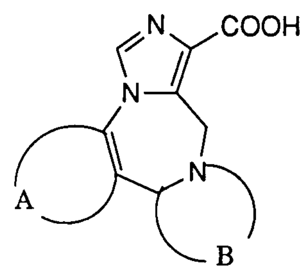

II

worin A und B die in Anspruch 1 angegebene Bedeutung besitzen, mit einem Oxim der allgemeinen Formel

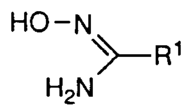

III

worin $R^1$ die in Anspruch 1 angegebene Bedeutung besitzt, umsetzt, oder

b) eine Verbindung der allgemeinen Formel

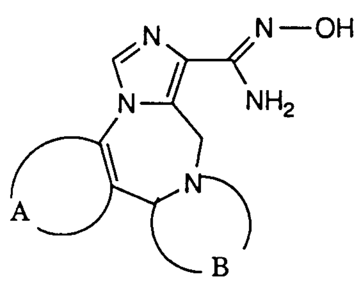

IV

worin A und B die in Anspruch 1 angegebene Bedeutung besitzen, mit einem reaktionsfähigen funktionellen Derivat einer Carbonsäure der allgemeinen Formel

$R^1$-COOH V

worin $R^1$ die in Anspruch 1 angegebene Bedeutung besitzt, umsetzt, oder

c) eine Verbindung der allgemeinen Formel

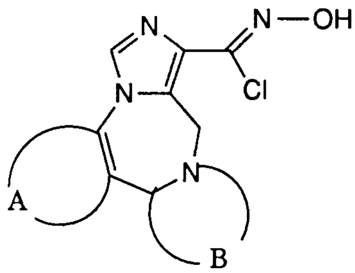

VI

worin A und B die in Anspruch 1 angegebene Bedeutung haben, mit einem Nitril der Formel

$R^{11}$-C≡N     VII

worin $R^{11}$ Arylamino-alkyl bedeutet,
umsetzt, oder
d) eine Verbindung der allgemeinen Formel

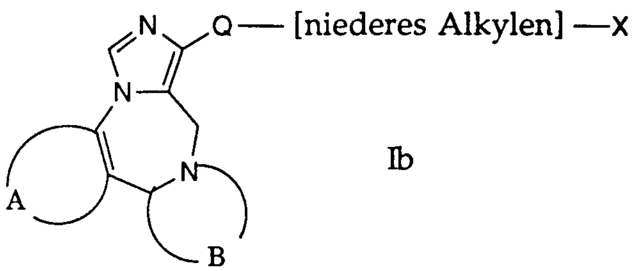

Ib

worin A, B und Q die in Anspruch 1 angegebene Bedeutung haben und X eine Abgangsgruppe bedeutet, mit einem Amin der Formel

$HNR^4R^5$     VIII

worin $R^4$ und $R^5$ die in Anspruch 1 angegebene Bedeutung haben, umsetzt, oder
e) eine Verbindung der allgemeinen Formel

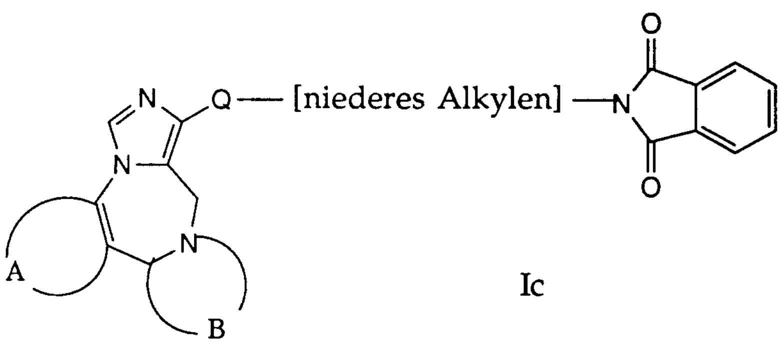

Ic

worin A, B und Q die in Anspruch 1 angegebene Bedeutung besitzen,
in das entsprechende Amin überführt, oder
f) eine Verbindung der allgemeinen Formel

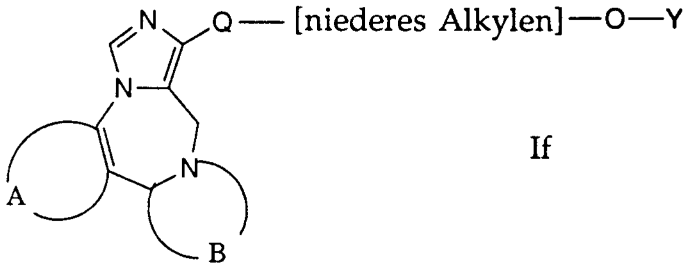

If

worin A, B und Q die in Anspruch 1 angegebene Bedeutung besitzen und Y eine leicht abspaltbare Arylalkylgruppe darstellt,
eine Etherspaltung unterzieht, oder
g) eine Verbindung der allgemeinen Formel

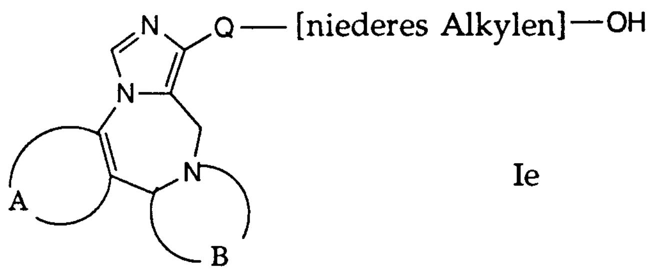

Ie

verestert, oder
h) eine Verbindung der allgemeinen Formel

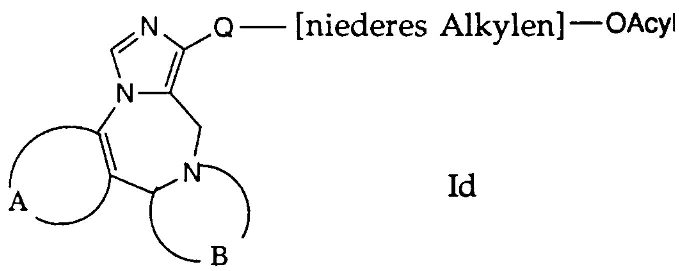

Id

worin A, B und Q die in Anspruch 1 angegebene Bedeutung besitzen,
verseift, oder
i) eine Verbindung der allgemeinen Formel

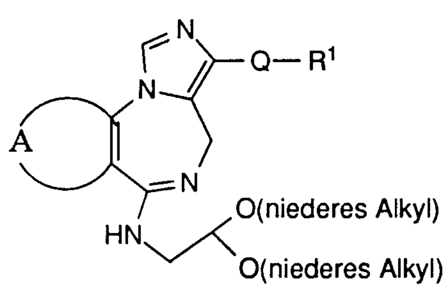

IX

worin A und Q die in Anspruch 1 angegebnene Bedeutung besitzen,
mit einer Säure behandelt, oder
j) eine Verbindung der allgemeinen Formel

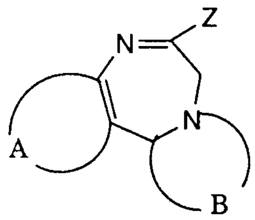

X

worin A und B die in Anspruch 1 angegebene Bedeutung besitzen und Z eine Abgangsgruppe bedeutet,
in Gegenwart einer Base mit einem Isonitril der Formel

$C=N\text{-}CH_2\text{-}Q\text{-}R^1$ XI

worin Q und $R^1$ die in Anspruch 1 angegebene Bedeutung besitzen,
umsetzt, und
k) erwünschtenfalls eine erhaltene basische Verbindung der Formel I in ein pharmazeutisch annehmbares Säureadditionssalz überführt .

**25.** Arzneimittel enthaltend eine Verbindung nach einem der Ansprüche 1-18 und ein therapeutisch inertes Trägermaterial.

**26.** Arzneimittel zur Bekämpfung oder Verhütung von epileptischen Anfallen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren, enthaltend eine Verbindung nach einem der Ansprüche 1-18 und ein therapeutisch inertes Trägermaterial.

**27.** Verwendung von Verbindungen nach einem der Ansprüche 1-18 zur Herstellung von Mitteln zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

**Patentansprüche für folgenden Vertragsstaat : ES**

**1.** Verfahren zur Herstellung von Verbindungen der allgemeinen Formel

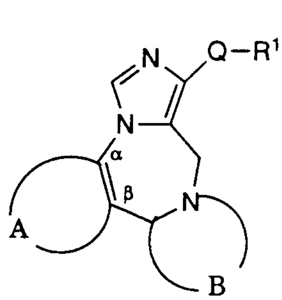

I

worin A zusammen mit den beiden mit $\alpha$ und $\beta$ bezeichneten Kohlenstoffatomen eine der Gruppen

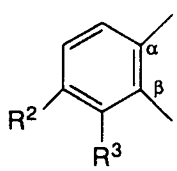 , 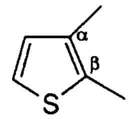 , 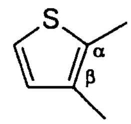 ,

(a) (b) (c)

B einen der Reste

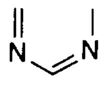 , 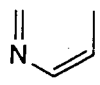 , 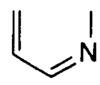 ,

(d) (e) (f)

Q eine der Gruppen

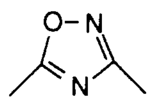 , 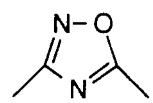 ,

(g) (h)

$R^1$ eine gegebenenfalls durch Hydroxy, niederes Alkoxy, Aryl, $C_{3-6}$-Cycloalkyl, Aroyl, Aryloxy, Heteroaroyloxy, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, eine Aroylgruppe, einen über ein Kohlenstoffatom gebundenen fünfgliedrigen Heterocyclus oder gegebenenfalls durch Acyl oder niederes Alkyl substituiertes $C_{3-6}$-Cycloalkyl,

$R^2$ und $R^3$ je Wasserstoff, Halogen oder niederes Alkyl,

$R^4$ Wasserstoff oder niederes Alkyl,

$R^5$ Wasserstoff, Aryl, Acyl, $C_{3-6}$-Cycloalkyl, Aralkoxycarbonyl oder gegebenenfalls durch Aryl, Morpholino, niederes Alkoxy, Hydroxy, 2,2-Dimethyl-1,3-dioxolan-4-yl, Alkoxycarbonyl, Carbamoyl, Alkoxycarbonylamino, Aralkoxycarbonyl oder Amino substituiertes niederes Alkyl oder $R^4$ und $R^5$ zusammen mit dem Stickstoffatom entweder Phthalimino oder einen sechsgliedrigen gesättigten Heterocyclus bedeuten,

und pharmazeutisch annehmbare Säureadditionssalze von basischen Verbindungen der Formel I, dadurch gekennzeichnet, dass man

a) ein reaktionsfähiges funktionelles Derivat einer Carbonsäure der allgemeinen Formel

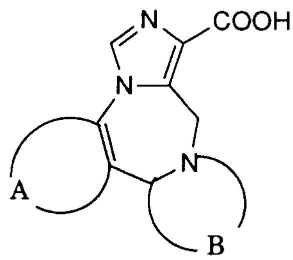

II

worin A und B die in Anspruch 1 angegebene Bedeutung besitzen, mit einem Oxim der allgemeinen Formel

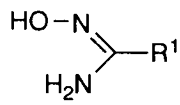

III

worin $R^1$ die in Anspruch 1 angegebene Bedeutung besitzt, umsetzt, oder

b) eine Verbindung der allgemeinen Formel

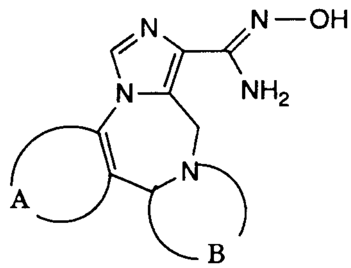

IV

worin A und B die in Anspruch 1 angegebene Bedeutung besitzen, mit einem reaktionsfahigen funktionellen Derivat einer Carbonsäure der allgemeinen Formel

$R^1$-COOH V

worin $R^1$ die in Anspruch 1 angegebene Bedeutung besitzt, umsetzt, oder

c) eine Verbindung der allgemeinen Formel

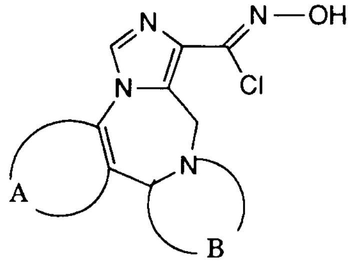

VI

worin A und B die in Anspruch 1 angegebene Bedeutung haben, mit einem Nitril der Formel

$R^{11}$-C≡N VII

worin $R^{11}$ Arylamino-alkyl bedeutet,
umsetzt, oder
d) eine Verbindung der allgemeinen Formel

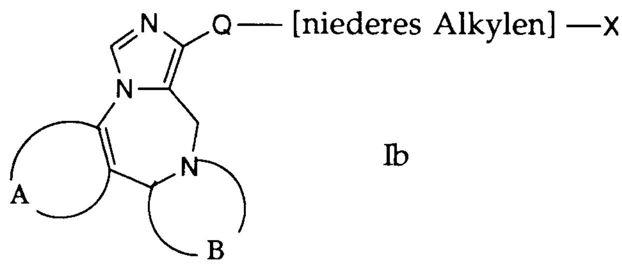

Ib

worin A, B und Q die in Anspruch 1 angegebene Bedeutung haben und X eine Abgangsgruppe bedeutet,
mit einem Amin der Formel

$HNR^4R^5$ VIII

worin $R^4$ und $R^5$ die in Anspruch 1 angegebene Bedeutung haben, umsetzt, oder
e) eine Verbindung der allgemeinen Formel

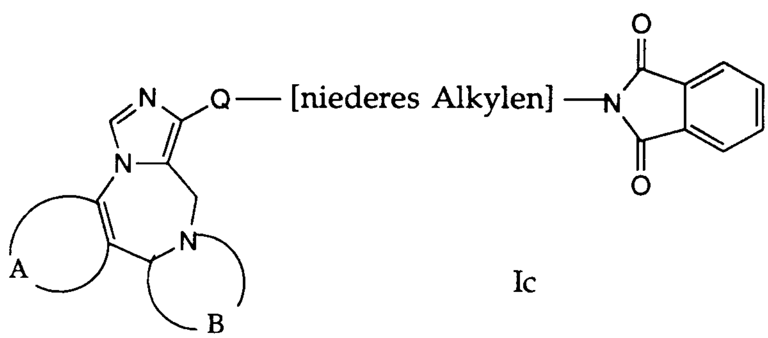

Ic

worin A, B und Q die in Anspruch 1 angegebene Bedeutung besitzen,
in das entsprechende Amin überführt, oder
f) eine Verbindung der allgemeinen Formel

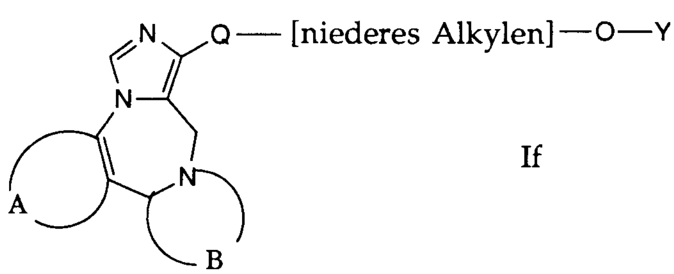

If

worin A, B und Q die in Anspruch 1 angegebene Bedeutung besitzen und Y eine leicht abspaltbare Arylalkylgruppe darstellt,
eine Etherspaltung unterzieht, oder
g) eine Verbindung der allgemeinen Formel

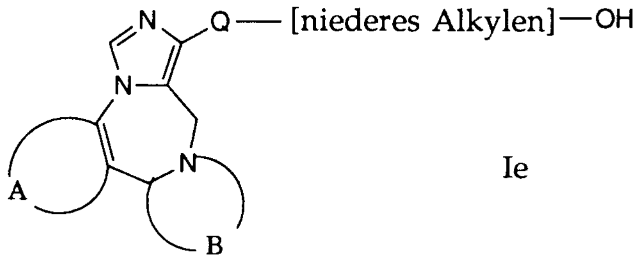

Ie

verestert, oder
h) eine Verbindung der allgemeinen Formel

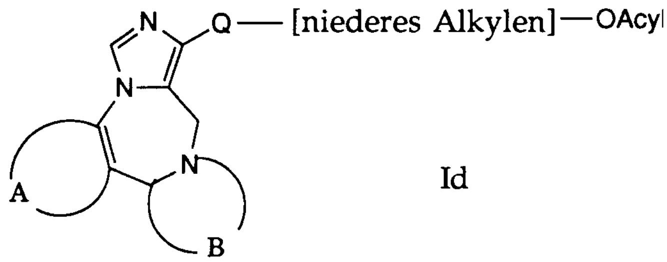

Id

worin A, B und Q die in Anspruch 1 angegebene Bedeutung besitzen,
verseift, oder
i) eine Verbindung der allgemeinen Formel

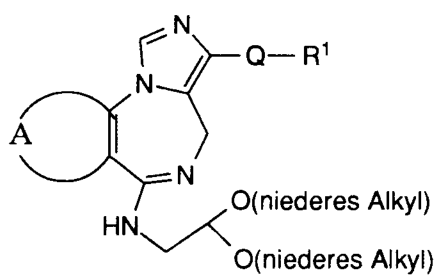

IX

worin A und Q die in Anspruch 1 angegebnene Bedeutung besitzen,
mit einer Säure behandelt, oder
j) eine Verbindung der allgemeinen Formel

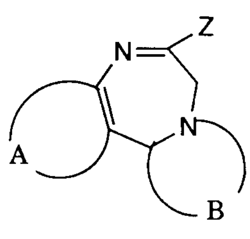

X

worin A und B die in Anspruch 1 angegebene Bedeutung besitzen und Z eine Abgangsgruppe bedeutet,
in Gegenwart einer Base mit einem Isonitril der Formel

$C=N-CH_2-Q-R^1$ XI

worin Q und $R^1$ die in Anspruch 1 angegebene Bedeutung besitzen,
umsetzt, und

k) erwünschtenfalls eine erhaltene basische Verbindung der Formel I in ein pharmazeutisch annehmbares Säureadditionssalz überführt .

**2.** Verfahren zur Herstellung von Verbindungen der in Anspruch 1 definierten Formel I, worin A, B und Q die dort angegebene Bedeutung haben und worin $R^1$ eine gegebenenfalls durch Hydroxy, niederes Alkoxy, Aryl, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, einen über ein Kohlenstoffatom gebundenen fünfgliedrigen Heterocyclus oder $C_{3-6}$-Cycloalkyl, $R^2$ und $R^3$ je Wasserstoff oder Halogen, $R^4$ Wasserstoff oder niederes Alkyl, $R^5$ Wasserstoff, niederes Alkyl, Aryl oder Acyl oder $R^4$ und $R^5$ zusammen mit dem Stickstoffatom entweder Phthalimino oder einen sechsgliedrigen gesättigten Heterocyclus bedeuten, dadurch gekennzeichnet, dass man

a) ein reaktionsfähiges funktionelles Derivat einer Carbonsäure der allgemeinen Formel

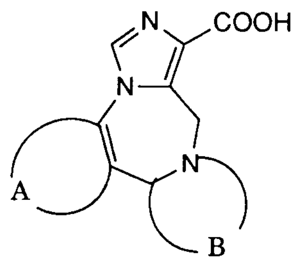

II

worin A und B die in Anspruch 1 angegebene Bedeutung besitzen, mit einem Oxim der allgemeinen Formel

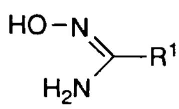

III

worin $R^1$ die in Anspruch 1 angegebene Bedeutung besitzt, umsetzt, oder

b) eine Verbindung der allgemeinen Formel

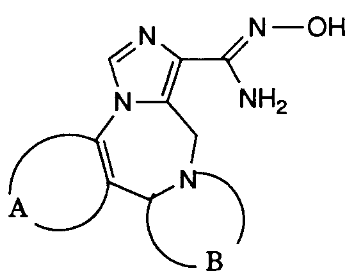

IV

worin A und B die in Anspruch 1 angegebene Bedeutung besitzen, mit einem reaktionsfähigen funktionellen Derivat einer Carbonsäure der allgemeinen Formel

$R^1$-COOH V

worin $R^1$ die in Anspruch 1 angegebene Bedeutung besitzt, umsetzt, oder

c) eine Verbindung der allgemeinen Formel

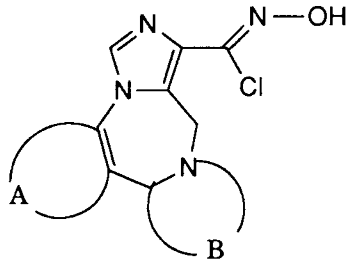

VI

worin A und B die in Anspruch 1 angegebene Bedeutung haben, mit einem Nitril der Formel

$R^{11}$-C≡N VII

worin $R^{11}$ Arylamino-alkyl bedeutet,
umsetzt, oder
d) eine Verbindung der allgemeinen Formel

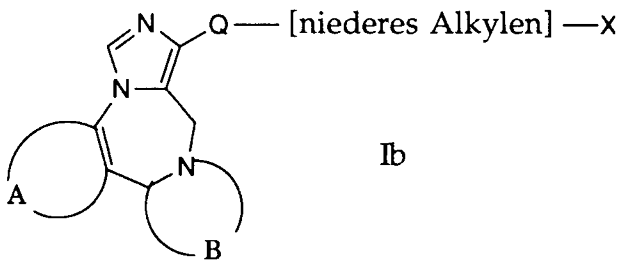

Ib

worin A, B und Q die in Anspruch 1 angegebene Bedeutung haben und X eine Abgangsgruppe bedeutet,
mit einem Amin der Formel

$HNR^4R^5$ VIII

worin $R^4$ und $R^5$ die in Anspruch 1 angegebene Bedeutung haben, umsetzt, oder
e) eine Verbindung der allgemeinen Formel

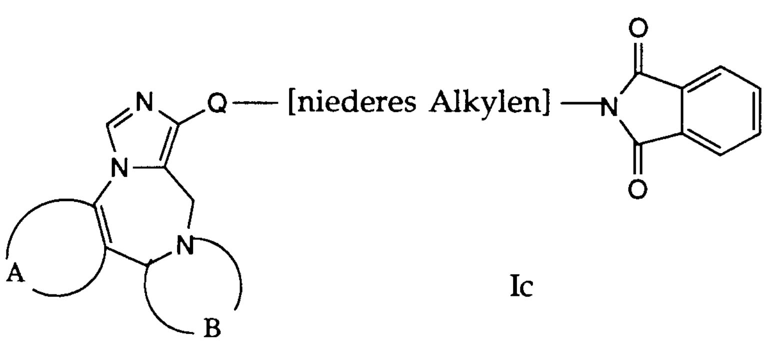

Ic

worin A, B und Q die in Anspruch 1 angegebene Bedeutung besitzen,
in das entsprechende Amin überführt, oder
f) eine Verbindung der allgemeinen Formel

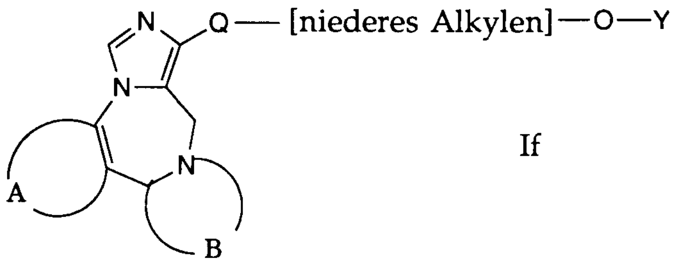

If

worin A, B und Q die in Anspruch 1 angegebene Bedeutung besitzen und Y eine leicht abspaltbare Arylalkylgruppe darstellt,
eine Etherspaltung unterzieht, oder
g) eine Verbindung der allgemeinen Formel

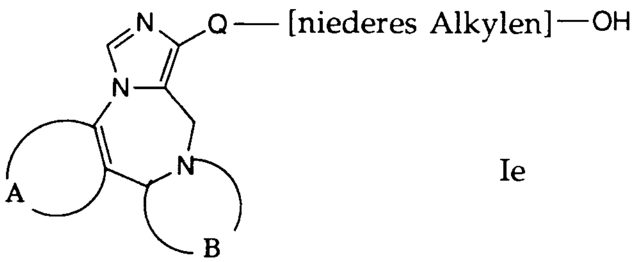

Ie

verestert, oder
h) eine Verbindung der allgemeinen Formel

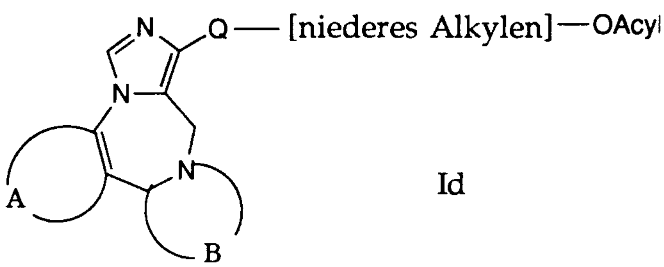

Id

worin A, B und Q die in Anspruch 1 angegebene Bedeutung besitzen,
verseift, oder
i) eine Verbindung der allgemeinen Formel

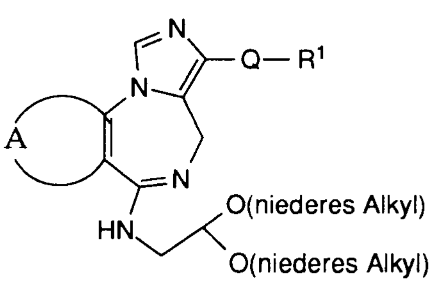

IX

worin A und Q die in Anspruch 1 angegebnene Bedeutung besitzen,
mit einer Säure behandelt, oder
j) eine Verbindung der allgemeinen Formel

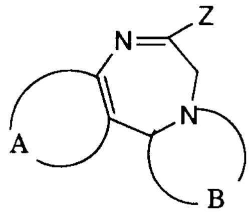

X

worin A und B die in Anspruch 1 angegebene Bedeutung besitzen und Z eine Abgangsgruppe bedeutet,
in Gegenwart einer Base mit einem Isonitril der Formel

$C=N\text{-}CH_2\text{-}Q\text{-}R^1$ XI

worin Q und $R^1$ die in Anspruch 1 angegebene Bedeutung besitzen,
umsetzt, und
k) erwünschtenfalls eine erhaltene basische Verbindung der Formel I in ein pharmazeutisch annehmbares Säureadditionssalz überführt .

**3.** Verfahren nach Anspruch 1 oder 2, dadurch gekennzeichnet, dass
a) der fünfgliedrige, über ein Kohlenstoffatom gebundene Heterocyclus aromatisch oder gesättigtist, ein Stickstoff-, Sauerstoff- oder Schwefelatom und gegebenenfalls zusätzlich ein Stickstoffatom als Ringglieder enthält, und unsubstituiert oder durch niederes Alkyl substituiert ist oder in Nachbarschaft zu einem nichtaromatischen Stickstoffatom eine Oxogruppe enthält;
b) der fünfgliedrige, über ein Stickstoffatom gebundene Heterocyclus aromatisch ist und gegebenenfalls ein zweites Stickstoffatom als zusätzliches Ringglied enthält;
c) der sechsgliedrige gesättigte Heterocyclus als Ringglied zusätzlich ein Sauerstoffatom oder die Gruppe $>N\text{-}R^6$ enthalten kann, wobei $R^6$ niederes Alkyl, Aryl, niederes Alkenyl oder niederes Alkinyl bedeutet.

**4.** Verfahren nach Anspruch 3, dadurch gekennzeichnet, dass
a) der fünfgliedrige, über ein Kohlenstoffatom gebundene Heterocyclus eine 2-Thienyl-, 3-Thienyl-, 2-Furyl-, 3-Furyl-, 5-Oxazolyl- oder 2-Tetrahydrofurylgruppe
b) der fünfgliedrige, über ein Stickstoffatom gebundene Heterocyclus eine 1-Imidazolylgruppe darstellt
c) der sechsgliedrige, gesättigte Heterocyclus eine 4-Morpholino- oder eine 1-Piperazinylgruppe ist, welche in 4-Stellung durch niederes Alkyl, Aryl, niederes Alkenyl oder niederes Alkinyl substituiert ist.

**5.** Verfahren nach Anspruch 1 oder 2, worin $R^1$ Cyclopropyl bedeutet.

**6.** Verfahren nach einem der Ansprüche 1-5, worin A die Gruppe a) bedeutet.

**7.** Verfahren nach einem der Ansprüche 1-6, worin $R^3$ Wasserstoff und $R^2$ Wasserstoff, Fluor oder Chlor bedeuten.

**8.** Verfahren nach einem der Ansprüche 1-7, worin B den Rest d) oder e)bedeutet.

**9.** Verfahren nach Anspruch 1, dadurch gekennzeichnet, dass man 10-(3-Cyclopropyl-1,2,4-oxadiazol-5-yl)-3-fluor-9H-imidazo[1,5-a][1,2,4]triazolo[1,5-d][1,4]benzodiazepin herstellt.

**10.** Verfahren nach Anspruch 1, dadurch gekennzeichnet, dass man 10-(5-Cyclopropyl-1,2,4-oxadiazol-3-yl)-3-fluor-9H-imidazo[1,5-a][1,2,4]triazolo[1,5-d][1,4]benzodiazepin herstellt.

**11.** Verfahren nach Anspruch 1, dadurch gekennzeichnet, dass man 10-(5-Cyclopropyl-1,2,4-oxadiazol-3-yl)-9H-imidazo[1,5-a][1,2,4]triazolo[1,5-d][1,4]benzodiazepin herstellt.

**12.** Verfahren nach Anspruch 1, dadurch gekennzeichnet, dass man 10-(3-Cyclopropyl-1,2,4-oxadiazol-5-yl)-9H-imidazo[1,5-a][1,2,4]triazolo[1,5-d][1,4]benzodiazepin herstellt.

**13.** Verfahren nach Anspruch 1, dadurch gekennzeichnet, dass man 10-(3-Cyclopropyl-1,2,4-oxadiazol-5-yl)-3-fluor-9H-diimidazo[1,5-a:1',2'-d][1,4]benzodiazepin herstellt.

**14.** Verfahren nach Anspruch 1, dadurch gekennzeichnet, dass man 10-(5-Cyclopropyl-1,2,4-oxadiazol-3-yl)-3-fluor-9H-diimidazo[1,5-a:1',2'-d][1,4]benzodiazepin herstellt.

**15.** Verfahren nach Anspruch 1, dadurch gekennzeichnet, dass man 10-(5-Cyclopropyl-1,2,4-oxadiazol-3-yl)-9H-diimidazo[1,5-a:1',2'-d]1,4]benzodiazepin herstellt.

**16.** Verfahren nach Anspruch 1, dadurch gekennzeichnet, dass man 3-Chlor-10-(5-cyclopropyl-1,2,4-oxadiazol-3-yl)-9H-diimidazo[1,5-a:1',2'-d][1,4]benzodiazepin herstellt.

**17.** Verfahren nach Anspruch 1, dadurch gekennzeichnet, dass man 4-Chlor-10-(3-cyclopropyl-1,2,4-oxadiazol-5-yl)-9H-diimidazo[1,5-a:1',2'-d][1,4]benzodiazepin herstellt.

**18.** Verfahren nach Anspruch 1, dadurch gekennzeichnet, dass man 4-Chlor-10-(5-cyclopropyl-1,2,4-oxadiazol-3-yl)-9H-diimidazo[1,5-a:1',2'-d][1,4]benzodiazepin herstellt.

**19.** Verfahren zur Herstellung von Arzneimittel, insbesondere zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren, dadurch gekennzeichnet, dass man eine Verbindung der in Anspruch 1 angegebenen Formel I und erwünschtenfalls einen anderen therapeutisch wirksamen Stoff zusammen mit einem therapeutisch inerten Trägermaterial in eine galenische Darreichungsform bringt.

**20.** Verwendung von Verbindungen der in Anspruch 1 angegebenen Formel I zur Herstellung von Mitteln zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

**Patentansprüche für folgenden Vertragsstaat : GR**

**1.** Verfahren zur Herstellung von Verbindungen der allgemeinen Formel

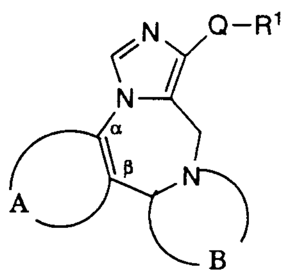

I

worin A zusammen mit den beiden mit $\alpha$ und $\beta$ bezeichneten Kohlenstoffatomen eine der Gruppen

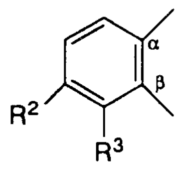 , 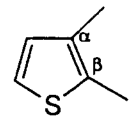 , 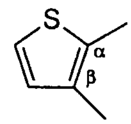 ,

(a) (b) (c)

B einen der Reste

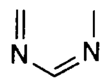 , 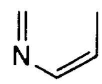 , 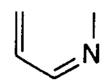 ,

(d) (e) (f)

Q eine der Gruppen

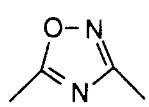 , 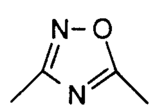 ,

(g) (h)

$R^1$ eine gegebenenfalls durch Hydroxy, niederes Alkoxy, Aryl, $C_{3-6}$-Cycloalkyl, Aroyl, Aryloxy, Heteroaroyloxy, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, eine Aroylgruppe, einen über ein Kohlenstoffatom gebundenen fünfgliedrigen Heterocyclus oder gegebenenfalls durch Acyl oder niederes Alkyl substituiertes $C_{3-6}$-Cycloalkyl,

$R^2$ und $R^3$ je Wasserstoff, Halogen oder niederes Alkyl,

$R^4$ Wasserstoff oder niederes Alkyl,

$R^5$ Wasserstoff, Aryl, Acyl, $C_{3-6}$-Cycloalkyl, Aralkoxycarbonyl oder gegebenenfalls durch Aryl, Morpholino, niederes Alkoxy, Hydroxy, 2,2-Dimethyl-1,3-dioxolan-4-yl, Alkoxycarbonyl, Carbamoyl, Alkoxycarbonylamino, Aralkoxycarbonyl oder Amino substituiertes niederes Alkyl oder $R^4$ und $R^5$ zusammen mit dem Stickstoffatom entweder Phthalimino oder einen sechsgliedrigen gesättigten Heterocyclus bedeuten,

und pharmazeutisch annehmbare Säureadditionssalze von basischen Verbindungen der Formel I, dadurch gekennzeichnet, dass man

a) ein reaktionsfähiges funktionelles Derivat einer Carbonsäure der allgemeinen Formel

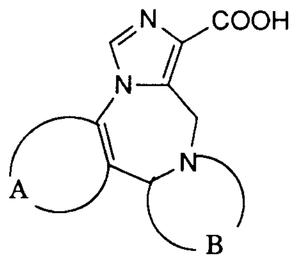

II

worin A und B die in Anspruch 1 angegebene Bedeutung besitzen, mit einem Oxim der allgemeinen Formel

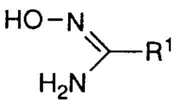

III

worin $R^1$ die in Anspruch 1 angegebene Bedeutung besitzt, umsetzt, oder
b) eine Verbindung der allgemeinen Formel

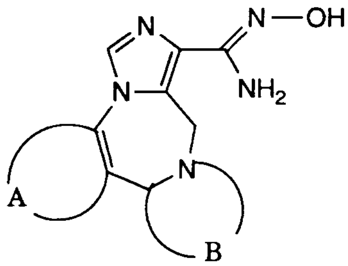

IV

worin A und B die in Anspruch 1 angegebene Bedeutung besitzen, mit einem reaktionsfähigen funktionellen Derivat einer Carbonsäure der allgemeinen Formel

$R^1$-COOH    V

worin $R^1$ die in Anspruch 1 angegebene Bedeutung besitzt, umsetzt, oder
c) eine Verbindung der allgemeinen Formel

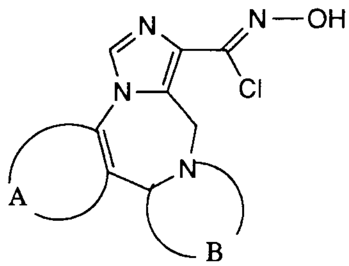

VI

worin A und B die in Anspruch 1 angegebene Bedeutung haben, mit einem Nitril der Formel

$R^{11}$-C≡N    VII

worin $R^{11}$ Arylamino-alkyl bedeutet,
umsetzt, oder
d) eine Verbindung der allgemeinen Formel

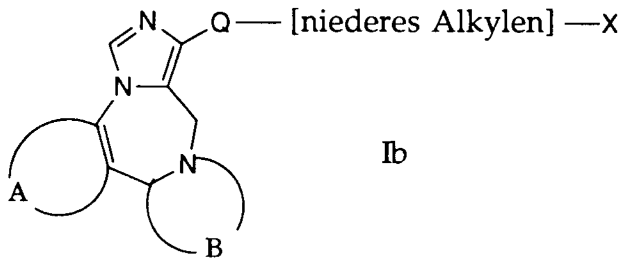

Ib

worin A, B und Q die in Anspruch 1 angegebene Bedeutung haben und X eine Abgangsgruppe bedeutet,
mit einem Amin der Formel

$HNR^4R^5$ VIII

worin $R^4$ und $R^5$ die in Anspruch 1 angegebene Bedeutung haben, umsetzt, oder
e) eine Verbindung der allgemeinen Formel

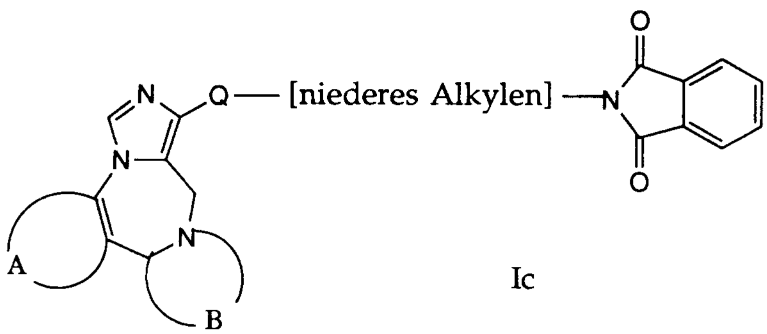

Ic

worin A, B und Q die in Anspruch 1 angegebene Bedeutung besitzen,
in das entsprechende Amin überführt, oder
f) eine Verbindung der allgemeinen Formel

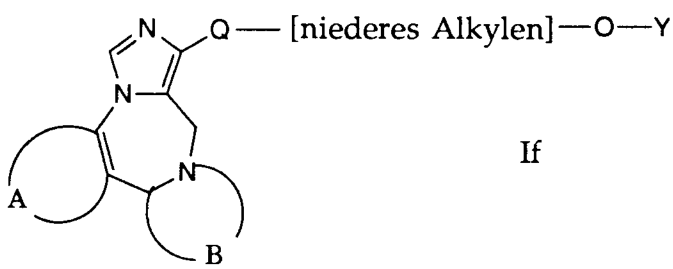

If

worin A, B und Q die in Anspruch 1 angegebene Bedeutung besitzen und Y eine leicht abspaltbare Arylalkylgruppe darstellt,
eine Etherspaltung unterzieht, oder
g) eine Verbindung der allgemeinen Formel

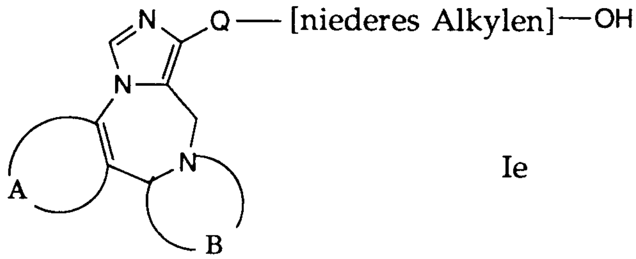

Ie

verestert, oder
h) eine Verbindung der allgemeinen Formel

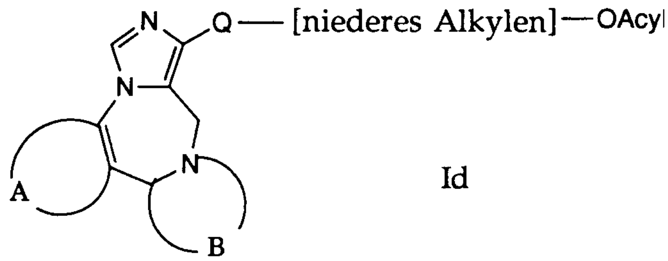

Id

worin A, B und Q die in Anspruch 1 angegebene Bedeutung besitzen,
verseift, oder
i) eine Verbindung der allgemeinen Formel

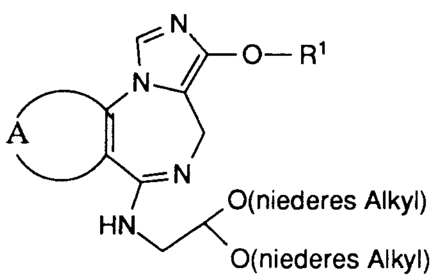

IX

worin A und Q die in Anspruch 1 angegebnene Bedeutung besitzen,
mit einer Säure behandelt, oder
j) eine Verbindung der allgemeinen Formel

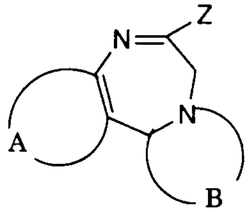

X

worin A und B die in Anspruch 1 angegebene Bedeutung besitzen und Z eine Abgangsgruppe bedeutet,
in Gegenwart einer Base mit einem Isonitril der Formel

$C=N-CH_2-Q-R^1$ XI

worin Q und $R^1$ die in Anspruch 1 angegebene Bedeutung besitzen,
umsetzt, und
k) erwünschtenfalls eine erhaltene basische Verbindung der Formel I in ein pharmazeutisch annehmbares Säureadditionssalz überführt .

2. Verfahren zur Herstellung von Verbindungen der in Anspruch 1 definierten Formel I, worin A, B und Q die dort angegebene Bedeutung haben und worin $R^1$ eine gegebenenfalls durch Hydroxy, niederes Alkoxy, Aryl, Acyloxy, Aryl-(niederes)-alkoxy, Halogen, die Gruppe $-NR^4R^5$ oder einen über ein Kohlenstoff- oder ein Stickstoffatom gebundenen fünfgliedrigen Heterocyclus substituierte niedere Alkylgruppe, eine niedere Alkenyl- oder Alkinylgruppe, einen über ein Kohlenstoffatom gebundenen fünfgliedrigen Heterocyclus oder $C_{3-6}$-Cycloalkyl, $R^2$ und $R^3$ je Wasserstoff oder Halogen, $R^4$ Wasserstoff oder niederes Alkyl, $R^5$ Wasserstoff, niederes Alkyl, Aryl oder Acyl oder $R^4$ und $R^5$ zusammen mit dem Stickstoffatom entweder Phthalimino oder einen sechsgliedrigen gesättigten Heterocyclus bedeuten, dadurch gekennzeichnet, dass man
a) ein reaktionsfahiges funktionelles Derivat einer Carbonsäure der allgemeinen Formel

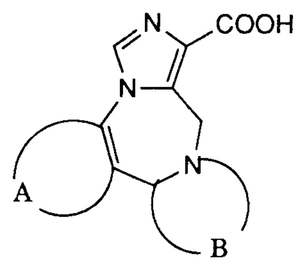

II

worin A und B die in Anspruch 1 angegebene Bedeutung besitzen,
mit einem Oxim der allgemeinen Formel

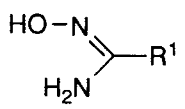

III

worin $R^1$ die in Anspruch 1 angegebene Bedeutung besitzt,
umsetzt, oder
b) eine Verbindung der allgemeinen Formel

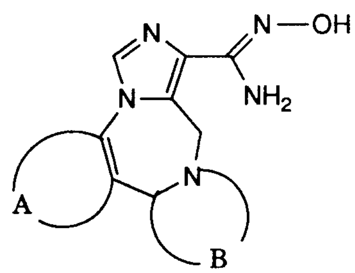

IV

worin A und B die in Anspruch 1 angegebene Bedeutung besitzen,
mit einem reaktionsfähigen funktionellen Derivat einer Carbonsäure der allgemeinen Formel

$R^1$-COOH V

worin $R^1$ die in Anspruch 1 angegebene Bedeutung besitzt,
umsetzt, oder
c) eine Verbindung der allgemeinen Formel

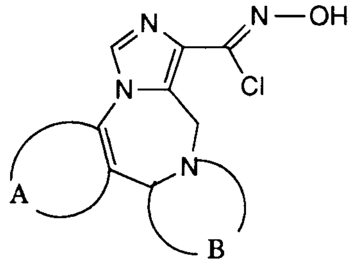

VI

worin A und B die in Anspruch 1 angegebene Bedeutung haben, mit einem Nitril der Formel

$R^{11}$-C≡N     VII

worin $R^{11}$ Arylamino-alkyl bedeutet, umsetzt, oder
d) eine Verbindung der allgemeinen Formel

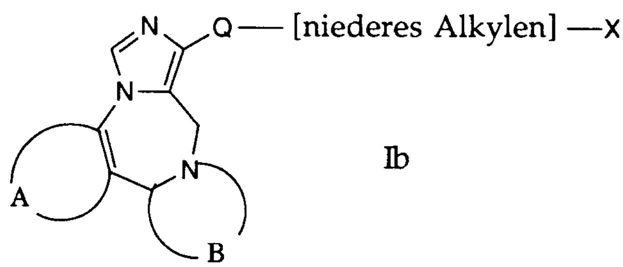

Ib

worin A, B und Q die in Anspruch 1 angegebene Bedeutung haben und X eine Abgangsgruppe bedeutet, mit einem Amin der Formel

$HNR^4R^5$     VIII

worin $R^4$ und $R^5$ die in Anspruch 1 angegebene Bedeutung haben, umsetzt, oder
e) eine Verbindung der allgemeinen Formel

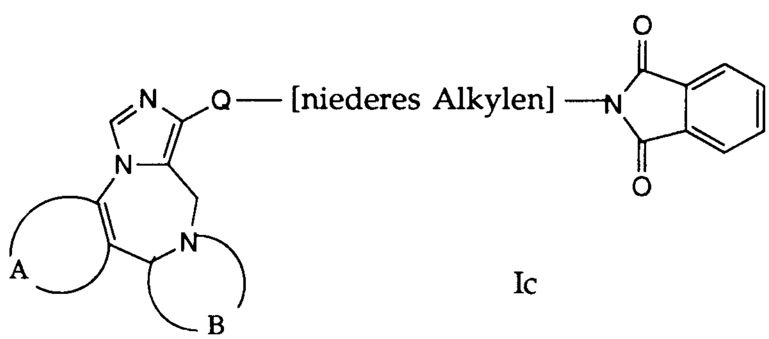

Ic

worin A, B und Q die in Anspruch 1 angegebene Bedeutung besitzen, in das entsprechende Amin überführt, oder
f) eine Verbindung der allgemeinen Formel

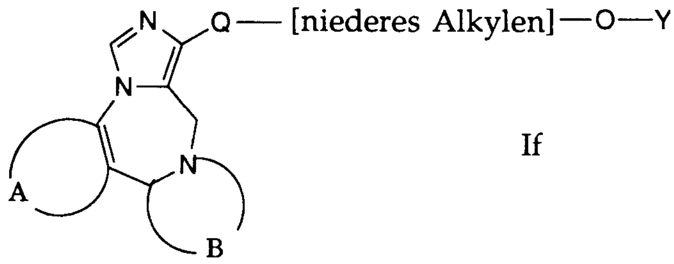

If

worin A, B und Q die in Anspruch 1 angegebene Bedeutung besitzen und Y eine leicht abspaltbare Arylalkylgruppe darstellt,
eine Etherspaltung unterzieht, oder
g) eine Verbindung der allgemeinen Formel

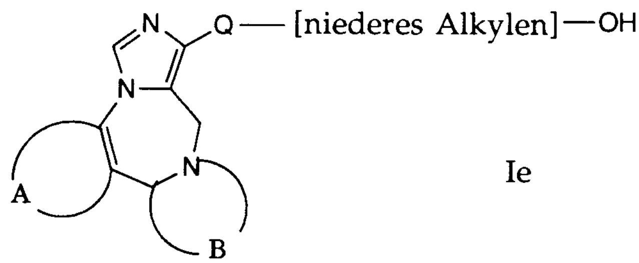

Ie

verestert, oder
h) eine Verbindung der allgemeinen Formel

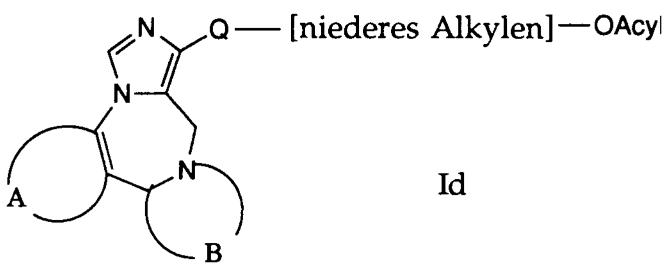

Id

worin A, B und Q die in Anspruch 1 angegebene Bedeutung besitzen,
verseift, oder
i) eine Verbindung der allgemeinen Formel

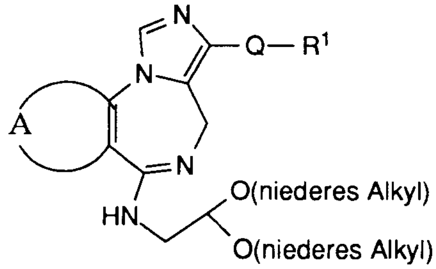

IX

worin A und Q die in Anspruch 1 angegebnene Bedeutung besitzen,
mit einer Säure behandelt, oder
j) eine Verbindung der allgemeinen Formel

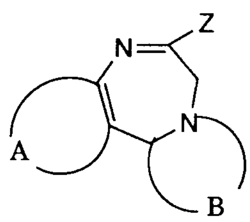

X

worin A und B die in Anspruch 1 angegebene Bedeutung besitzen und Z eine Abgangsgruppe bedeutet,
in Gegenwart einer Base mit einem Isonitril der Formel

$C=N\text{-}CH_2\text{-}Q\text{-}R^1$ XI

worin Q und $R^1$ die in Anspruch 1 angegebene Bedeutung besitzen,
umsetzt, und
k) erwünschtenfalls eine erhaltene basische Verbindung der Formel I in ein pharmazeutisch annehmbares Säureadditionssalz überführt .

**3.** Verfahren nach Anspruch 1 oder 2, dadurch gekennzeichnet, dass
a) der fünfgliedrige, über ein Kohlenstoffatom gebundene Heterocyclus aromatisch oder gesättigt ist, ein Stickstoff-, Sauerstoff- oder Schwefelatom und gegebenenfalls zusätzlich ein Stickstoffatom als Ringglieder enthält, und unsubstituiert oder durch niederes Alkyl substituiert ist oder in Nachbarschaft zu einem nichtaromatischen Stickstoffatom eine Oxogruppe enthält;
b) der fünfgliedrige, über ein Stickstoffatom gebundene Heterocyclus aromatisch ist und gegebenenfalls ein zweites Stickstoffatom als zusätzliches Ringglied enthält;
c) der sechsgliedrige gesättigte Heterocyclus als Ringglied zusätzlich ein Sauerstoffatom oder die Gruppe >N-$R^6$ enthaltenkann, wobei $R^6$ niederes Alkyl, Aryl, niederes Alkenyl oder niederes Alkinyl bedeutet.

**4.** Verfahren nach Anspruch 3, dadurch gekennzeichnet, dass
a) der fünfgliedrige, über ein Kohlenstoffatom gebundene Heterocyclus eine 2-Thienyl-, 3-Thienyl-, 2-Furyl-, 3-Furyl-, 5-Oxazolyl- oder 2-Tetrahydrofurylgruppe
b) der fünfgliedrige, über ein Stickstoffatom gebundene Heterocyclus eine 1-Imidazolylgruppe darstellt
c) der sechsgliedrige, gesättigte Heterocyclus eine 4-Morpholino- oder eine 1-Piperazinylgruppe ist, welche in 4-Stellung durch niederes Alkyl, Aryl, niederes Alkenyl oder niederes Alkinyl substituiert ist.

**5.** Verfahren nach Anspruch 1 oder 2, worin $R^1$ Cyclopropyl bedeutet.

**6.** Verfahren nach einem der Ansprüche 1-5, worin A die Gruppe a) bedeutet.

**7.** Verfahren nach einem der Ansprüche 1-6, worin $R^3$ Wasserstoff und $R^2$ Wasserstoff, Fluor oder Chlor bedeuten.

**8.** Verfahren nach einem der Ansprüche 1-7, worin B den Rest d) oder e) bedeutet.

**9.** Verfahren nach Anspruch 1, dadurch gekennzeichnet, dass man 10-(3-Cyclopropyl-1,2,4-oxadiazol-5-yl)-3-fluor-9H-imidazo[1,5-a][1,2,4]triazolo[1,5-d][1,4]benzodiazepin herstellt.

**10.** Verfahren nach Anspruch 1, dadurch gekennzeichnet, dass man 10-(5-Cyclopropyl-1,2,4-oxadiazol-3-yl)-3-fluor-9H-imidazo[1,5-a][1,2,4]triazolo[1,5-d][1,4]benzodiazepin herstellt.

**11.** Verfahren nach Anspruch 1, dadurch gekennzeichnet, dass man 10-(5-Cyclopropyl-1,2,4-oxadiazol-3-yl)-9H-imidazo[1,5-a][1,2,4]triazolo[1,5-d][1,4]benzodiazepin herstellt.

**12.** Verfahren nach Anspruch 1, dadurch gekennzeichnet, dass man 10-(3-Cyclopropyl-1,2,4-oxadiazol-5-yl)-9H-imidazo[1,5-a][1,2,4]triazolo[1,5-d][1,4]benzodiazepin herstellt.

**13.** Verfahren nach Anspruch 1, dadurch gekennzeichnet, dass man 10-(3-Cyclopropyl-1,2,4-oxadiazol-5-yl)-3-fluor-9H-diimidazo[1,5-a:1',2'-d][1,4]benzodiazepin herstellt.

**14.** Verfahren nach Anspruch 1, dadurch gekennzeichnet, dass man 10-(5-Cyclopropyl-1,2,4-oxadiazol-3-yl)-3-fluor-9H-diimidazo[1,5-a:1',2'-d][1,4]benzodiazepin herstellt.

**15.** Verfahren nach Anspruch 1, dadurch gekennzeichnet, dass man 10-(5-Cyclopropyl-1,2,4-oxadiazol-3-yl)-9H-diimidazo[1,5-a:1',2'-d][1,4]benzodiazepin herstellt.

**16.** Verfahren nach Anspruch 1, dadurch gekennzeichnet, dass man 3-Chlor-10-(5-cyclopropyl-1,2,4-oxadiazol-3-yl)-9H-diimidazo]1,5-a:1',2'-d][1,4]benzodiazepin herstellt.

**17.** Verfahren nach Anspruch 1, dadurch gekennzeichnet, dass man 4-Chlor-10-(3-cyclopropyl-1,2,4-oxadiazol-5-yl)-9H-diimidazo[1,5-a:1',2'-d][1,4]benzodiazepin herstellt.

**18.** Verfahren nach Anspruch 1, dadurch gekennzeichnet, dass man 4-Chlor-10-(5-cyclopropyl-1,2,4-oxadiazol-3-yl)-9H-diimidazo[1,5-a:1',2'-d][1,4]benzodiazepin herstellt.

**19.** Verfahren zur Herstellung von Arzneimittel, insbesondere zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren, dadurch gekennzeichnet, dass man eine Verbindung der in Anspruch 1 angegebenen Formel I und erwünschtenfalls einen anderen therapeutisch wirksamen Stoff zusammen mit einem therapeutisch inerten Trägermaterial in eine galenische Darreichungsform bringt.

**20.** Verwendung von Verbindungen der in Anspruch 1 angegebenen Formel I zur Herstellung von Mitteln zur Bekämpfung oder Verhütung von epileptischen Anfällen, Angst-, Spannungs- und Erregungszuständen, Schlafstörungen, Symptome der Schizophrenie, hepatischen Enzephalopathie und der senilen Demenzen, sowie um partiell oder ganz unerwünschte Nebenwirkungen von am Benzodiazepin-Rezeptor wirkenden Stoffen nach Ueberdosierung oder nach deren Verwendung in der Intensivmedizin und in der Anaesthesie zu antagonisieren.

**21.** Verbindungen der allgemeinen Formel

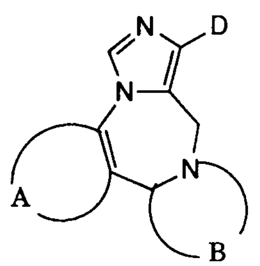

XXIV

worin A und B die in Anspruch 1 angegebene Bedeutung besitzen und D eine der Gruppen -COOH, $-C(NH_2)=NOH$, -C(Cl) = NOH, $-CONH_2$, -CN, $-CH_2-OH$, -CHO, -CH = NOH, oder $-COOR^9$ bedeuten, wobei $R^9$ niederes Alkyl bedeutet.

**22.** Verbindungen der allgemeinen Formel

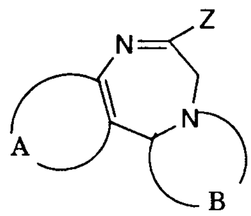

X

worin A und B die in Anspruch 1 angegebene Bedeutung besitzen und Z eine Abgangsgruppe bedeutet.

**23.** Verbindungen der allgemeinen Formel

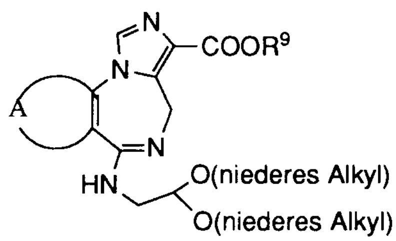

XVIII

worin A die in Anspruch 1 angegebene Bedeutung besitzt und $R^9$ niederes Alkyl bedeutet.